# EUROPEAN PATENT APPLICATION

(11) **EP 0 925 786 A1**
(43) Date of publication of application: **30.06.1999**
(21) Application number: 97930735.2
(22) Date of filing: 09.07.1997
(51) Int. Cl.: A61K 31/335, A61K 31/34, A61K 31/35, C07D 305/08, C07D 307/22, C07D 309/14

(54) **REMEDIES FOR ISCHEMIC DISEASES**

(30) Priority: 10.07.1996 JP 18078396; 06.08.1996 JP 20701196
(71) Applicant: MITSUBISHI CHEMICAL CORPORATION, Chiyoda-ku, Tokyo 100-0005 (JP)
(72) Inventor: YOSHII, Narihiko, Mitsubishi Chemical Corporation, Yokohama-shi, Kanagawa 227 (JP); SAITO, Ken-ichi, Mitsubishi Chemical Corporation, Yokohama-shi, Kanagawa 227 (JP); KAWASUMI, Hisashi, Mitsubishi Chemical Corporation, Yokohama-shi, Kanagawa 227 (JP); ANABUKI, Jun, Mitsubishi Chemical Corporation, Yokohama-shi, Kanagawa 227 (JP); ANDO, Ryoichi, Mitsubishi Chemical Corporation, Yokohama-shi, Kanagawa 227 (JP)
(74) Representative: Weisert, Annekäte, Dipl.-Ing. Dr.-Ing.
(86) International application number: JP9702378
(87) International publication number: WO9801130

(57) **Abstract**

A medicament for ischemic diseases, which comprises as its active ingredient an oxygen-containing heterocyclic derivative represented by the following general formula (I): 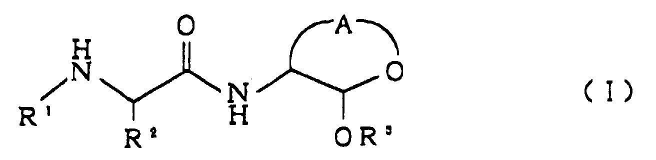 wherein R¹ represents 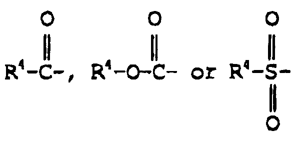 (wherein R⁴ represents an alkyl group, an aryl group or a cycloalkyl group), R² represents an alkyl group, R³ represents hydrogen atom or an acyl group and A represents an alkylene group.

This derivative is useful as a drug for the treatment of ischemic diseases such as ischemic brain diseases, cerebral stroke, cerebral thrombosis, cerebral embolism and myocardial infarction.

## Description

### TECHNICAL FIELD:

This invention relates to novel medicaments for use in ischemic diseases. More particularly, it relates to a novel medicament for use in ischemic diseases, which contains an oxygen-containing heterocyclic derivative represented by a specified chemical formula, a pharmaceutically acceptable salt thereof, or a solvate or hydrate thereof as the active ingredient.

### BACKGROUND ART:

Cerebrovascular accidents broadly known as cerebral stroke exist in various hemorrhagic and ischemic disease types and are regarded as one of the three major causes of death in Europe and America and also in Japan, and their prevention and treatment are important medical problems to be resolved in anticipation of an aging society in the 21st century. Though fatal cerebral bleeding has been decreasing in recent years, ischemic brain diseases have been increasing on the contrary, so that it seems that patients in the chronic stage of ischemic brain diseases will further increase in the present state of aging society. Since such patients have a possibility of falling into cerebrovascular dementia, it seems that the importance of its proper treatment will increase more and more in the future, so that development of effective drugs is called for in this field.

In addition, while myocardial infarction and the like coronary vessel disorders have already been posing problems in Europe and America, persons with advanced arteriosclerosis are rapidly increasing in Japan caused by the westernization of eating habits and, as the results, the number of persons with the onset of an ischemic disease, namely myocardial infarction, is also increasing. Since myocardial infarction is a fatal disease, great concern has been directed toward the development of a reliable and effective drug for its attack.

### DISCLOSURE OF THE INVENTION:

The present invention contemplates satisfying such demands.

The medicament for ischemic diseases according to the present invention uses, as its active ingredient, an oxygen-containing heterocyclic derivative represented by the following general formula (I): 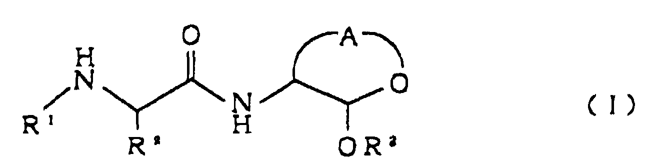 [wherein R¹ represents 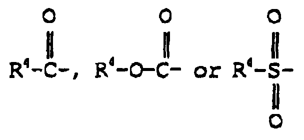 (wherein R⁴ represents a C₁-C₂₀ alkyl group which may be substituted with a C₆-C₁₄ aryl group that may have a substituent group; a C₃-C₈ cycloalkyl group; or a C₆-C₁₄ aryl group which may have a substituent group), R² represents a C₁-C₆ alkyl group, R³ represents hydrogen atom or 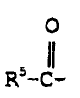 (wherein R⁵ represents a C₁-C₁₀ alkyl group), and A represents a C₁-C₃ alkylene group which may have a C₁-C₃ alkyl group], a pharmaceutically acceptable salt thereof or a solvate or hydrate thereof.

### BEST MODE OF CARRYING OUT THE INVENTION

The following describes the present invention in detail.

In the aforementioned general formula (I), examples of the C₁-C₂₀ alkyl group defined by R⁴ include methyl, ethyl, propyl, isopropyl, butyl, isobutyl, sec-butyl, tert-butyl, pentyl, isopentyl, neopentyl, tert-pentyl, hexyl, isohexyl, heptyl, octyl, nonyl, decyl, undecyl, dodecyl, tridecyl, tetradecyl, pentadecyl, hexadecyl, heptadecyl, octadecyl, nonadecyl, eicosyl and the like groups, of which methyl group or a C₁₃-C₁₆ alkyl group such as tridecyl, tetradecyl, pentadecyl and hexadecyl is preferred. These alkyl groups may be substituted with a C₆-C₁₄ aryl group such as phenyl, naphthyl and anthryl. These aryl groups may further have substituent groups which will be described later. Examples of the C₃-C₈ cycloalkyl group include cyclopropyl, cyclobutyl, cyclopentyl, cyclohexyl, cycloheptyl, cyclooctyl and the like groups. Examples of the C₆-C₁₄ aryl group include phenyl, naphthyl, anthryl and the like groups. These aryl groups may further have one or more substituent groups selected from a halogen atom such as fluorine, chlorine and bromine; a C₁-C₅ alkyl group such as methyl, ethyl, propyl, isopropyl, butyl, isobutyl, sec-butyl, tert-butyl, pentyl, isopentyl, neopentyl and tert-pentyl; trifluoromethyl group; carboxyl group; nitro group; hydroxyl group; methylenedioxy group; and a C₁-C₅ alkoxy group such as methoxy, ethoxy, propoxy, isopropoxy, butoxy, isobutoxy, tert-butoxy, pentyloxy and isopentyloxy.

When R¹ is R⁴CO-, it is desirable that R⁴ is a C₁-C₂₀ alkyl group which may be substituted with a C₆-C₁₄ aryl group that may have a substituent group, particularly an unsubstituted C₁₄-C₁₆ alkyl group, or a C₆-C₁₄ aryl group which may have a substituent group. Also, A is preferably a C₁-C₃ alkylene group. In a particularly preferred combination, R⁴ is a C₁₄-C₁₆ alkyl group and A is a C₁-C₃ alkylene group.

When R¹ is R¹OCO-, it is desirable that R⁴ is a C₁-C₂₀ alkyl group which may be substituted with a C₆-C₁₄ aryl group that may have a substituent group, particularly an unsubstituted C₁₃-C₁₅ alkyl group, or a C₃-C₈ cycloalkyl group. Also, A is preferably a C₁-C₃ alkylene group. In a particularly preferred combination, R⁴ is a C₁₃-C₁₅ alkyl group and A is a C₁-C₃ alkylene group.

When R¹ is R⁴SO₂-, it is desirable that R⁴ is a C₁-C₂₀ alkyl group or a C₆-C₁₄ aryl group which may have a substituent group. Among these alkyl groups, a C₁₄-C₁₆ alkyl group is particularly preferred.

Examples of the C₁-C₆ alkyl group defined by R² include methyl, ethyl, propyl, isopropyl, butyl, isobutyl, sec-butyl, tert-butyl, pentyl, isopentyl, neopentyl, tert-pentyl, hexyl, isohexyl and the like groups.

Examples of the C₁-C₁₀ alkyl group defined by R⁵ include methyl, ethyl, propyl, isopropyl, butyl, isobutyl, sec-butyl, tert-butyl, pentyl, isopentyl, neopentyl, tert-pentyl, hexyl, isohexyl, heptyl, octyl, nonyl, decyl and the like groups.

Examples of the C₁-C₃ alkylene group defined by A include methylene, ethylene, propylene and the like groups, and these alkylene groups may have one or two C₁-C₃ alkyl groups such as methyl, ethyl and propyl. In general, A is preferably an unsubstituted alkylene group.

Examples of particularly preferred compounds include
(3S)-3-((S)-4-methyl-2-phenylsulfonylaminovalerylamino)-2-tetrahydrofuranol,
(2S,3S)-2-acetoxy-3-((S)-4-methyl-2-phenylsulfonylaminovalerylamino)tetrahydrofuran,
(3S)-3-{(S)-4-methyl-2-(2,4,6-trimethylphenylsulfonylamino)valerylamino}-2-tetrahydrofuranol,
(2S,3S)-2-acetoxy-3-{(S)-4-methyl-2-(2,4,6-trimethylphenylsulfonylamino)valerylamino}tetrahydrofuran,
(3S)-3-((S)-4-methyl-2-pentadecanoylaminovalerylamino)-2-tetrahydrofuranol,
(2S,3S)-2-acetoxy-3-((S)-4-methyl-2-pentadecanoylaminovalerylamino)tetrahydrofuran,
(3S)-3-((S)-2-hexadecanoylamino-4-methylvalerylamino)-2-tetrahydrofuranol,
(2S,3S)-2-acetoxy-3-((S)-2-hexadecanoylamino-4-methylvalerylamino)tetrahydrofuran,
(3S)-3-((S)-2-heptadecanoylamino-4-methylvalerylamino)-2-tetrahydrofuranol,
(2S,3S)-2-acetoxy-3-((S)-2-heptadecanoylamino-4-methylvalerylamino)tetrahydrofuran,
(3S)-3-((S)-4-methyl-2-tridecyloxycarbonylaminovalerylamino)-2-tetrahydrofuranol,
(2S,3S)-2-acetoxy-3-((S)-4-methyl-2-tridecyloxycarbonylaminovalerylamino)tetrahydrofuran,
(3S)-3-((S)-4-methyl-2-tetradecyloxycarbonylaminovalerylamino)-2-tetrahydrofuranol,
(2S,3S)-2-acetoxy-3-((S)-4-methyl-2-tetradecyloxycarbonylaminovalerylamino)tetrahydrofuran,
(3S)-3-((S)-4-methyl-2-pentadecyloxycarbonylaminovalerylamino)-2-tetrahydrofuranol,
(2S,3S)-2-acetoxy-3-((S)-4-methyl-2-pentadecyloxycarbonylaminovalerylamino)tetrahydrofuran,
(3S)-3-((S)-4-methyl-2-tetradecylsulfonylaminovalerylamino)-2-tetrahydrofuranol,
(2S,3S)-2-acetoxy-3-((S)-4-methyl-2-tetradecylsulfonylaminovalerylamino)tetrahydrofuran,
(3S)-3-((S)-4-methyl-2-pentadecylsulfonylaminovalerylamino)-2-tetrahydrofuranol,
(2S,3S)-2-acetoxy-3-((S)-4-methyl-2-pentadecylsulfonylaminovalerylamino)tetrahydrofuran,
(3S)-3-((S)-2-hexadecylsulfonylamino-4-methylvalerylamino)-2-tetrahydrofuranol,
(2S,3S)-2-acetoxy-3-((S)-2-hexadecylsulfonylamino-4-methylvalerylamino)tetrahydrofuran,
(3S)-4-methyl-3-((S)-4-methyl-2-phenylsulfonylaminovalerylamino)-2-tetrahydrofuranol,
and salts thereof, solvates thereof and hydrates thereof.

A compound represented by the following formula (A) is known as a compound related to the compound represented by the aforementioned general formula (I) (JP-A-8-104685; the term "JP-A" as used herein means an "unexamined published Japanese patent application").

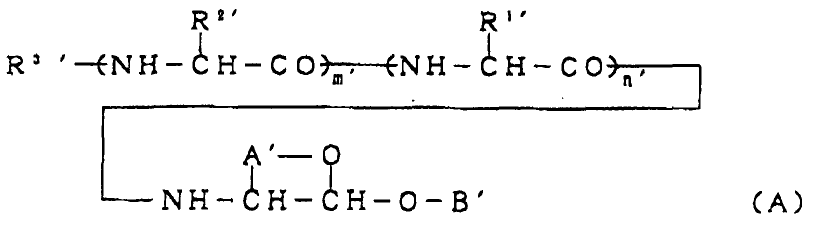

In the above general formula, R^{1'} and R^{2'} may be the same or different from each other and each represents hydrogen atom or a hydrocarbon radical which may be substituted, R^{3'} represents carboxyl group which may be esterificated or an acyl group, A' represents an alkylene group, B' represents hydrogen atom, an alkyl group which may be substituted or an acyl group, and each of m' and n' is 0 or 1, with the proviso that when m' and n' are both 0, R^{3'} represents carboxyl group which may be esterificated or an acyl group, having 7 or more carbon atoms.

However, only a cathepsin L inhibitor, a bone resorption inhibitor and a drug for the prevention and treatment of osteoporosis are described in the aforementioned patent as the use of the compound disclosed therein, and it is not known that the compound represented by the aforementioned general formula (I) can be used as a drug for the treatment of ischemic diseases.

The ischemic disease treating drug of the present invention represented by the aforementioned general formula (I) can be used as a pharmaceutically acceptable salt. As illustrative examples of such salt, lithium salt, sodium salt, potassium salt, magnesium salt, calcium salt and the like metal sales or ammonium salt, methylammonium salt, dimethylammonium salt, trimethylammonium salt, dicyclohexylammonium salt and the like ammonium salts can be formed. Also, the ischemic disease treating drug of the present invention represented by the aforementioned general formula (I) can be used as a solvate or hydrate.

With regard to the stereochemistry of asymmetric carbon atoms existing in the ischemic disease treating drug of the present invention represented by the aforementioned general formula (I), they can each independently take (R) isomer, (S) isomer or (RS) isomer.

Illustrative examples of the ischemic disease treating drug of the present invention represented by the aforementioned general formula (I) include those compounds shown in the following Table 1 when R³ is hydrogen atom and those compounds shown in the following Table 2 when R³ is not hydrogen atom.

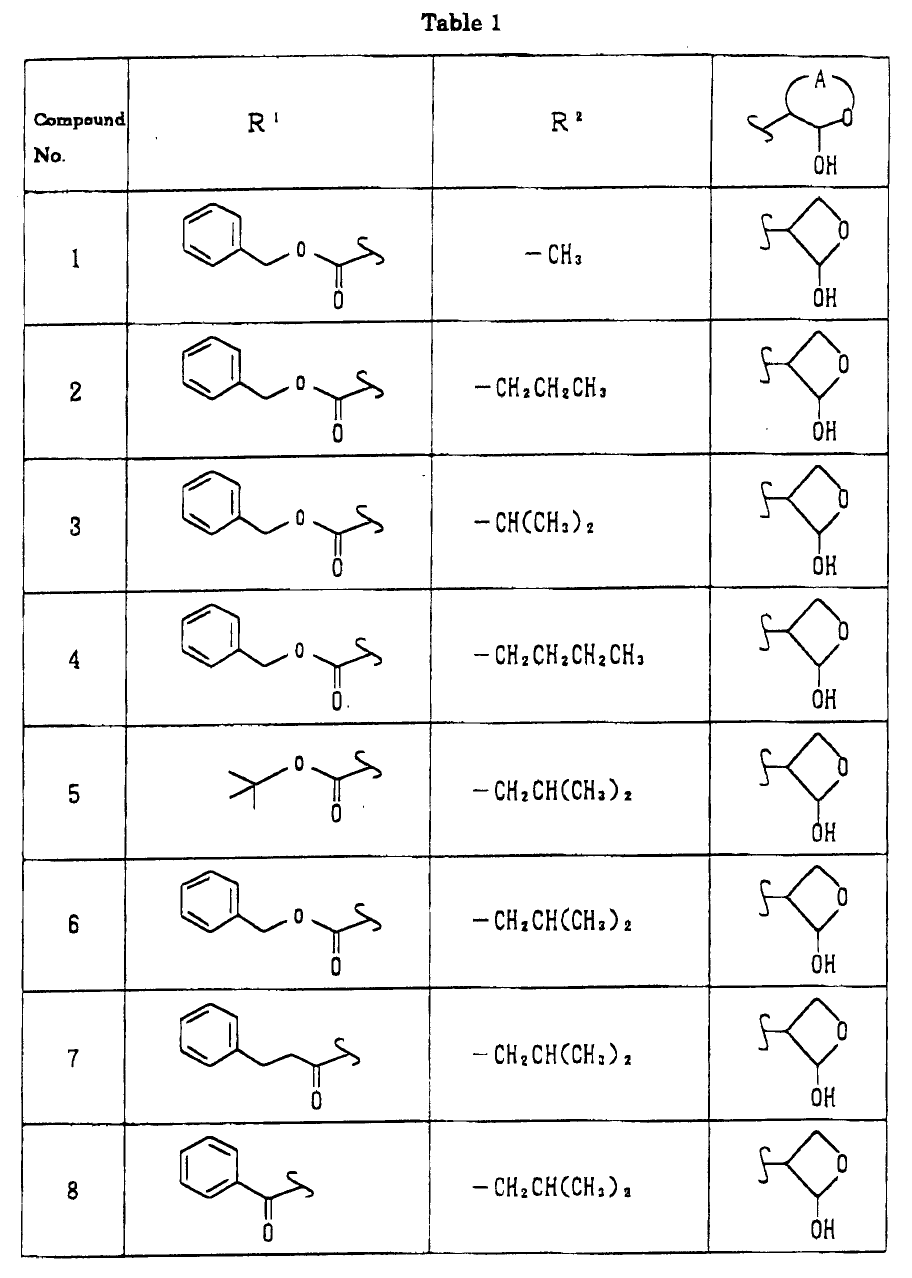

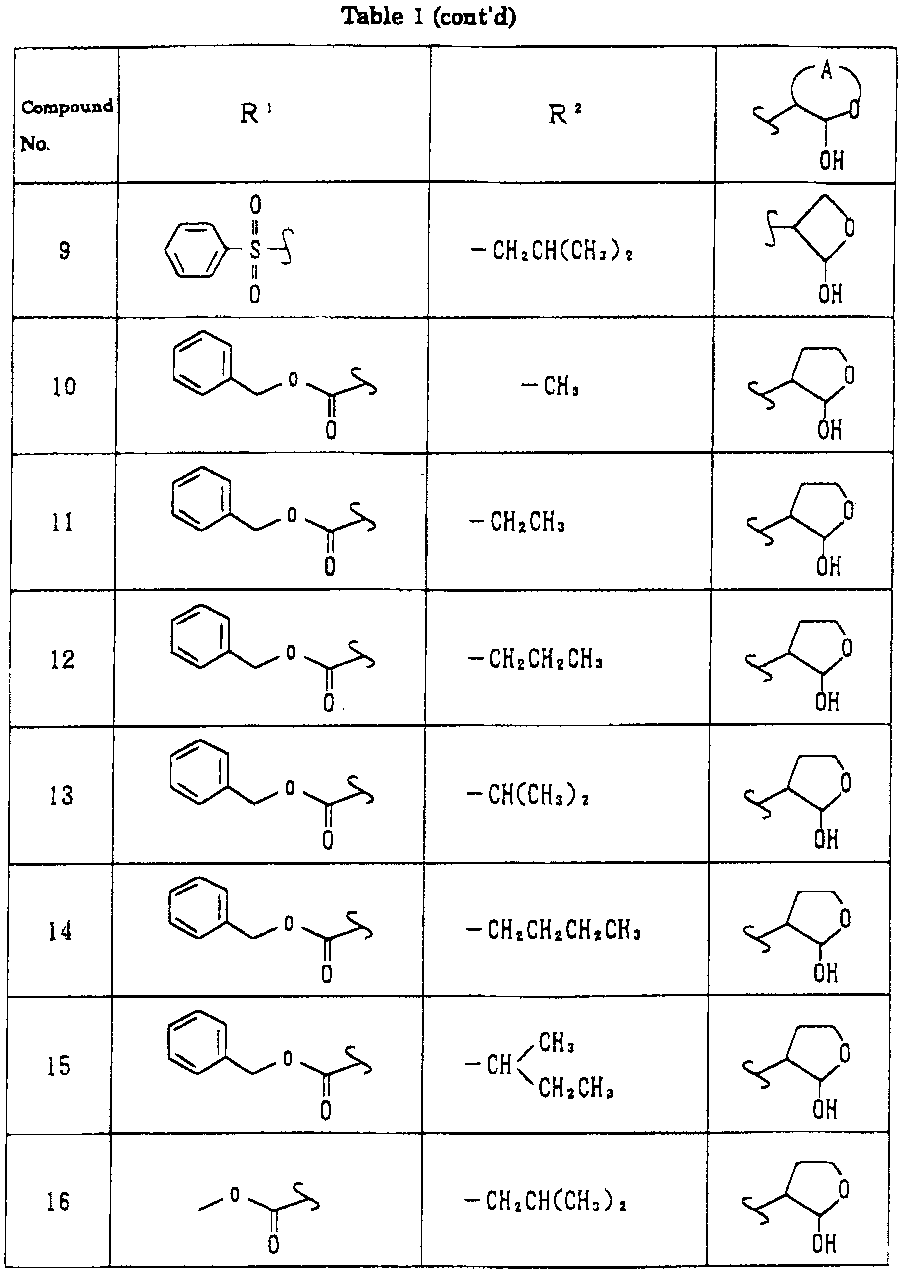

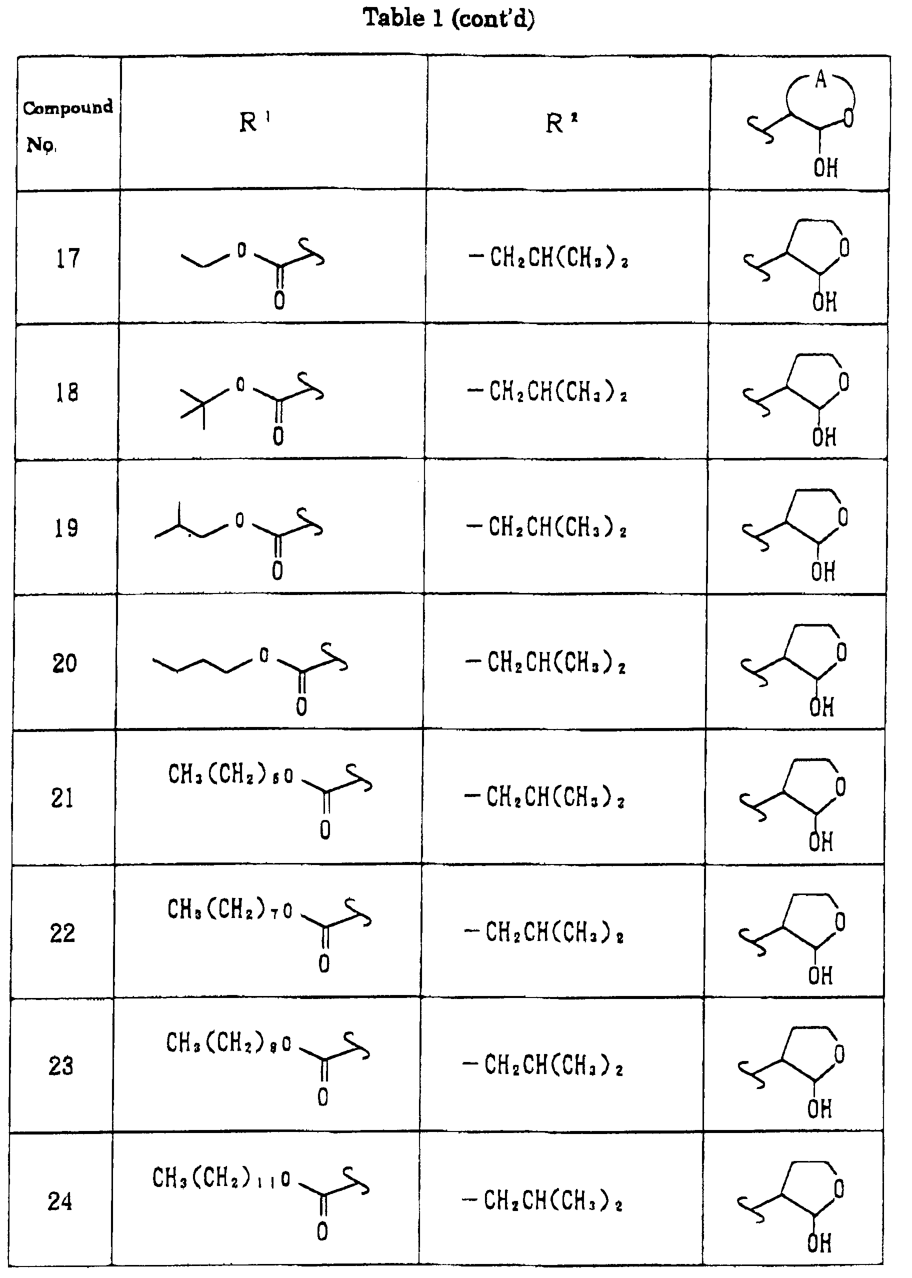

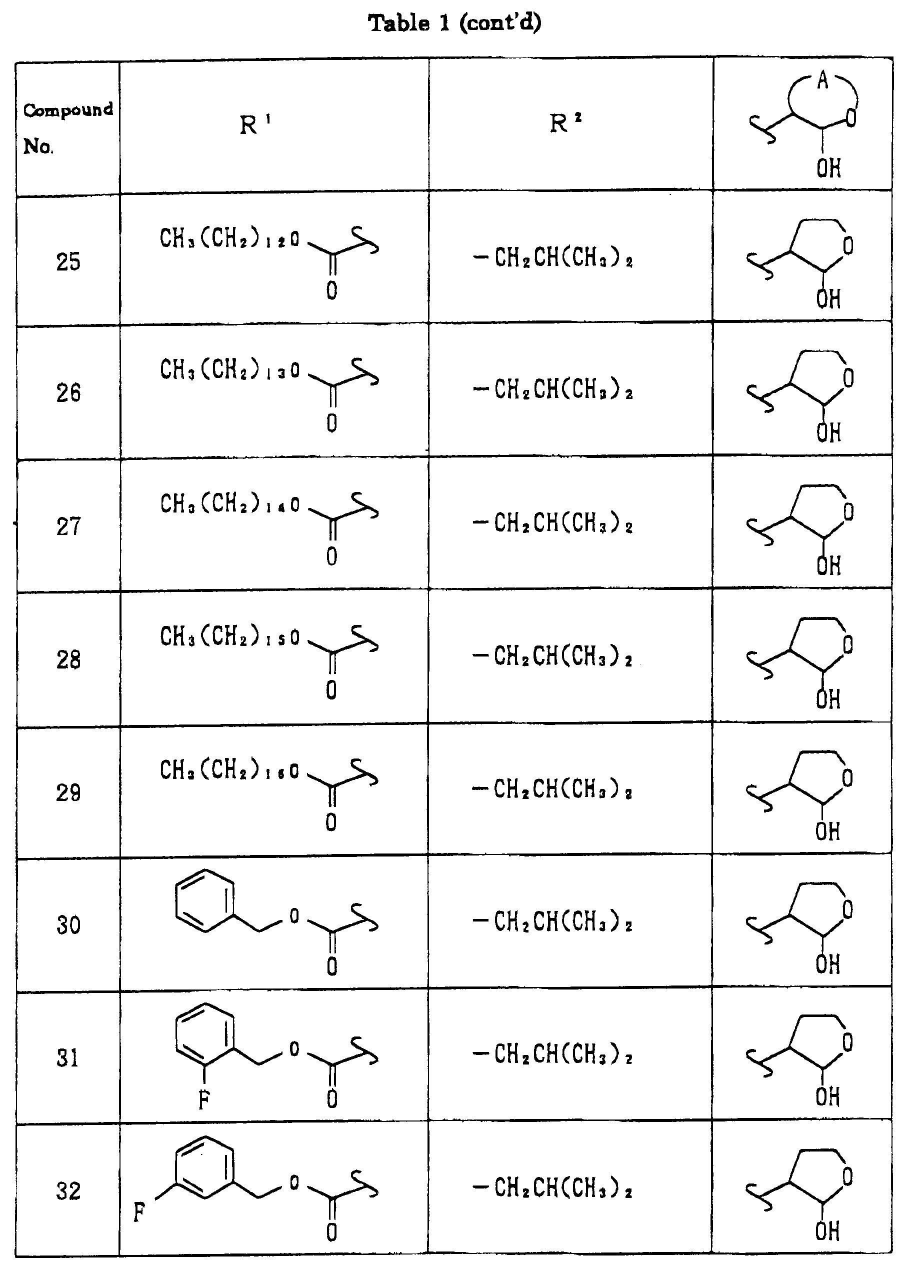

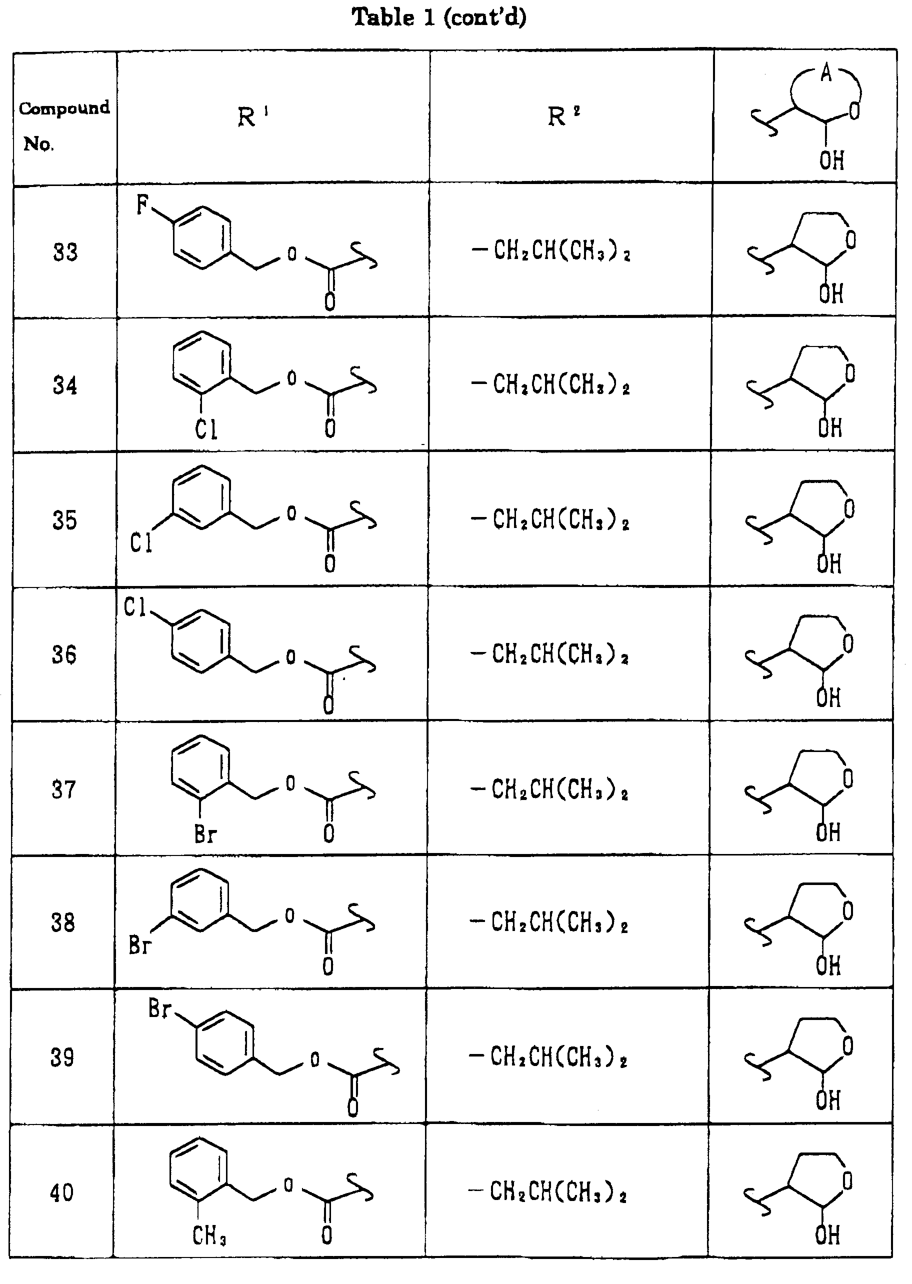

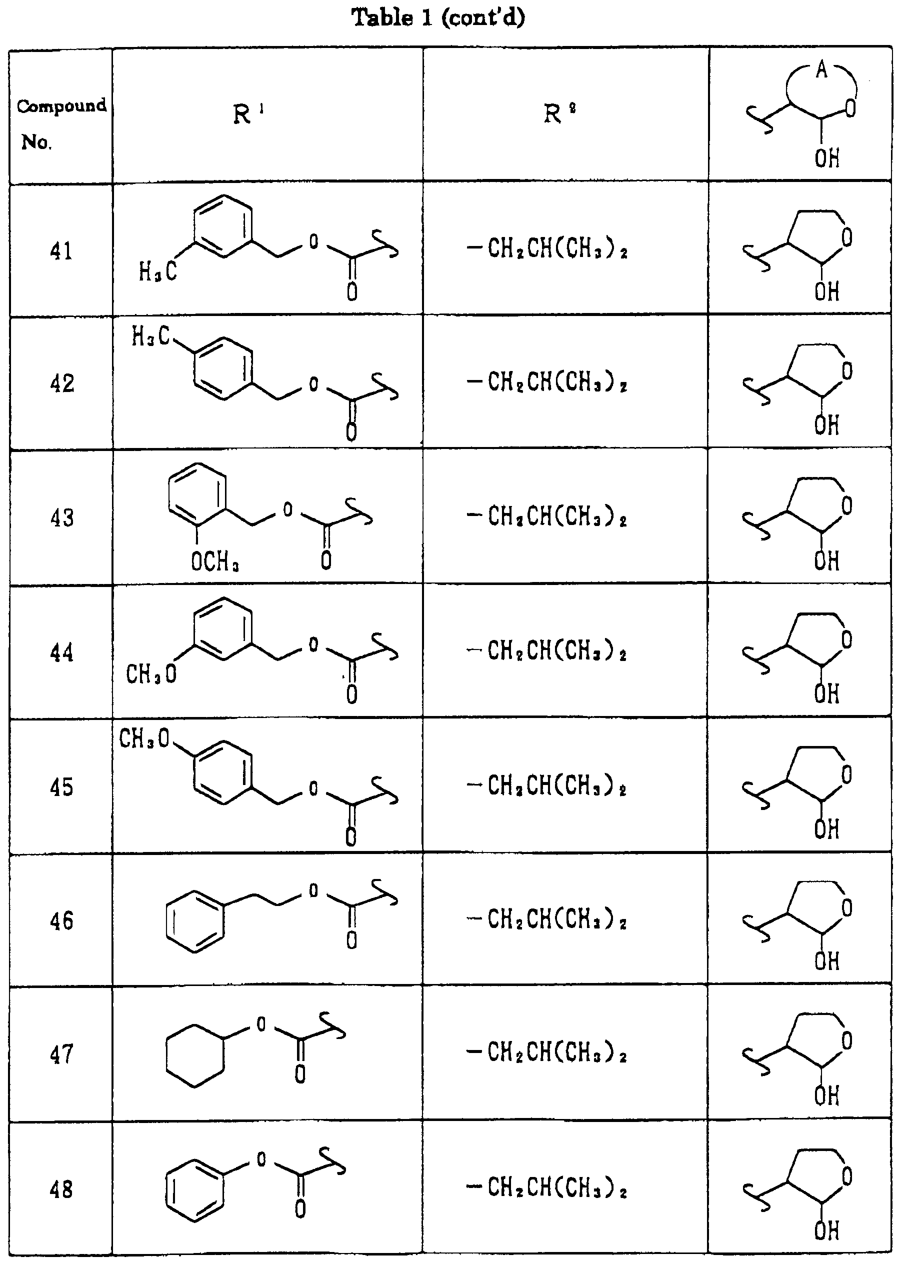

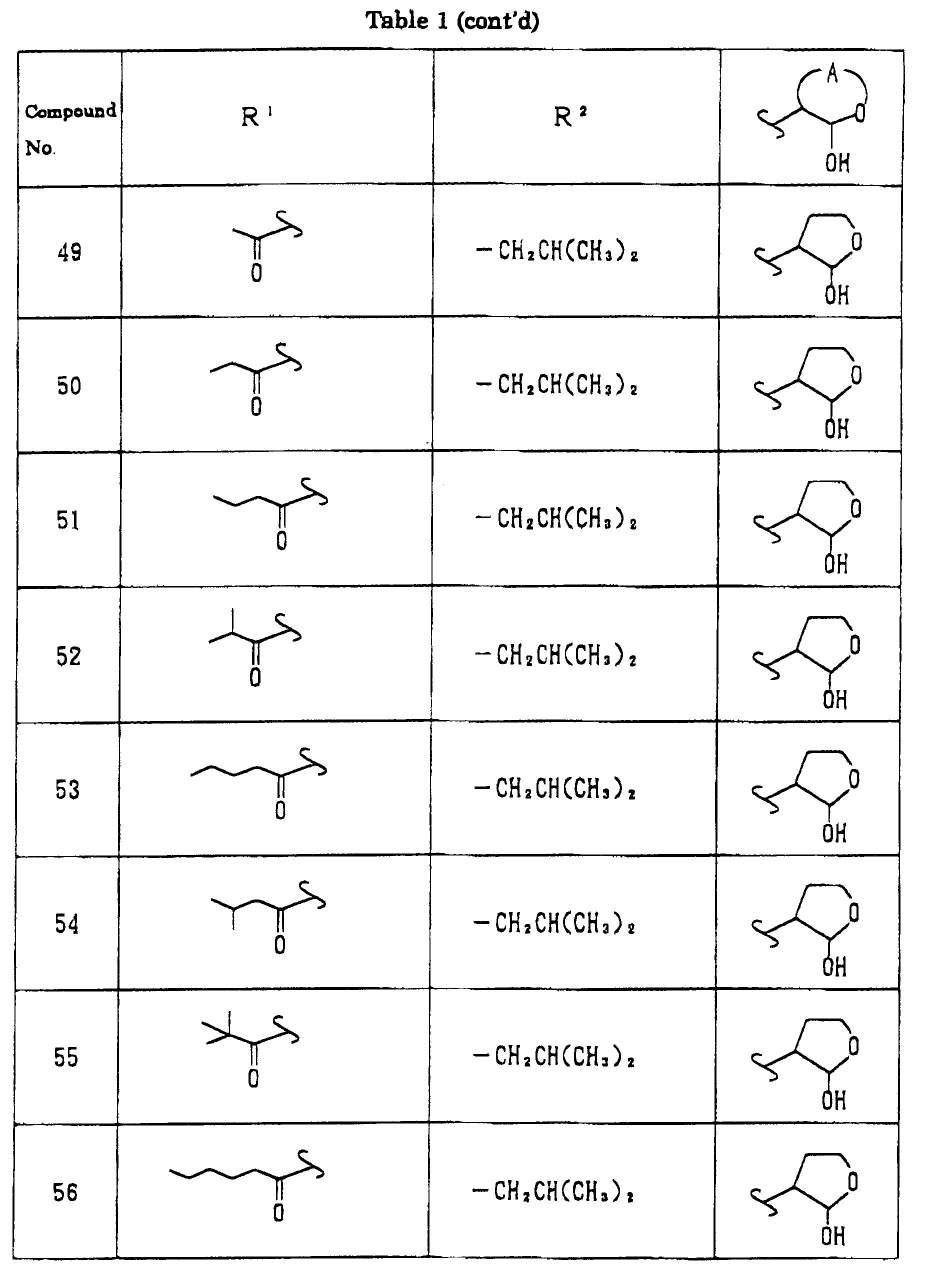

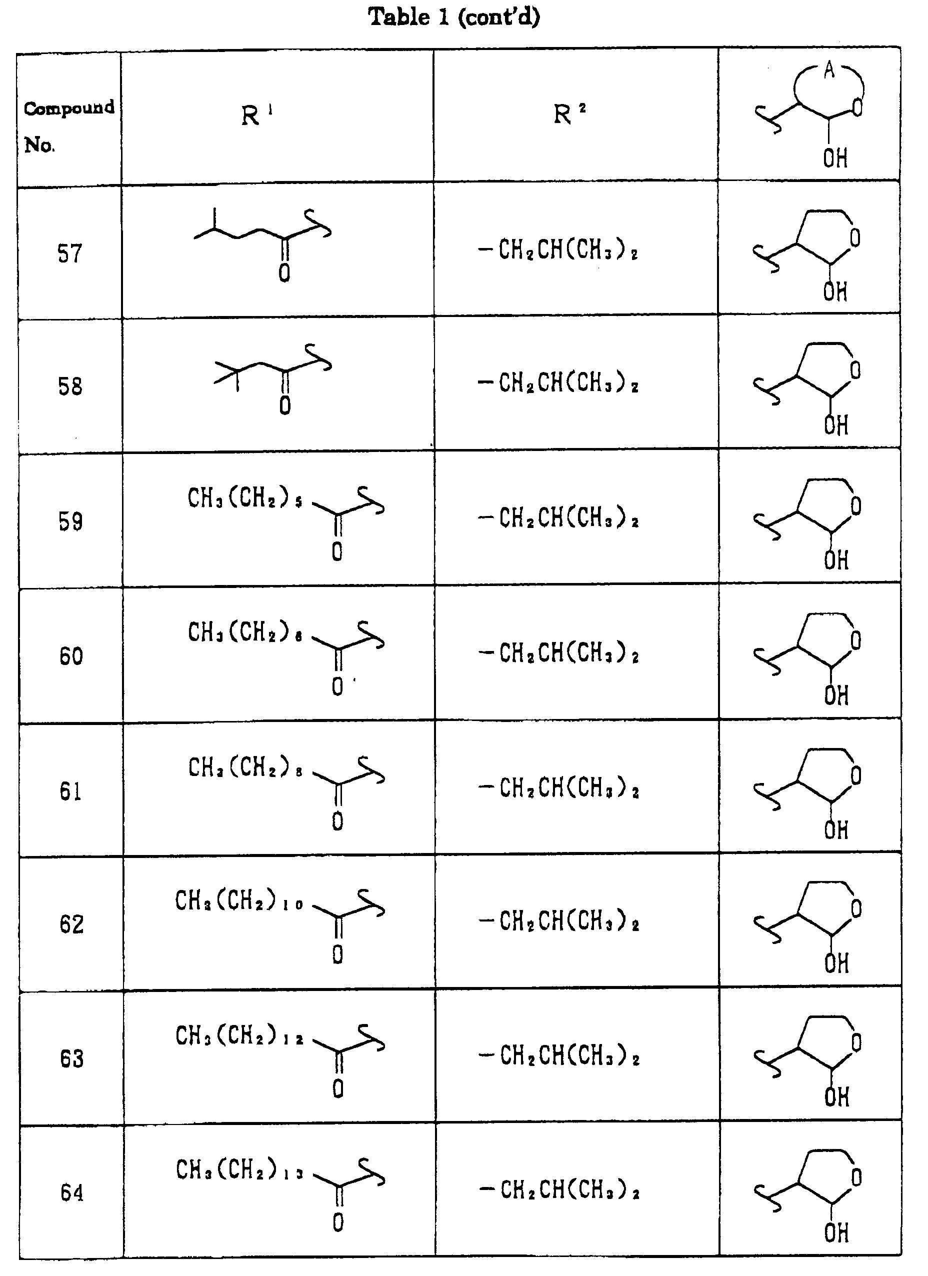

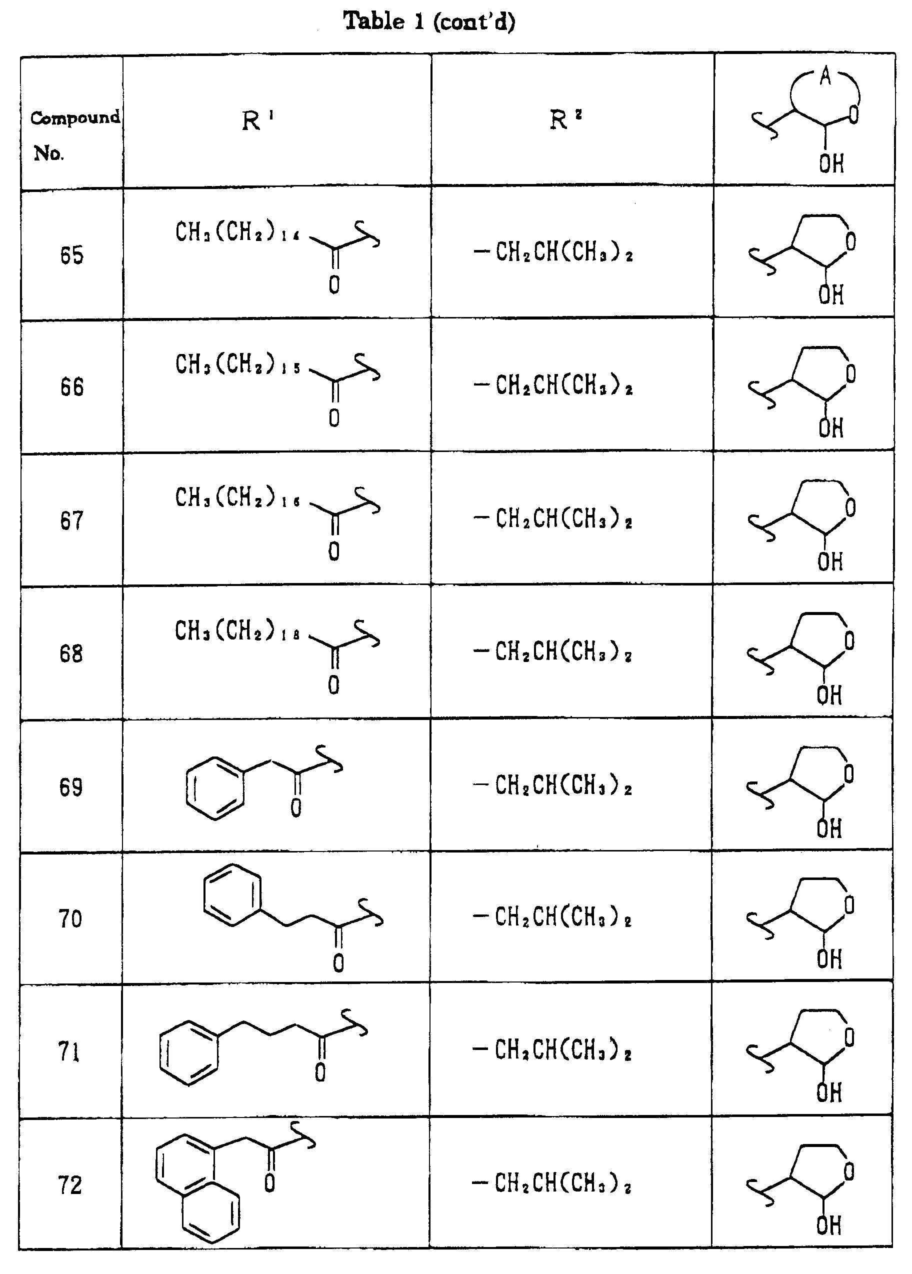

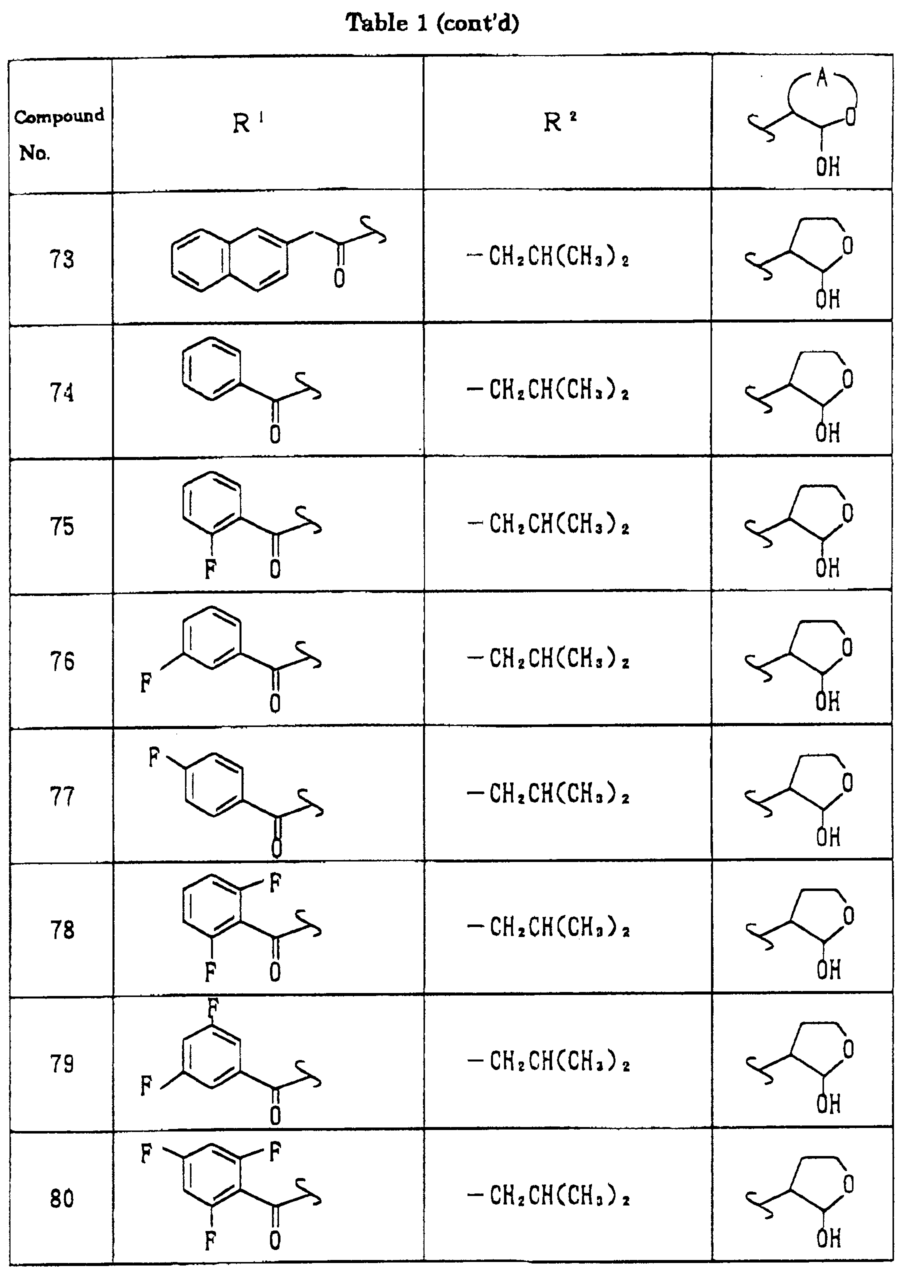

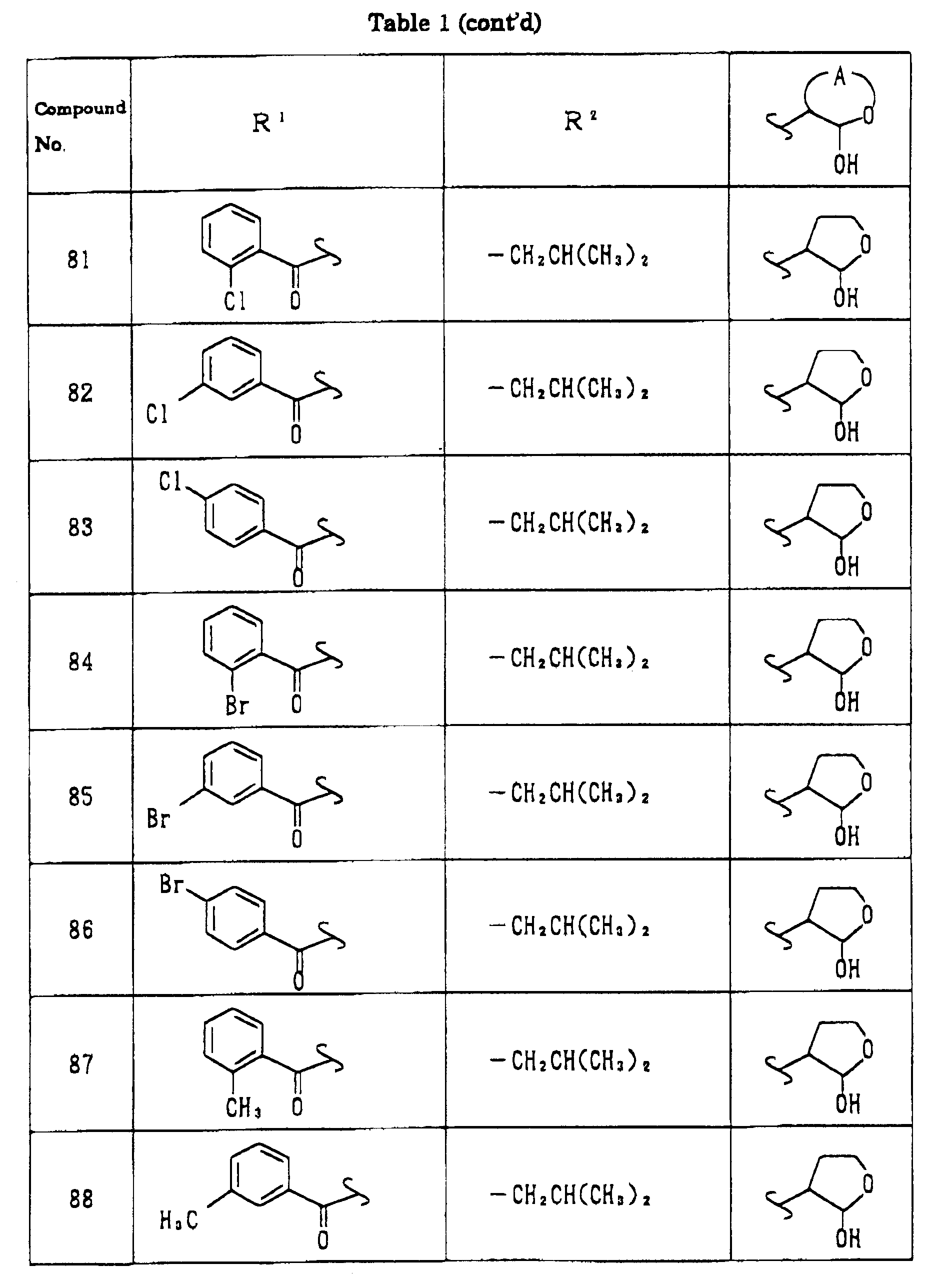

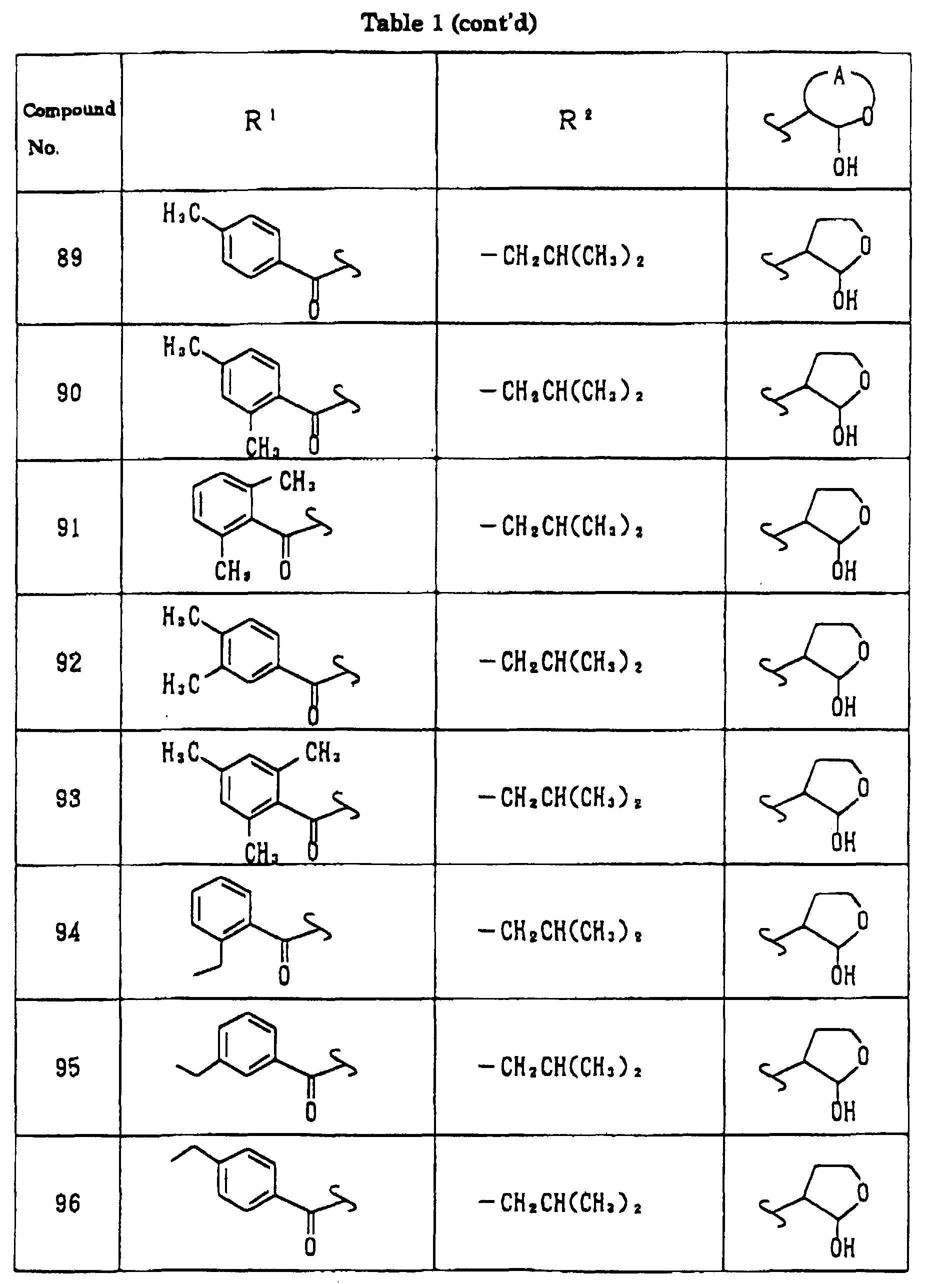

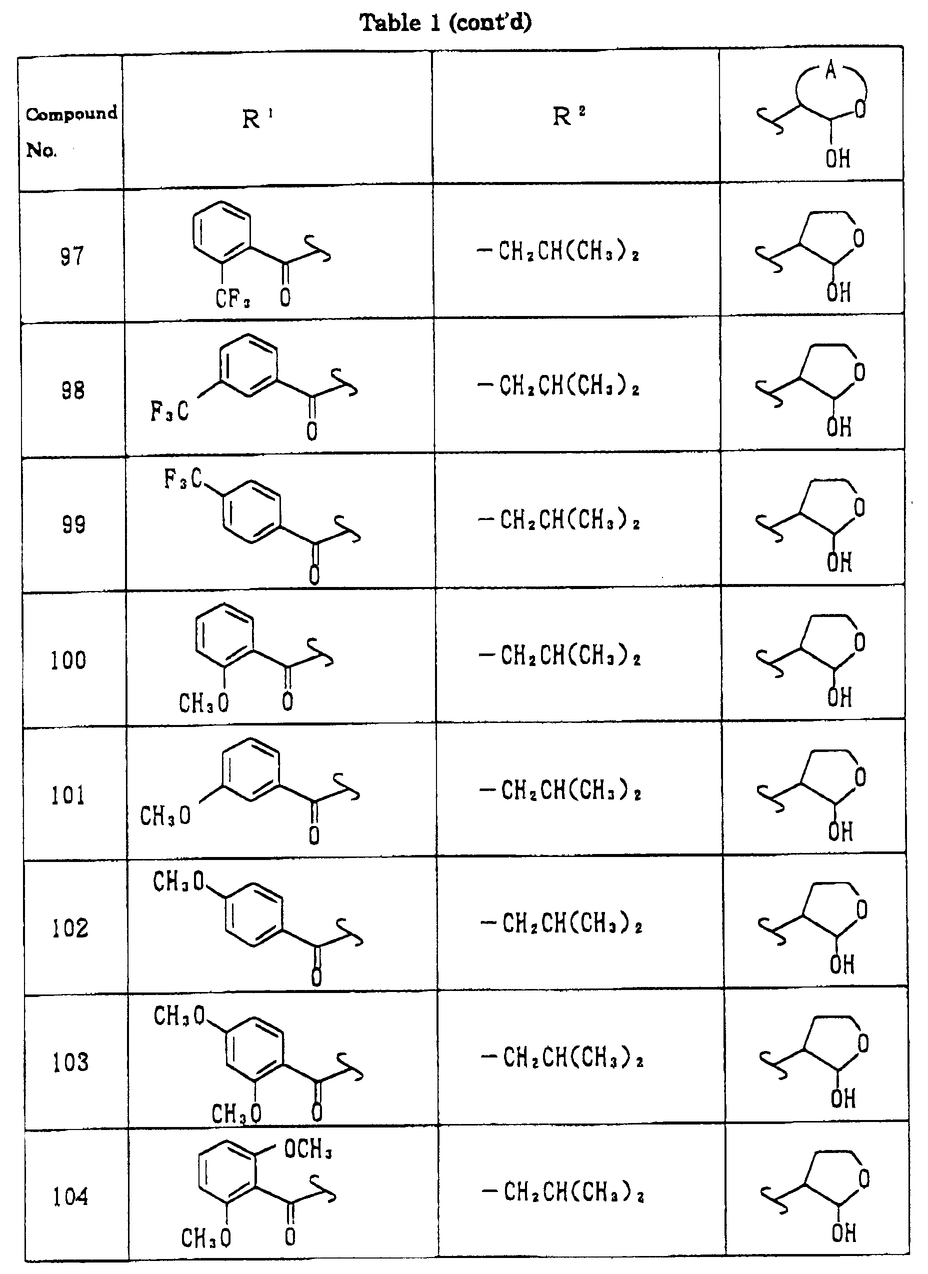

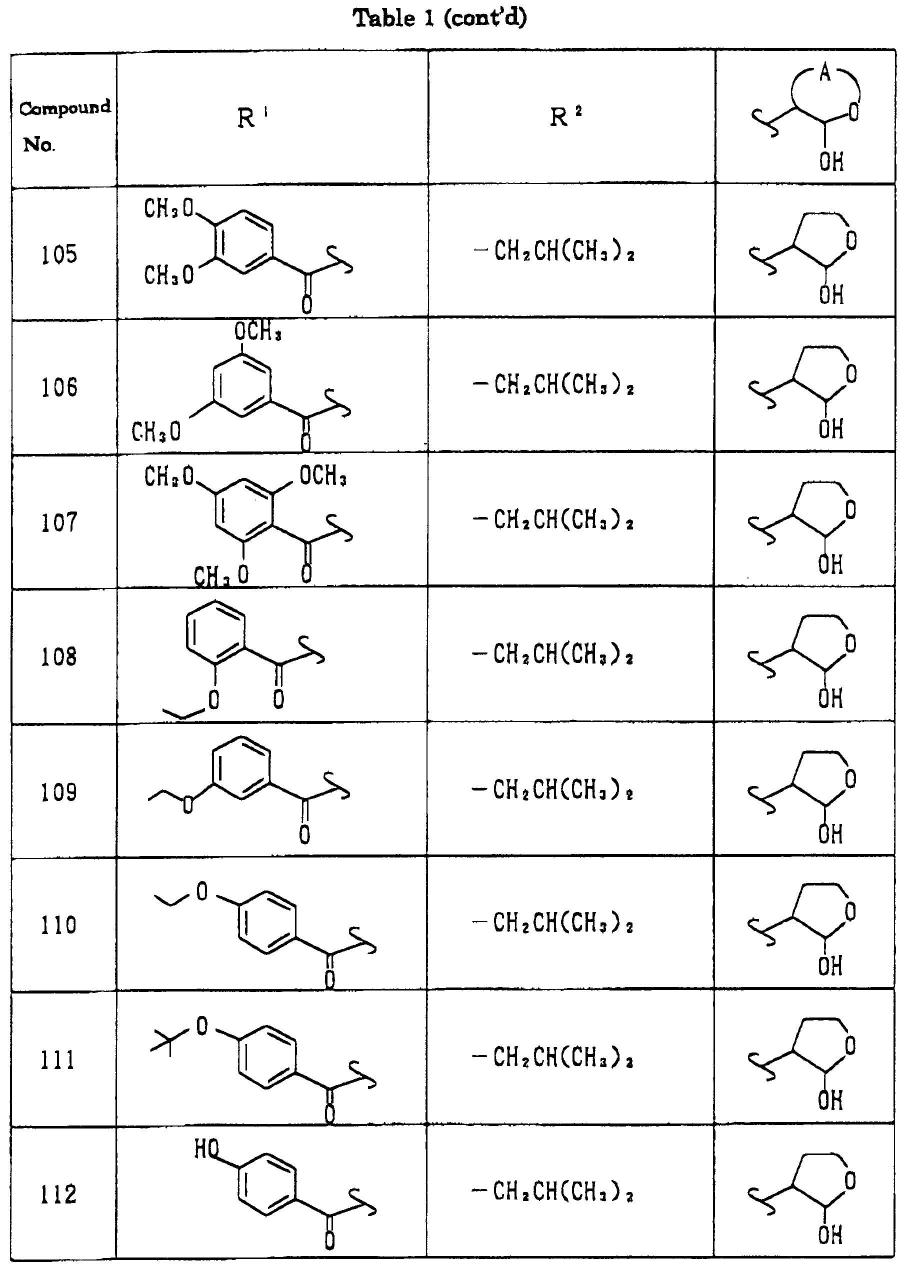

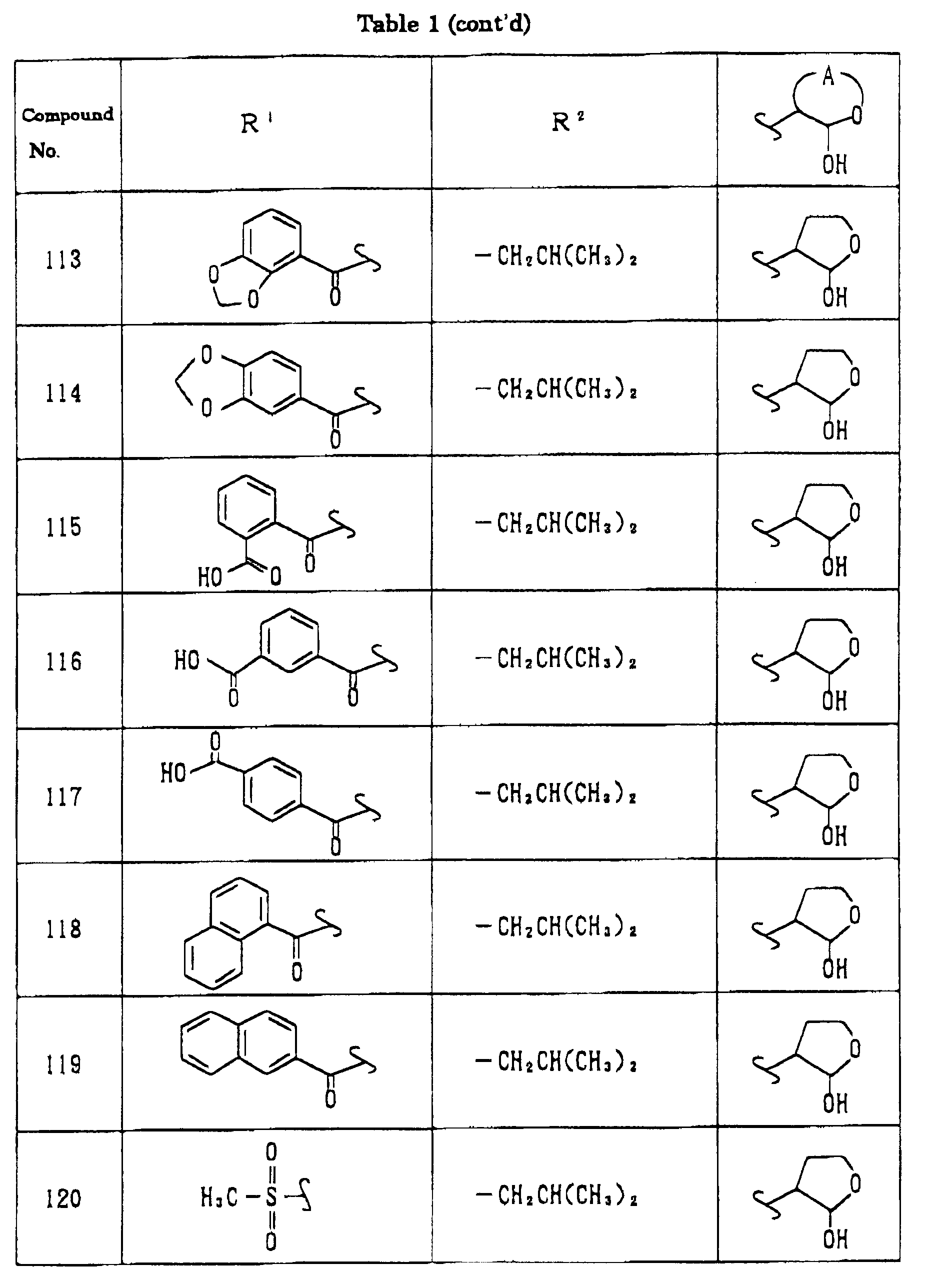

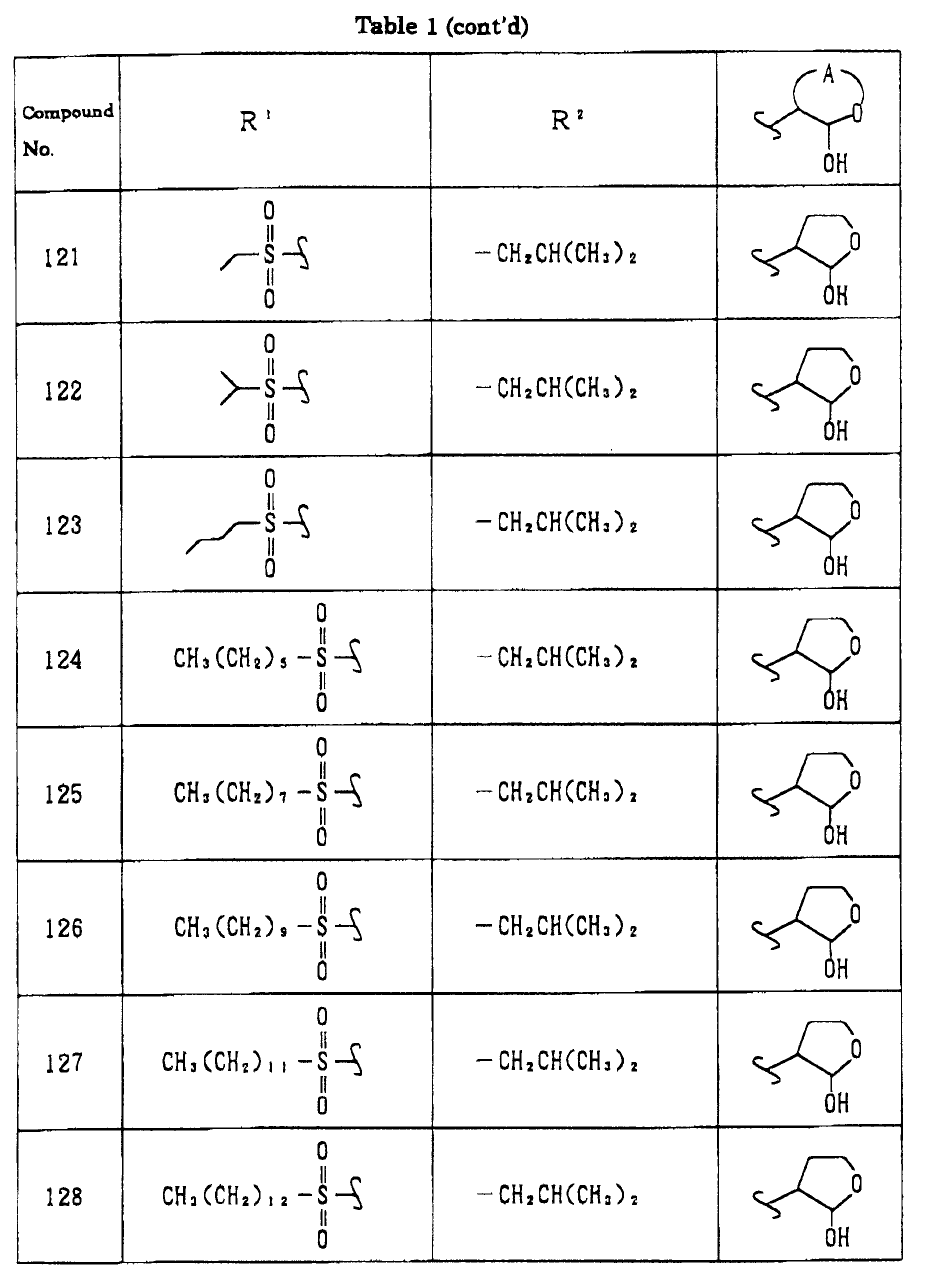

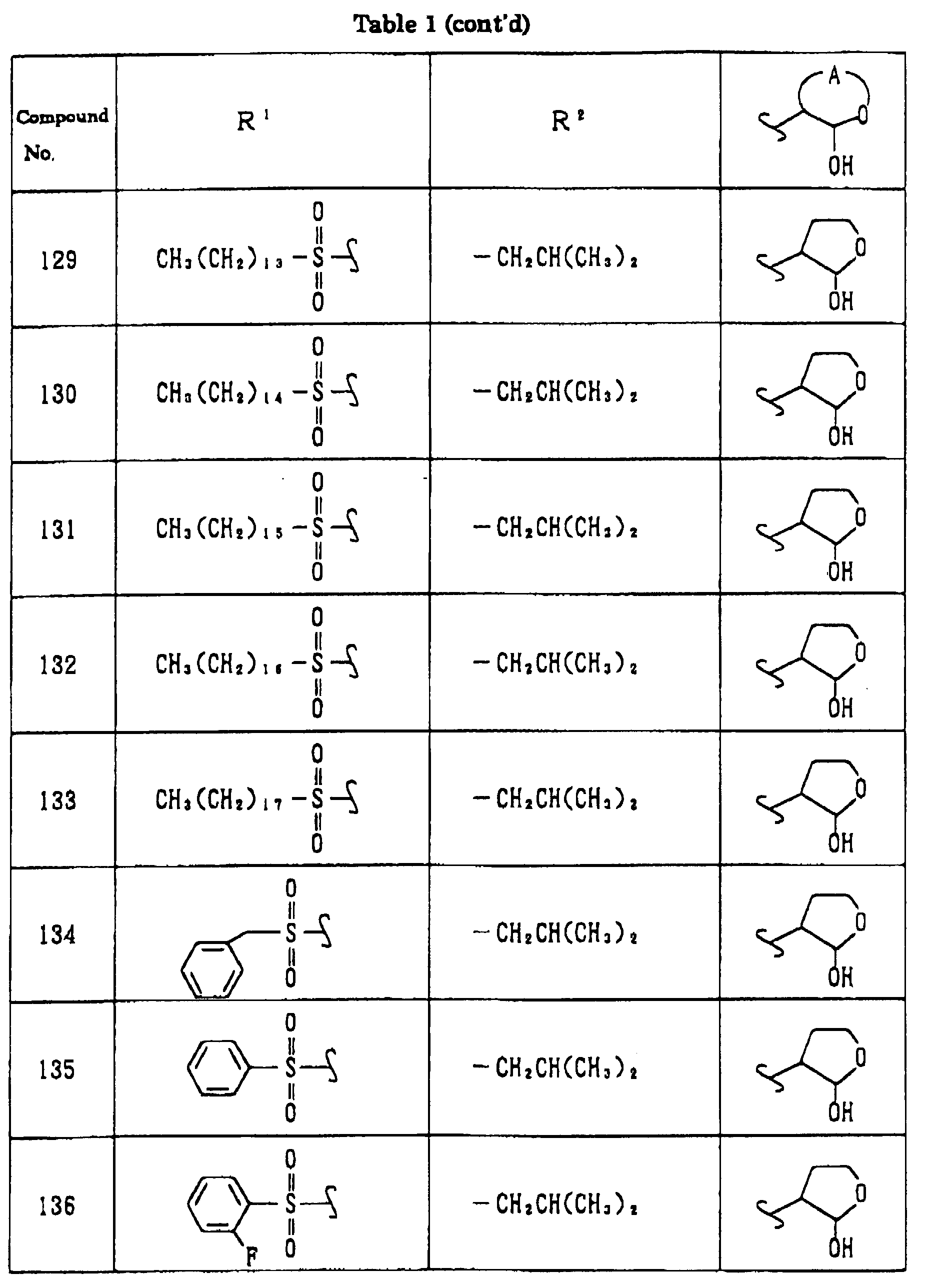

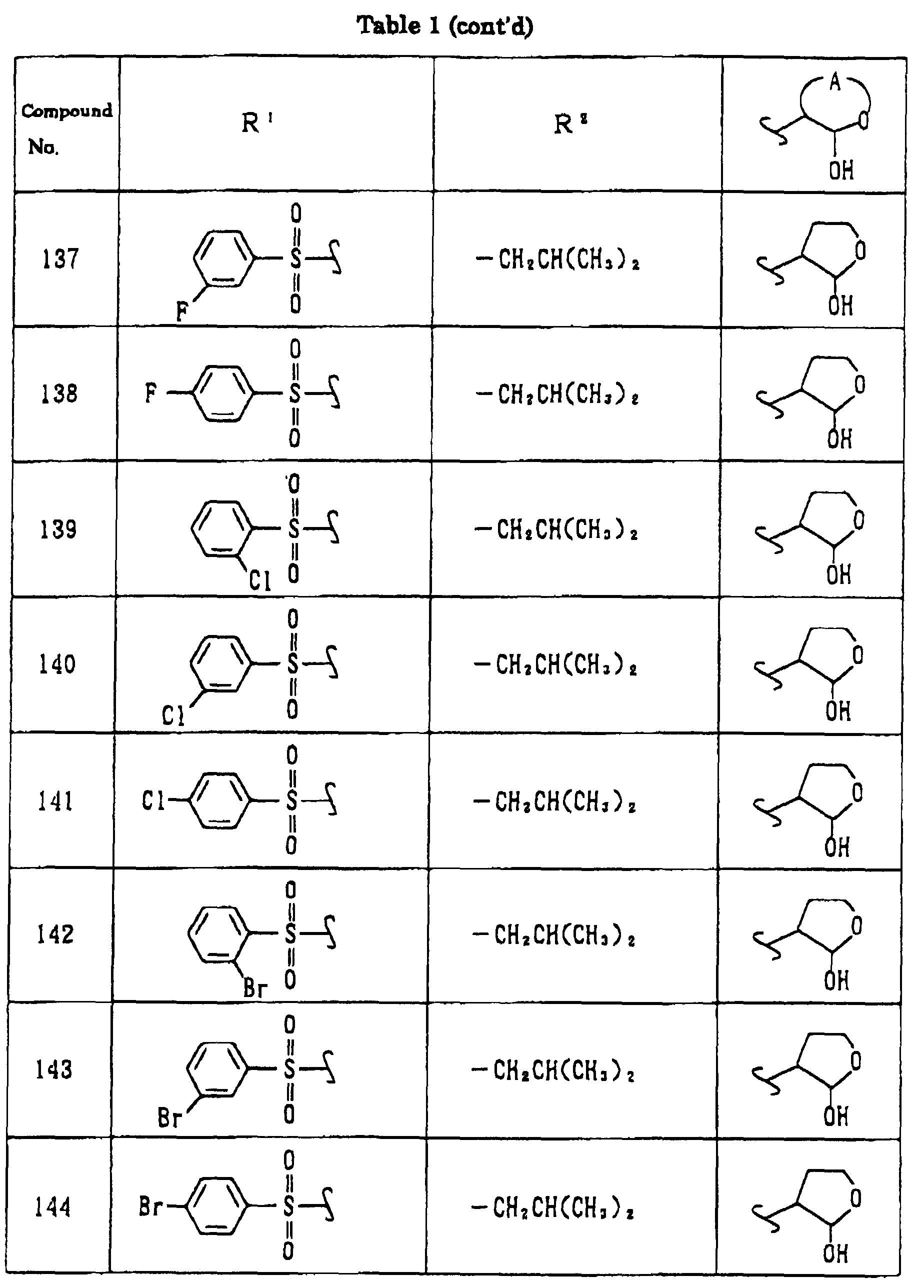

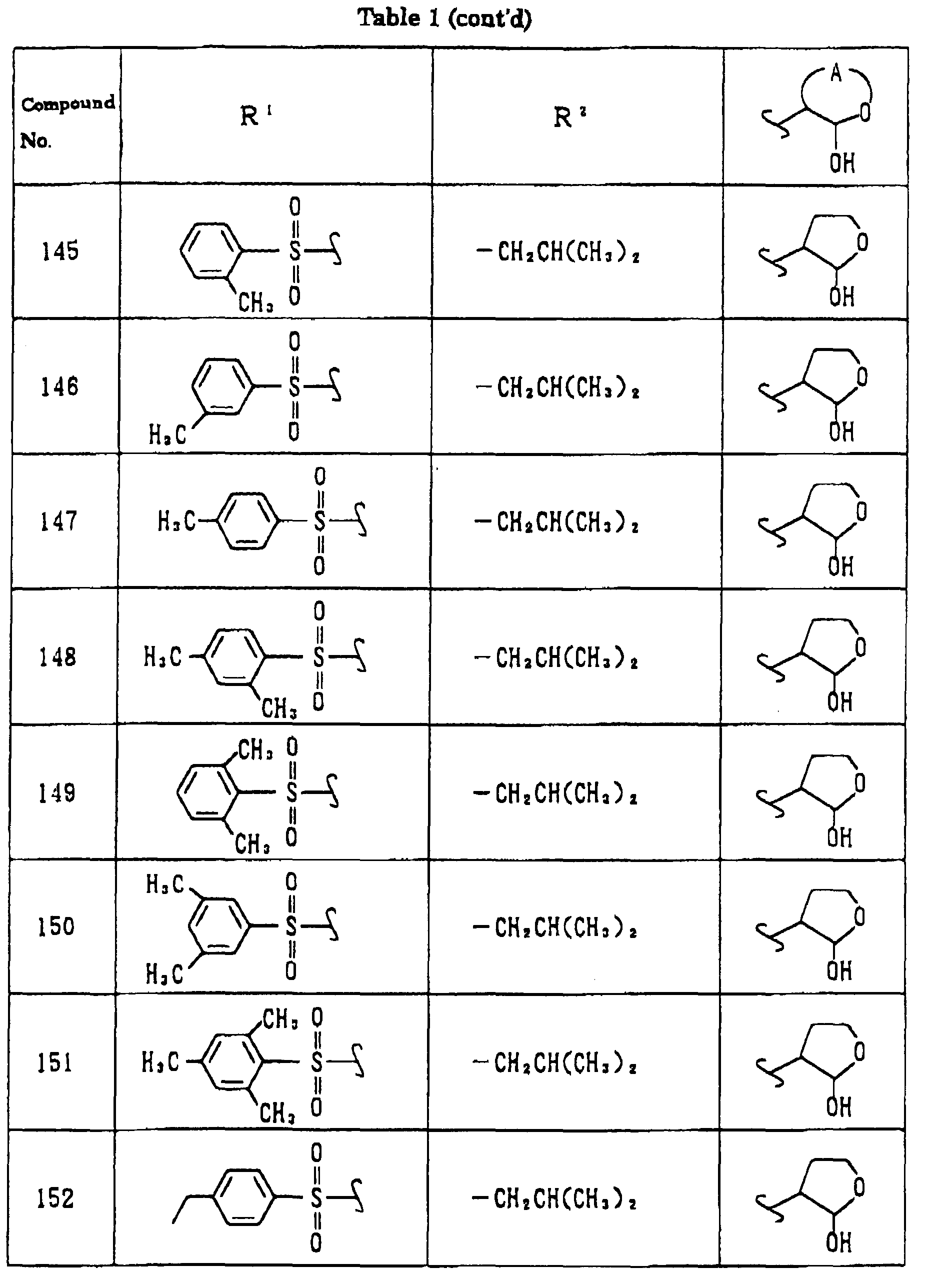

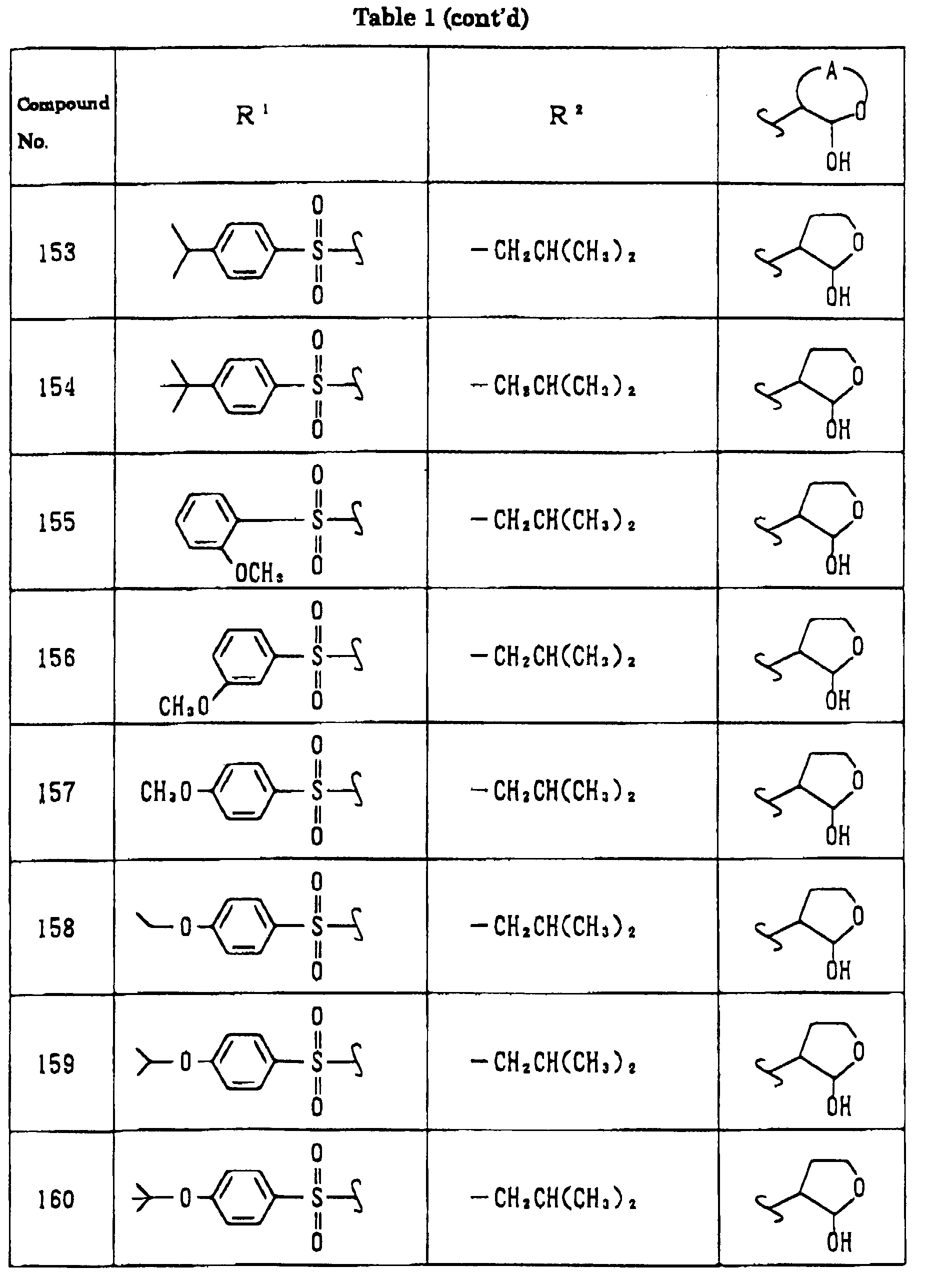

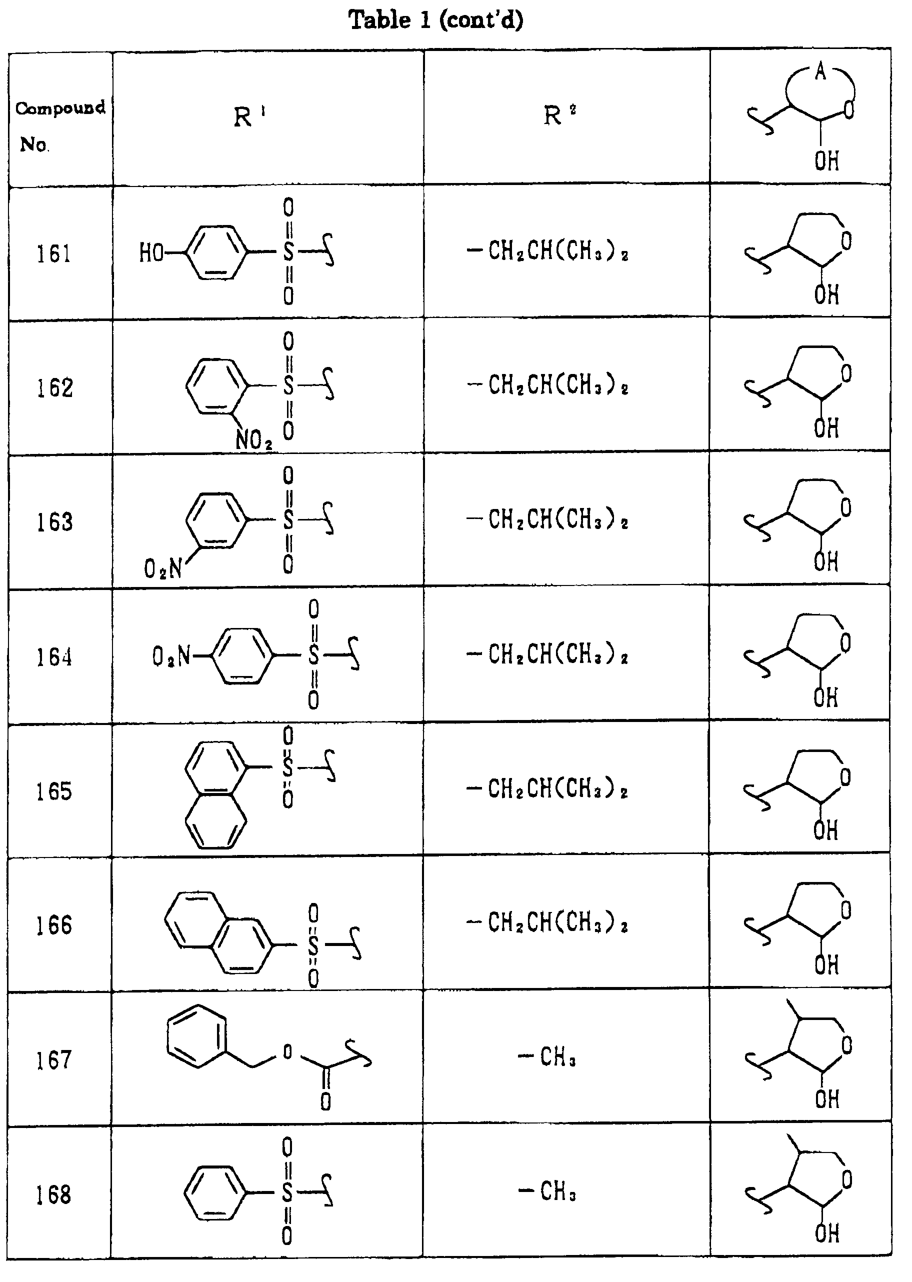

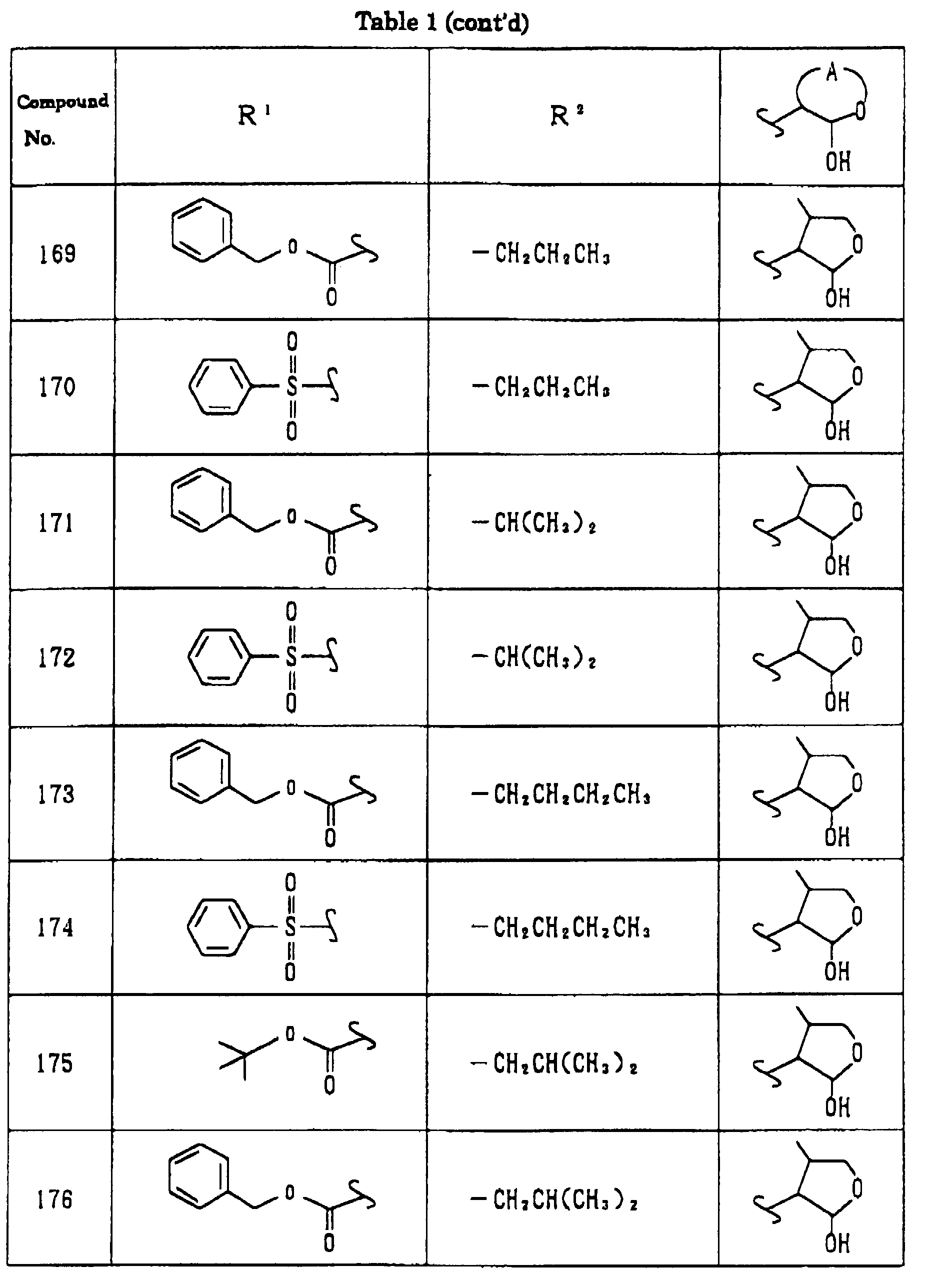

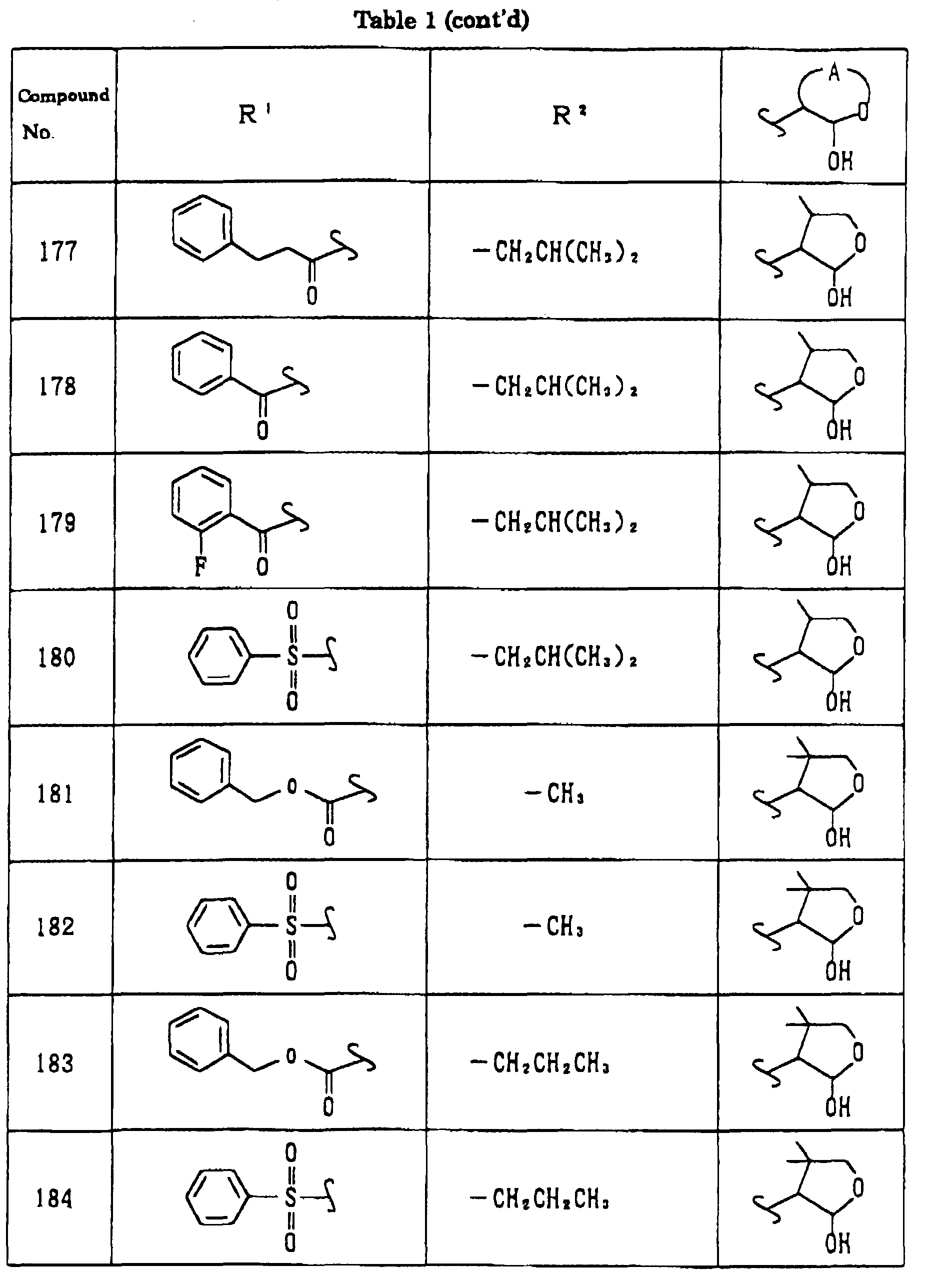

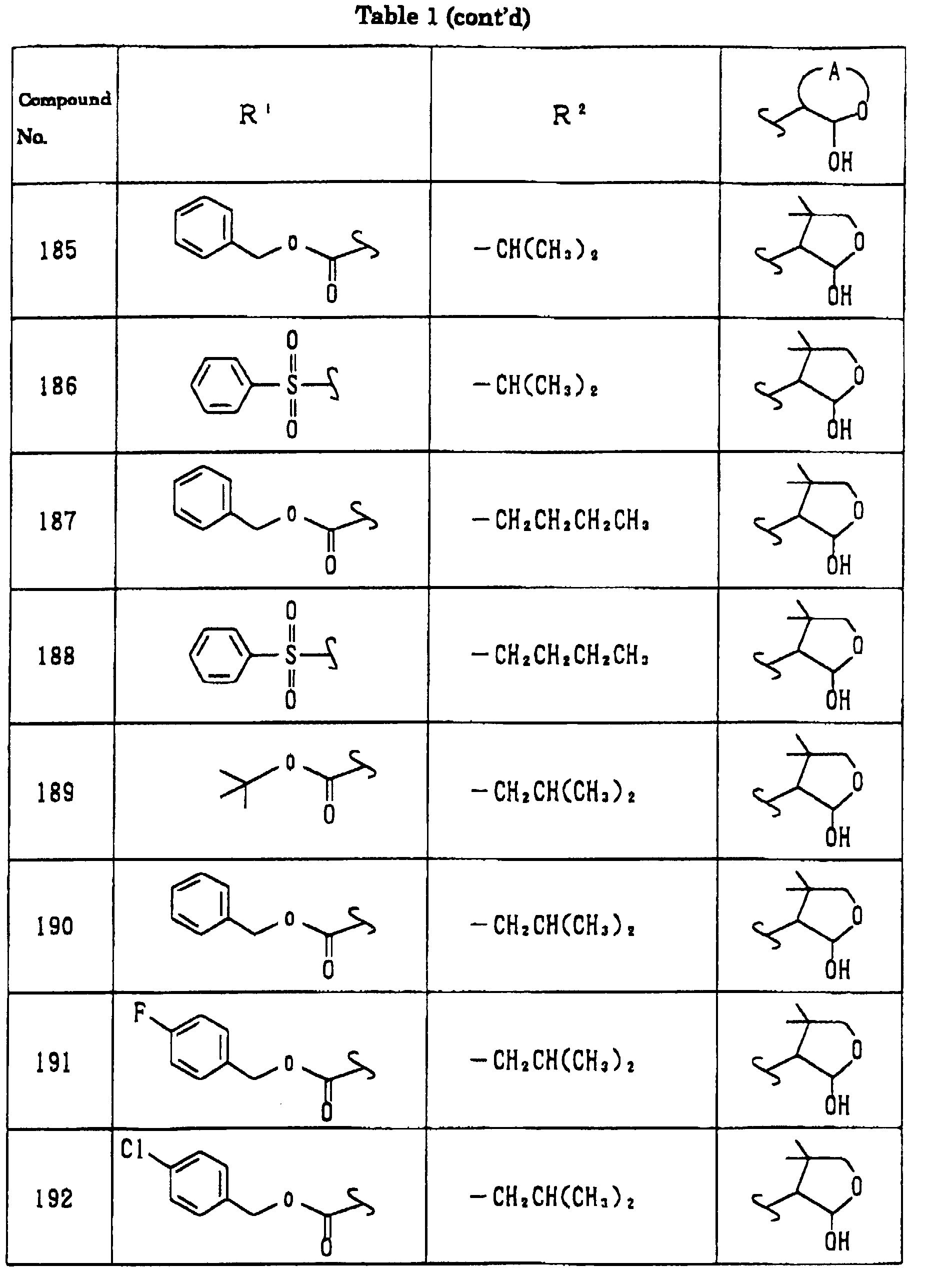

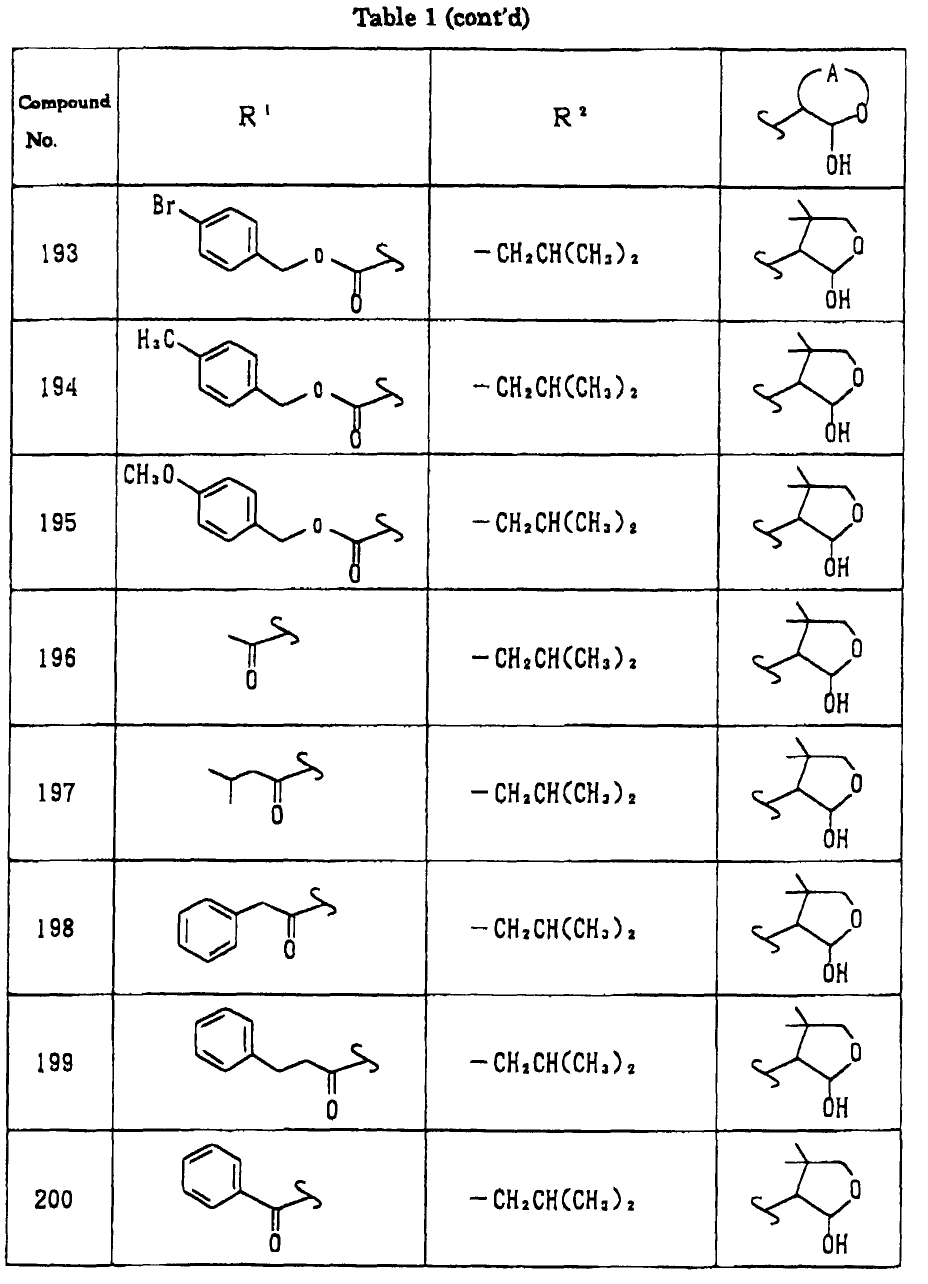

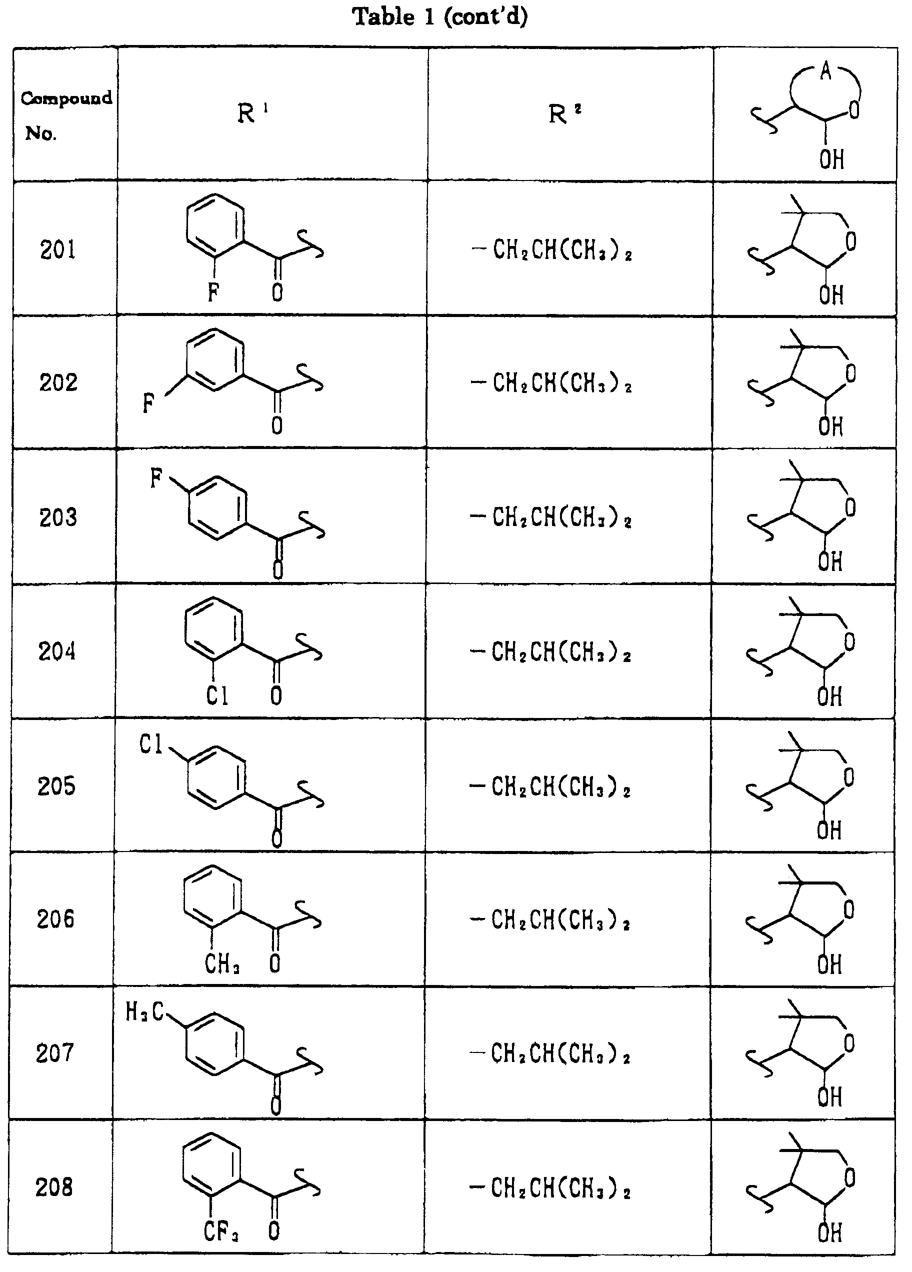

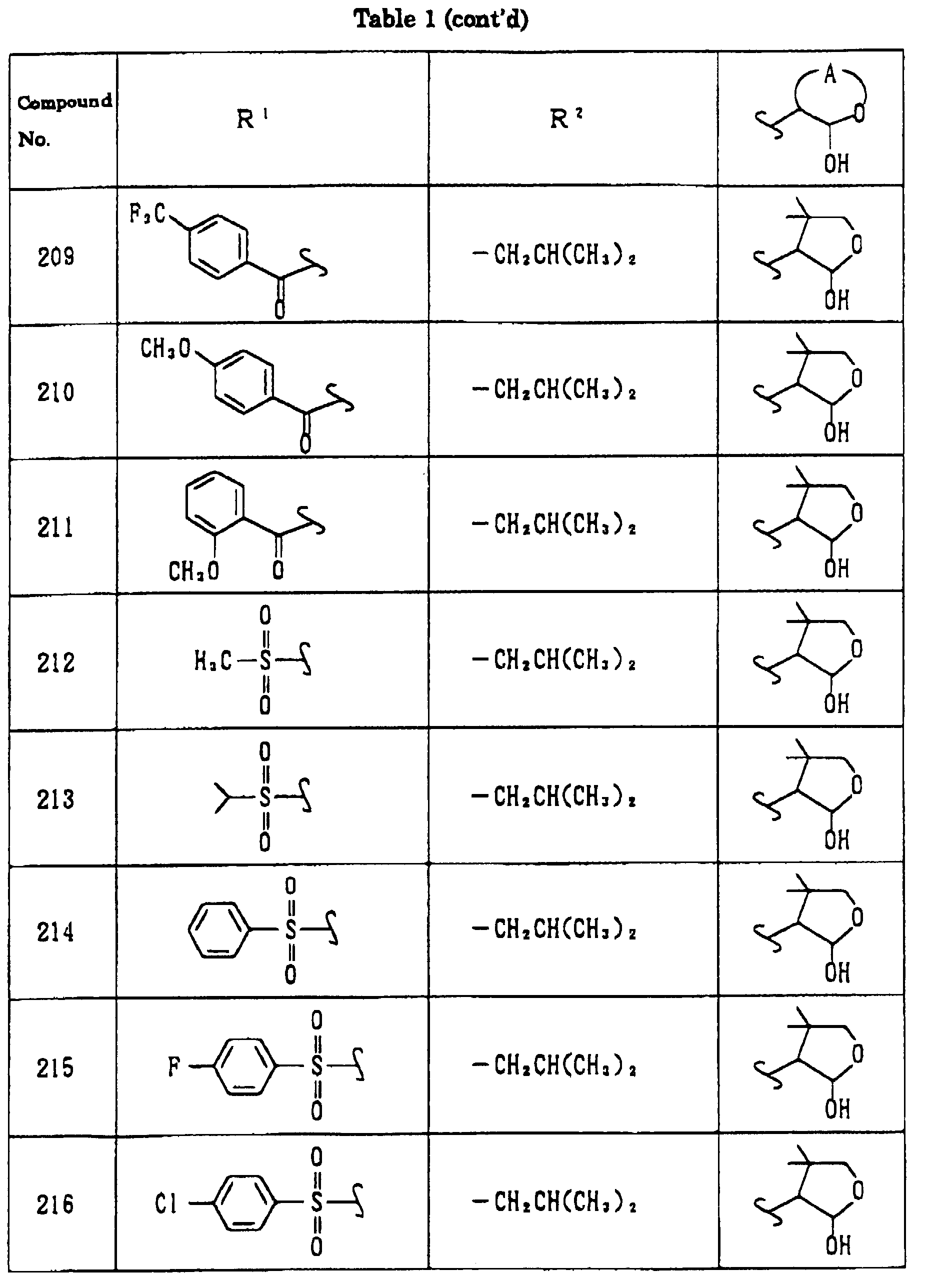

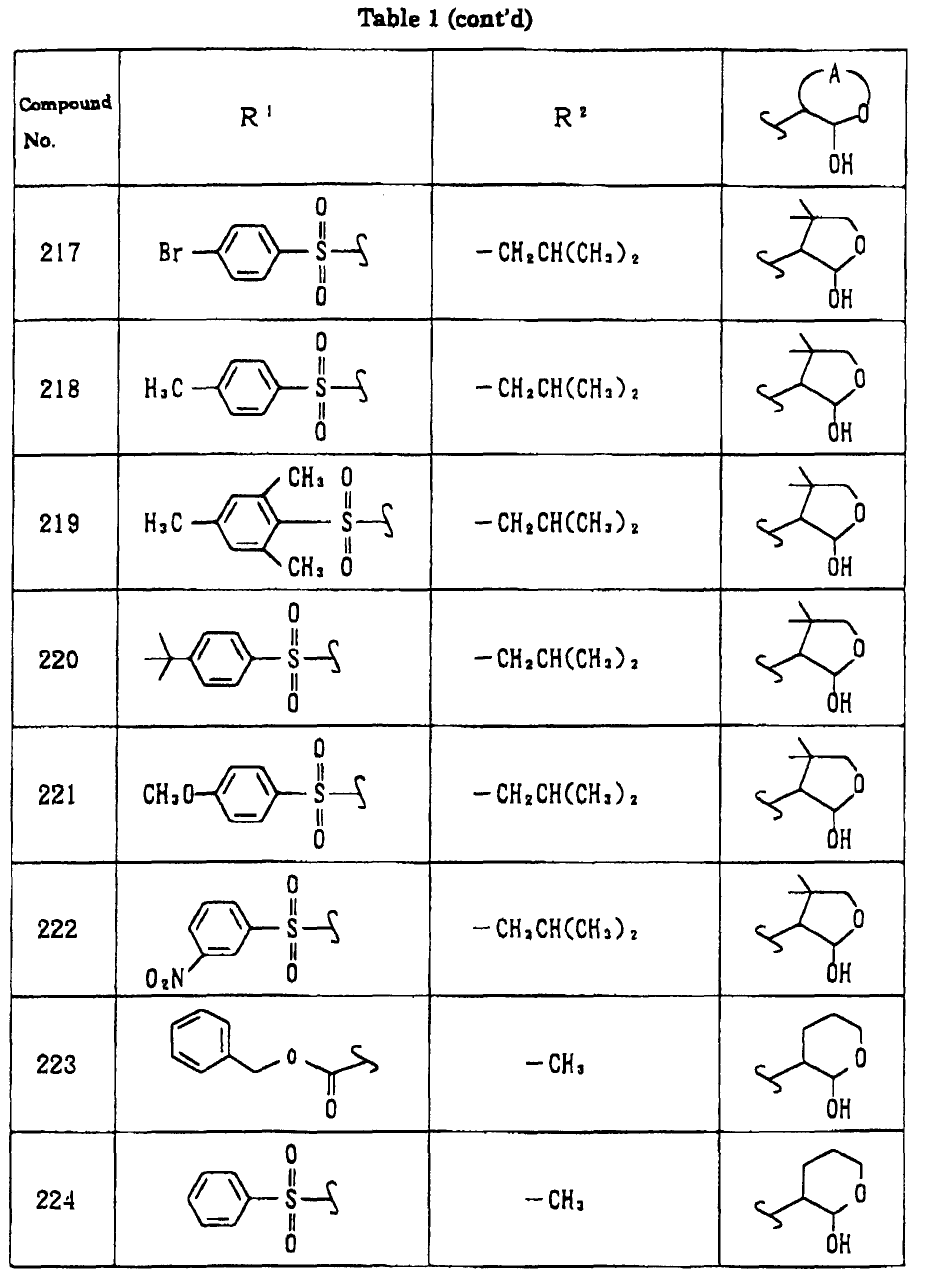

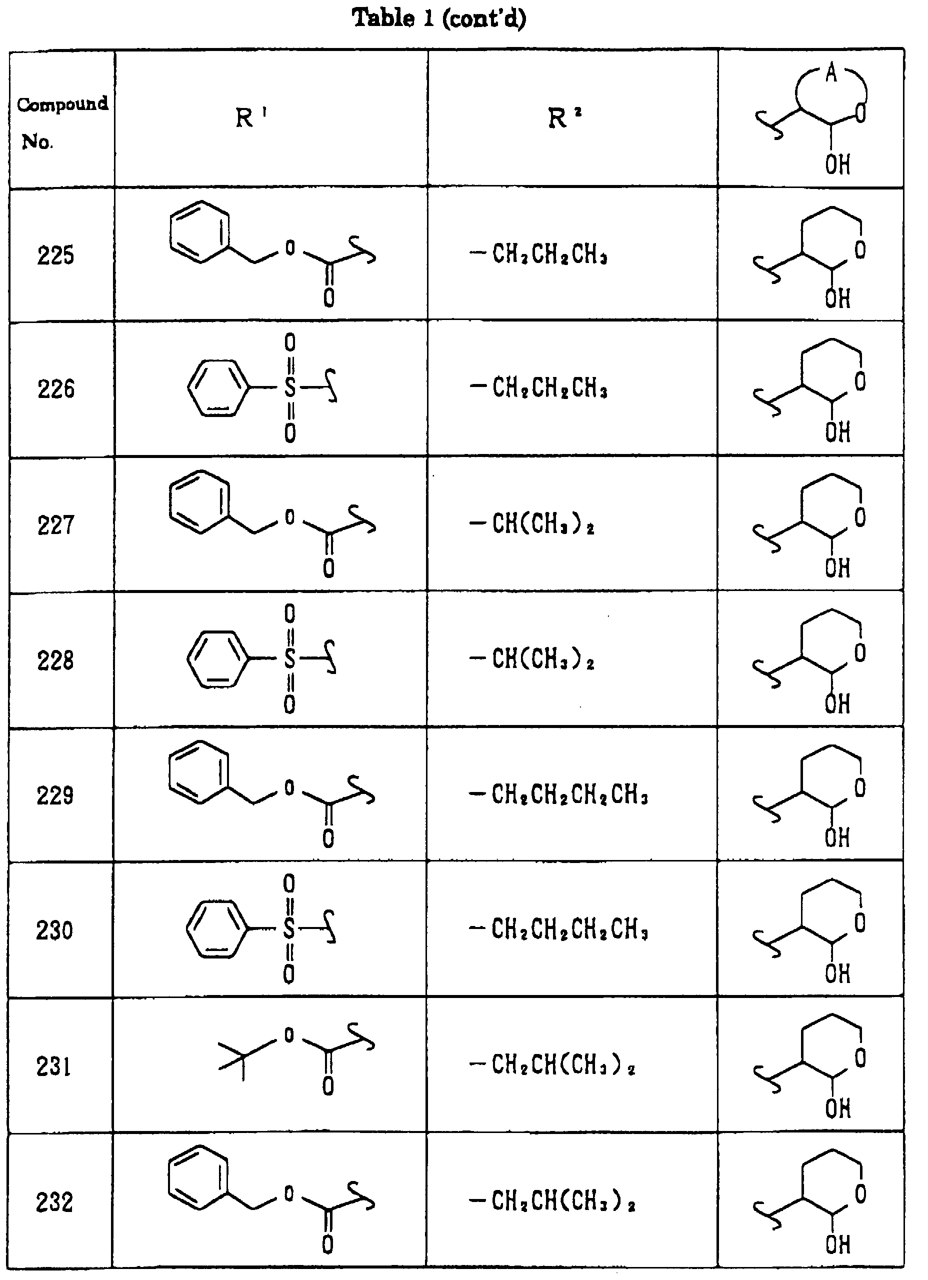

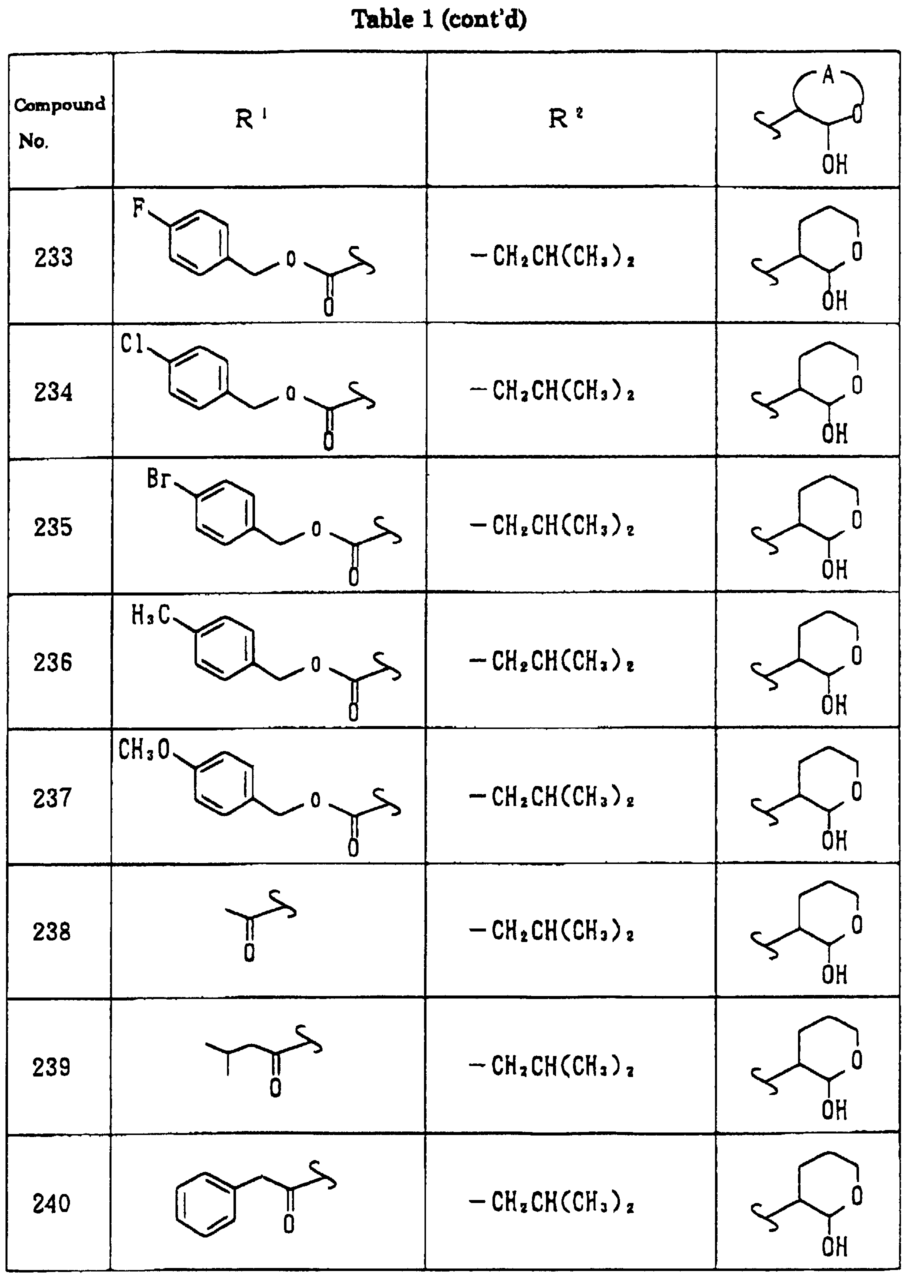

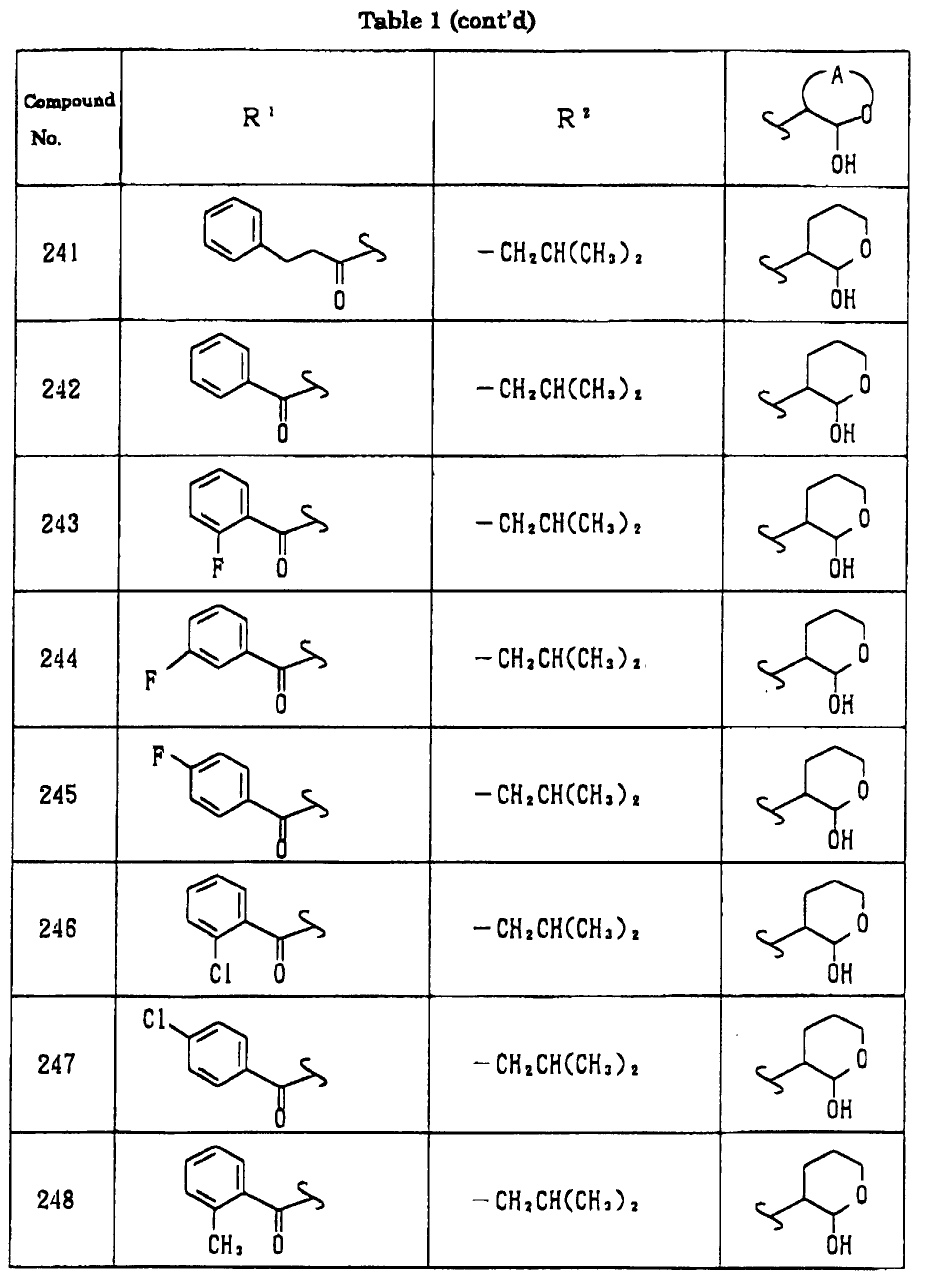

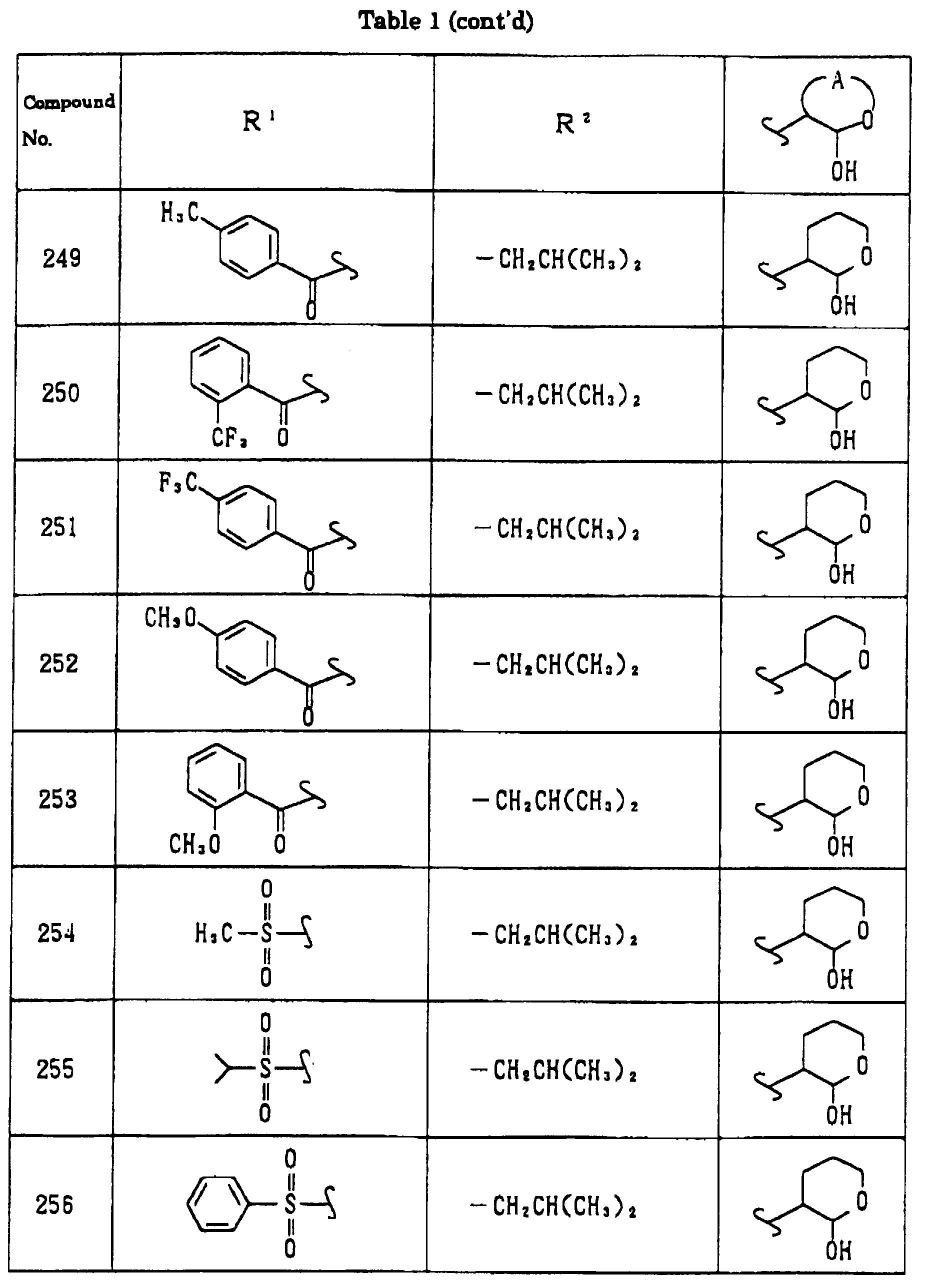

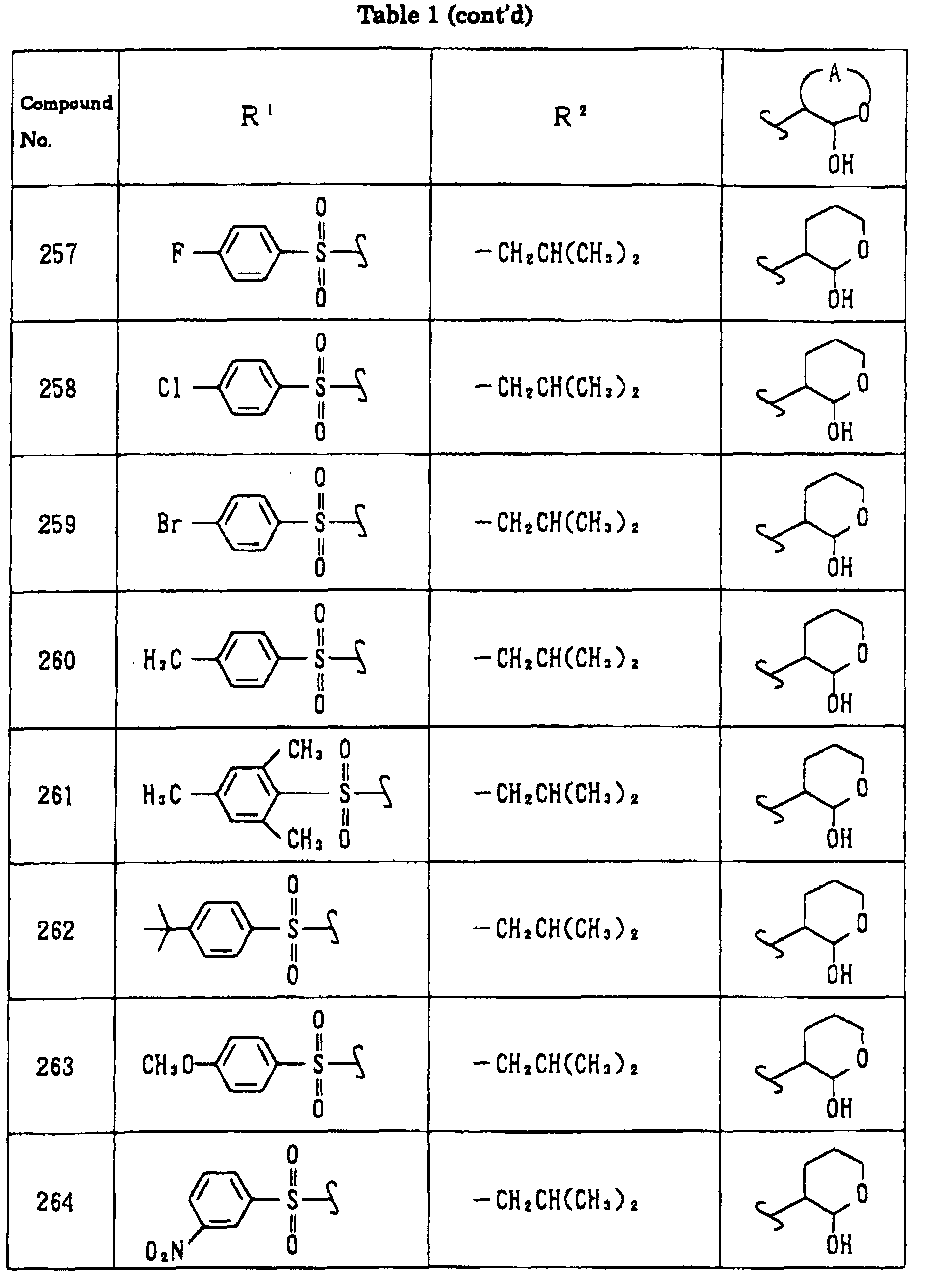

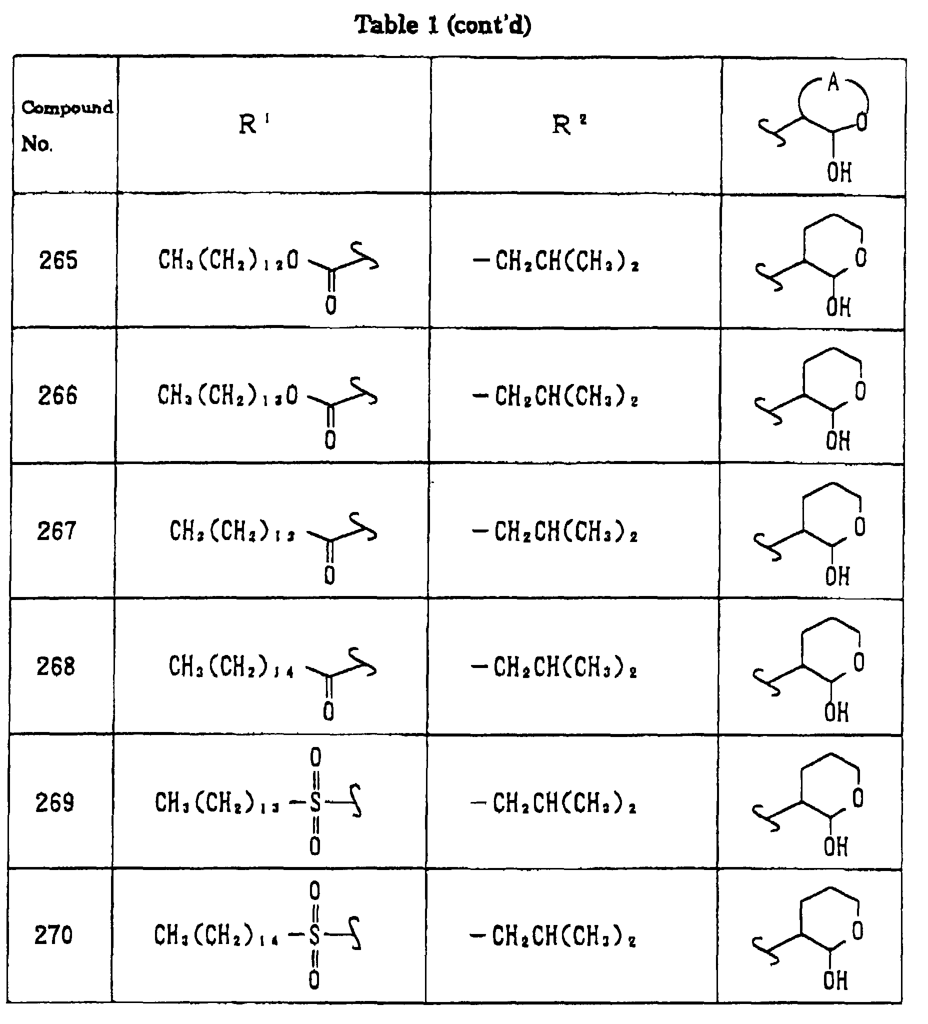

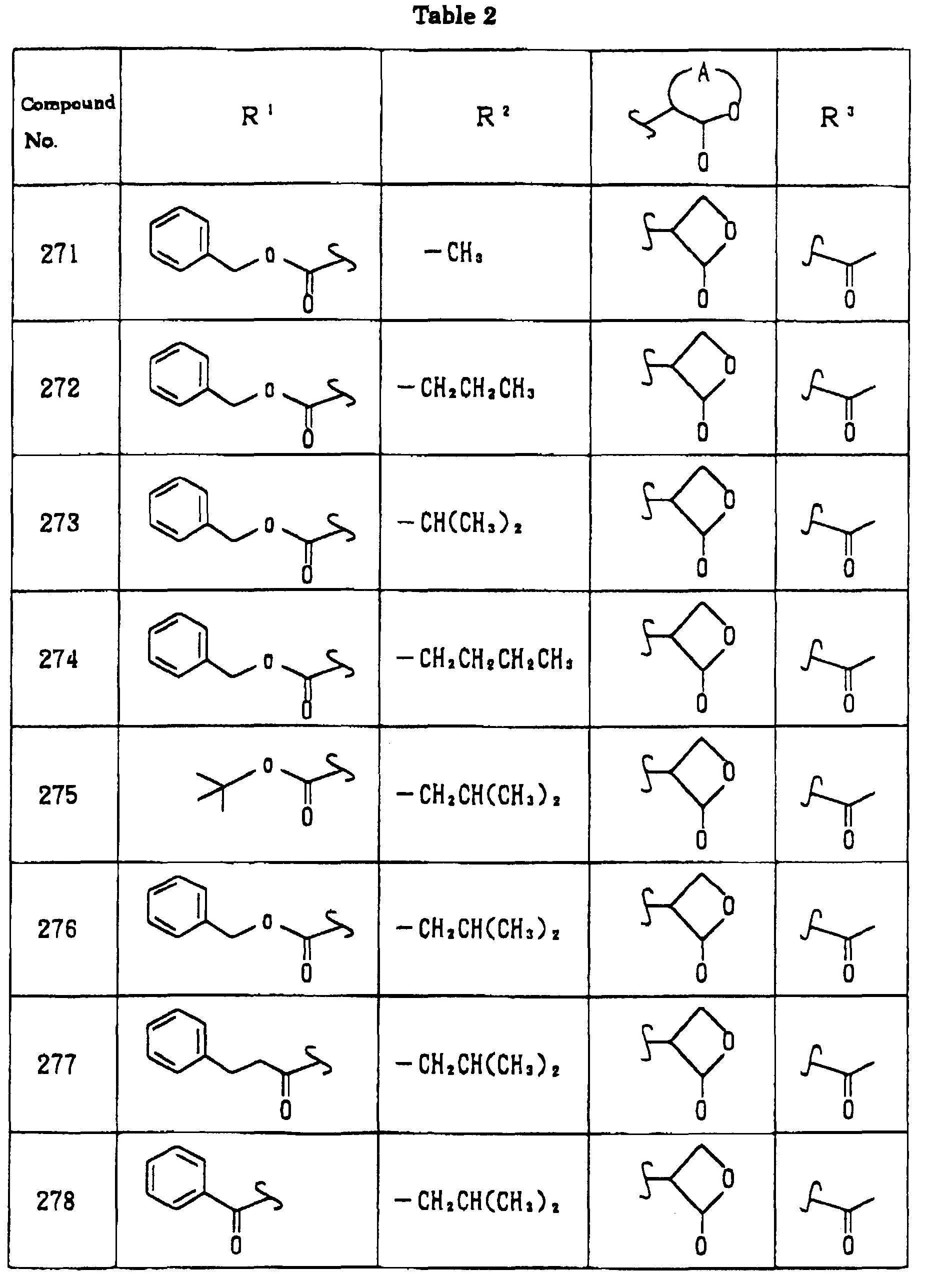

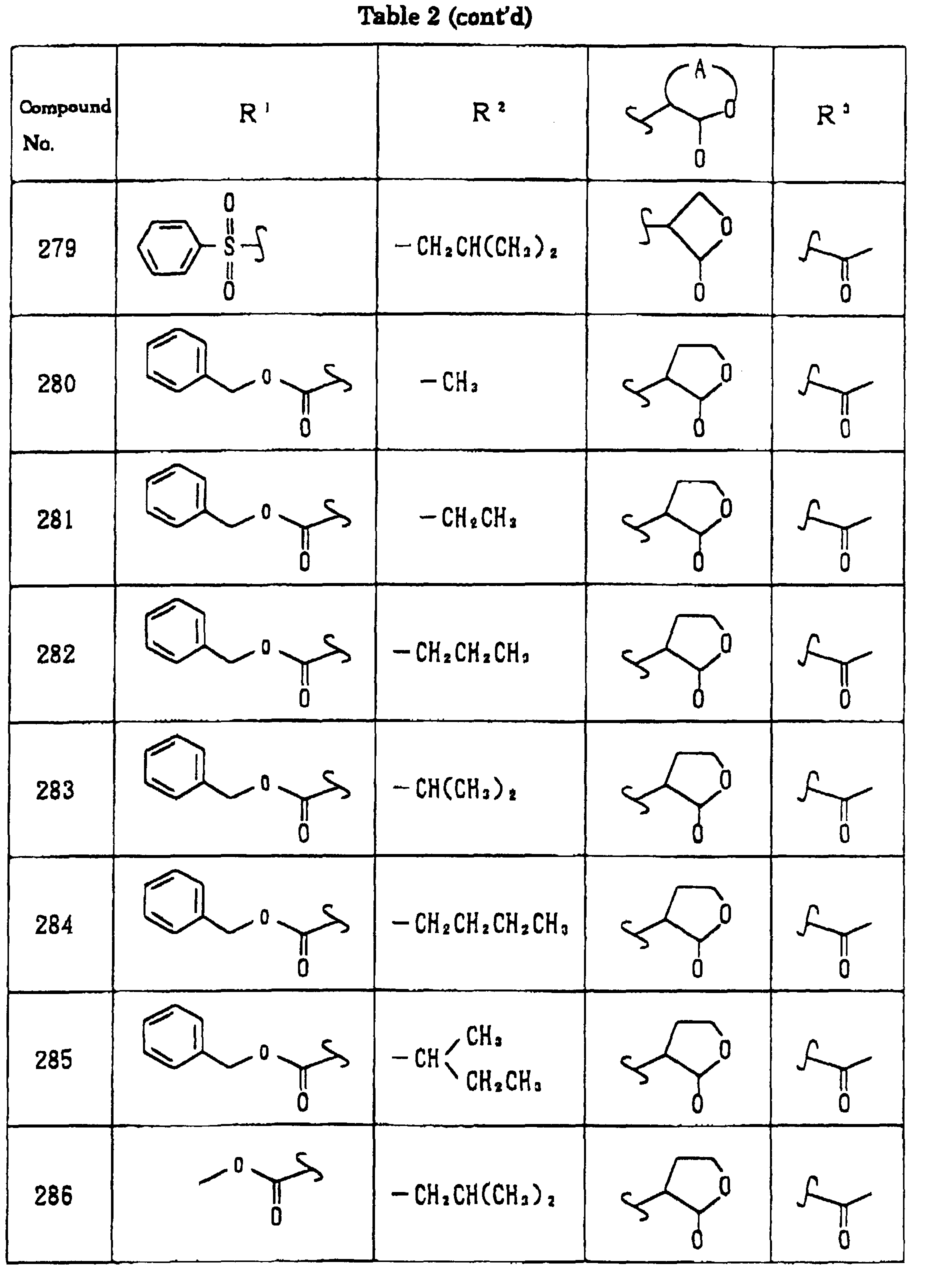

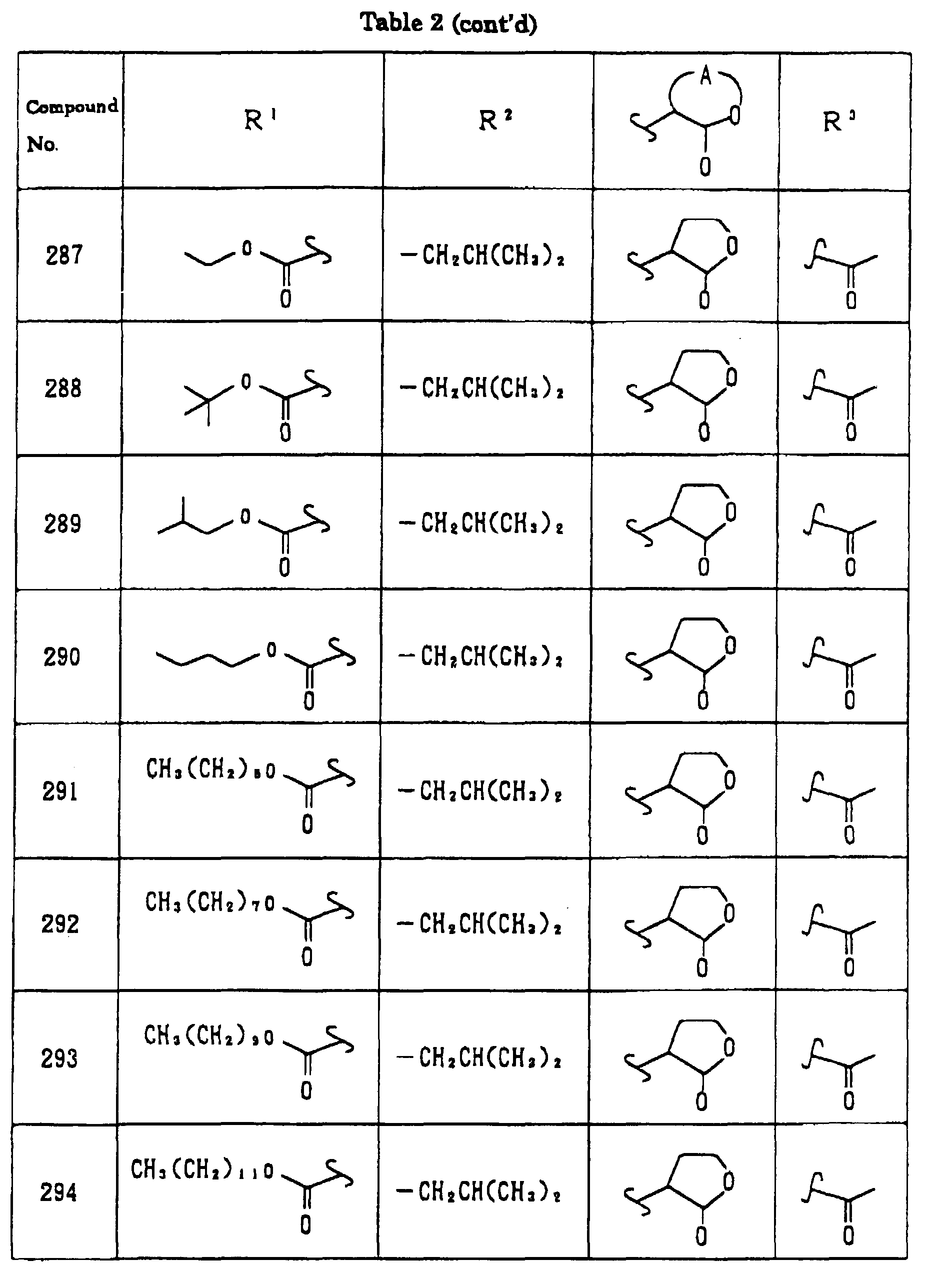

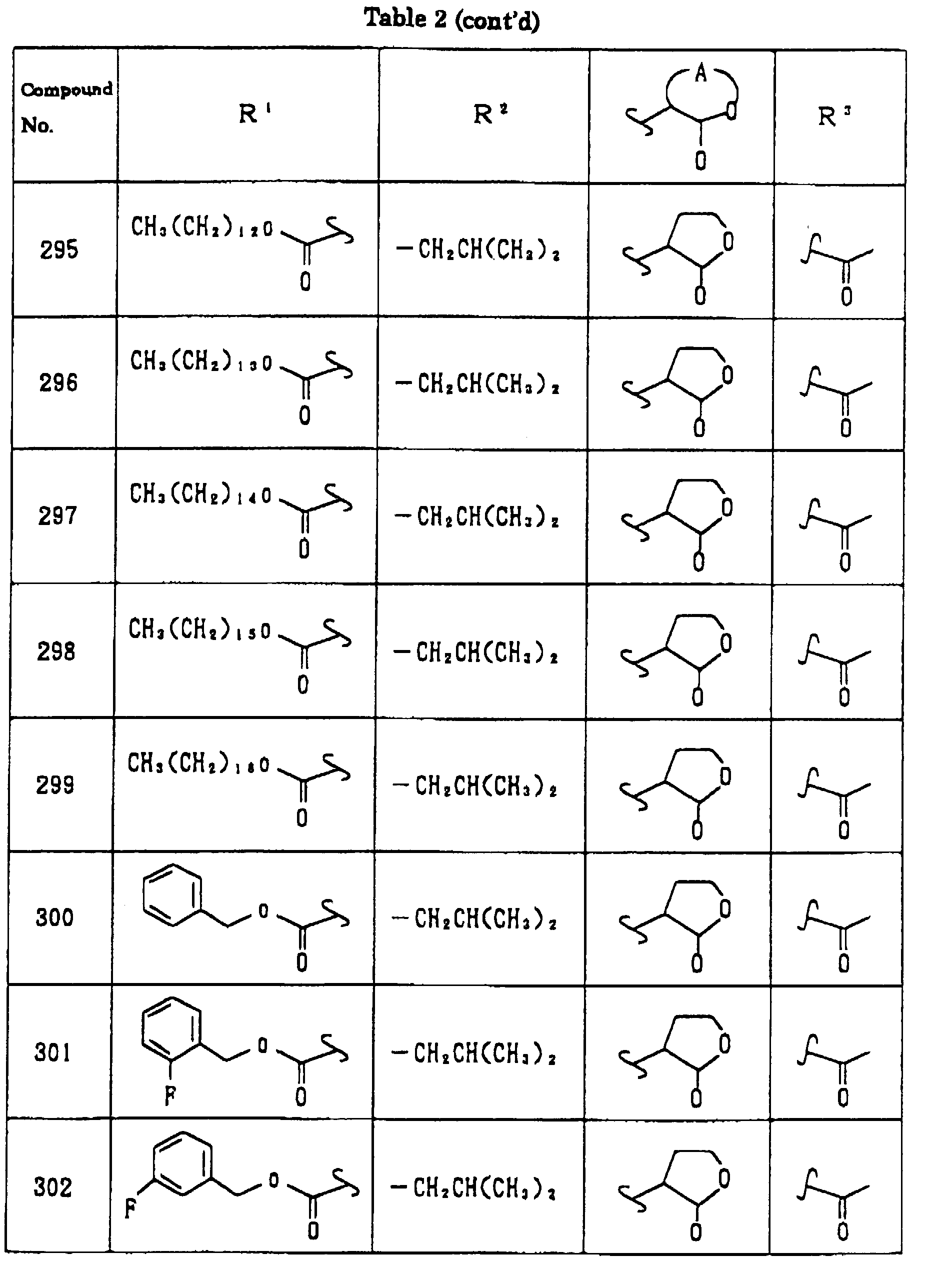

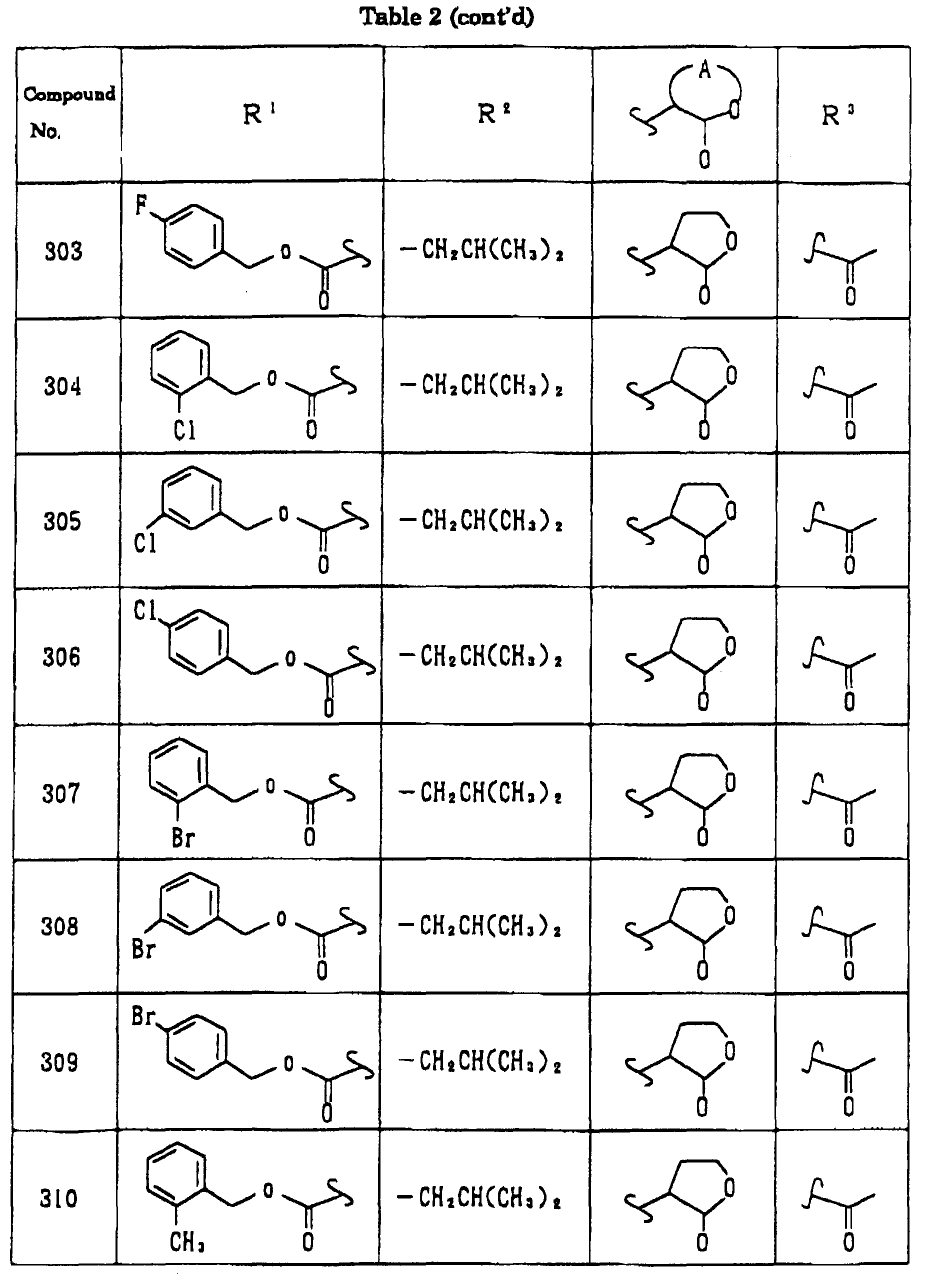

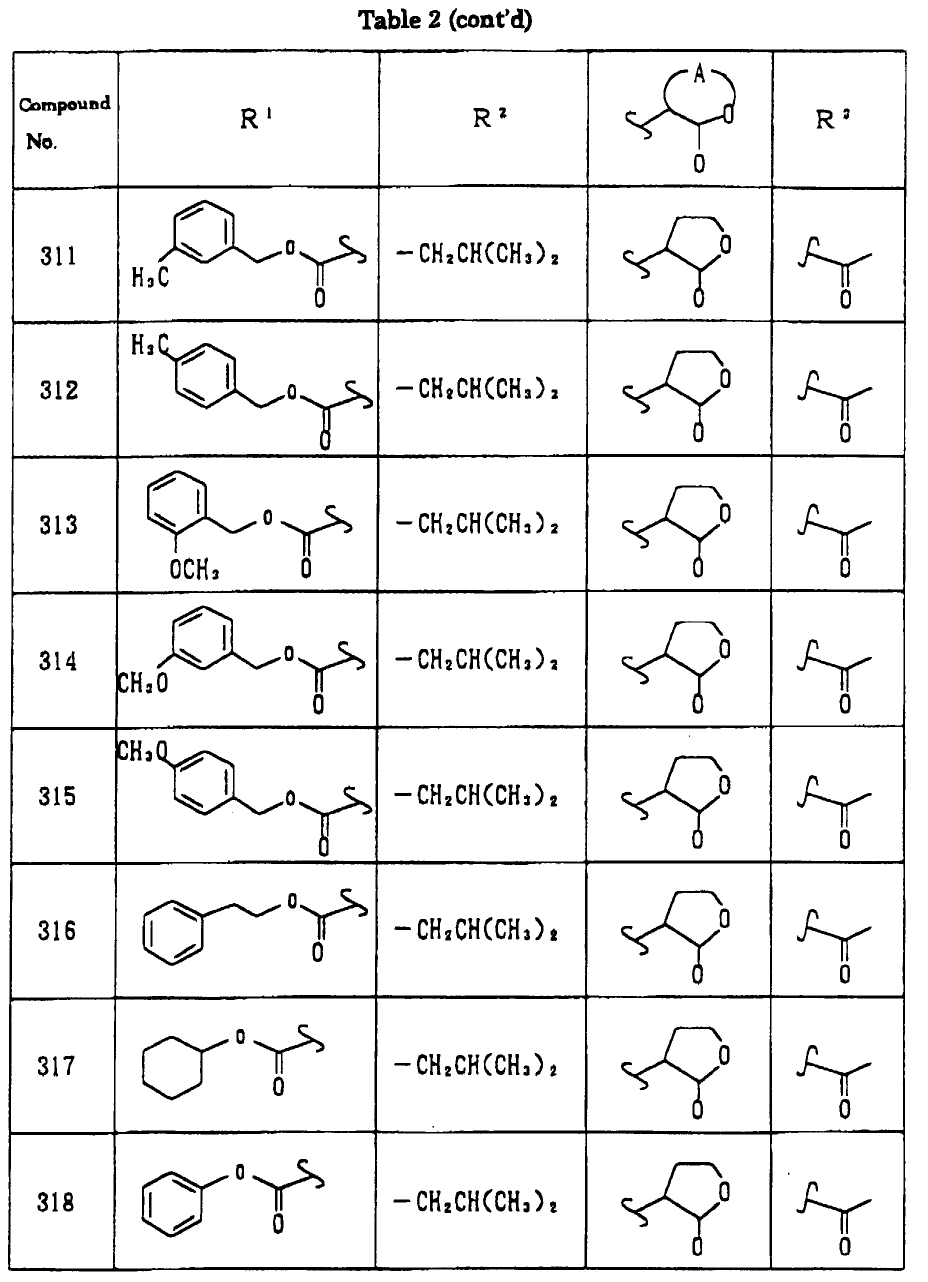

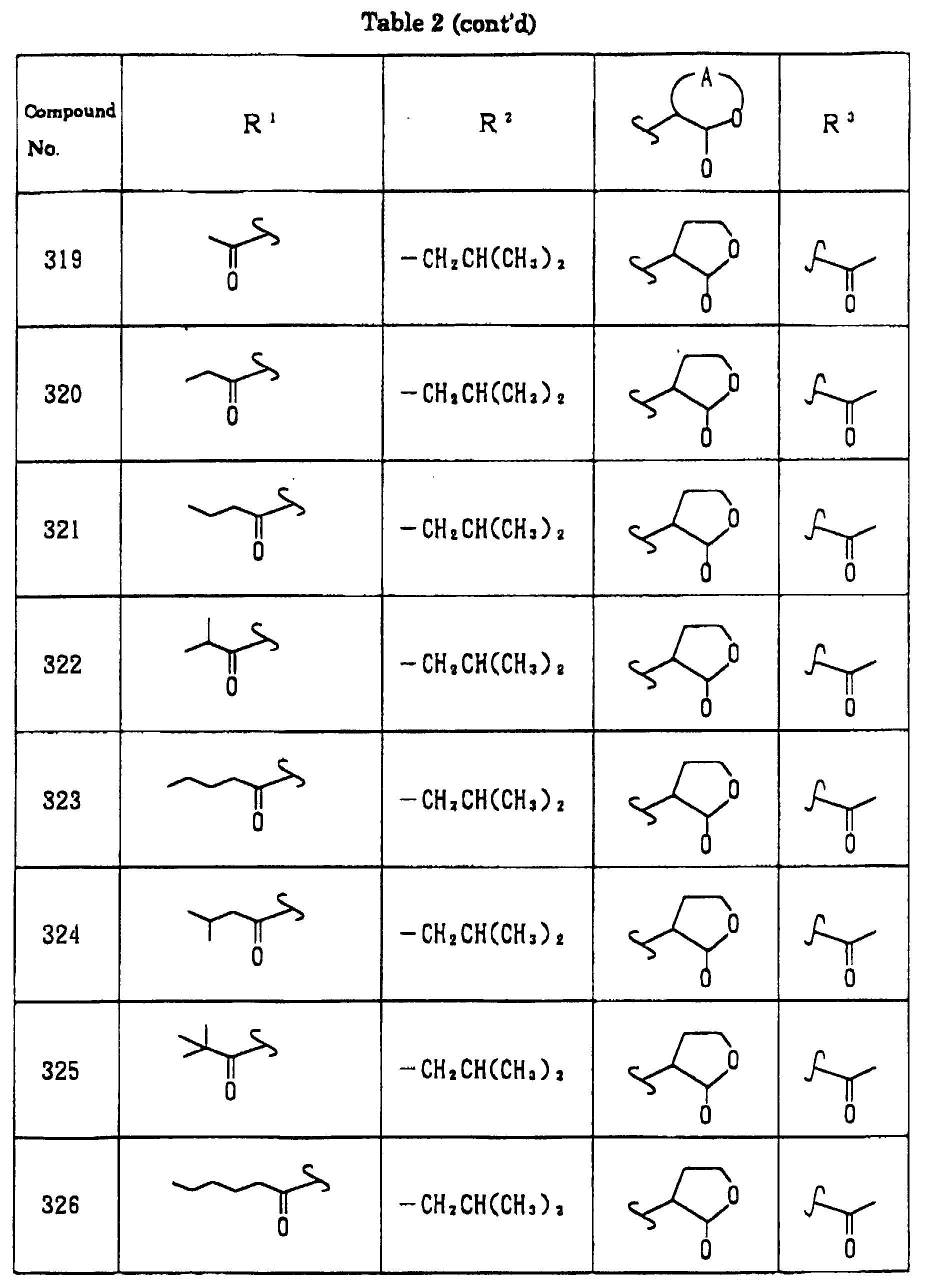

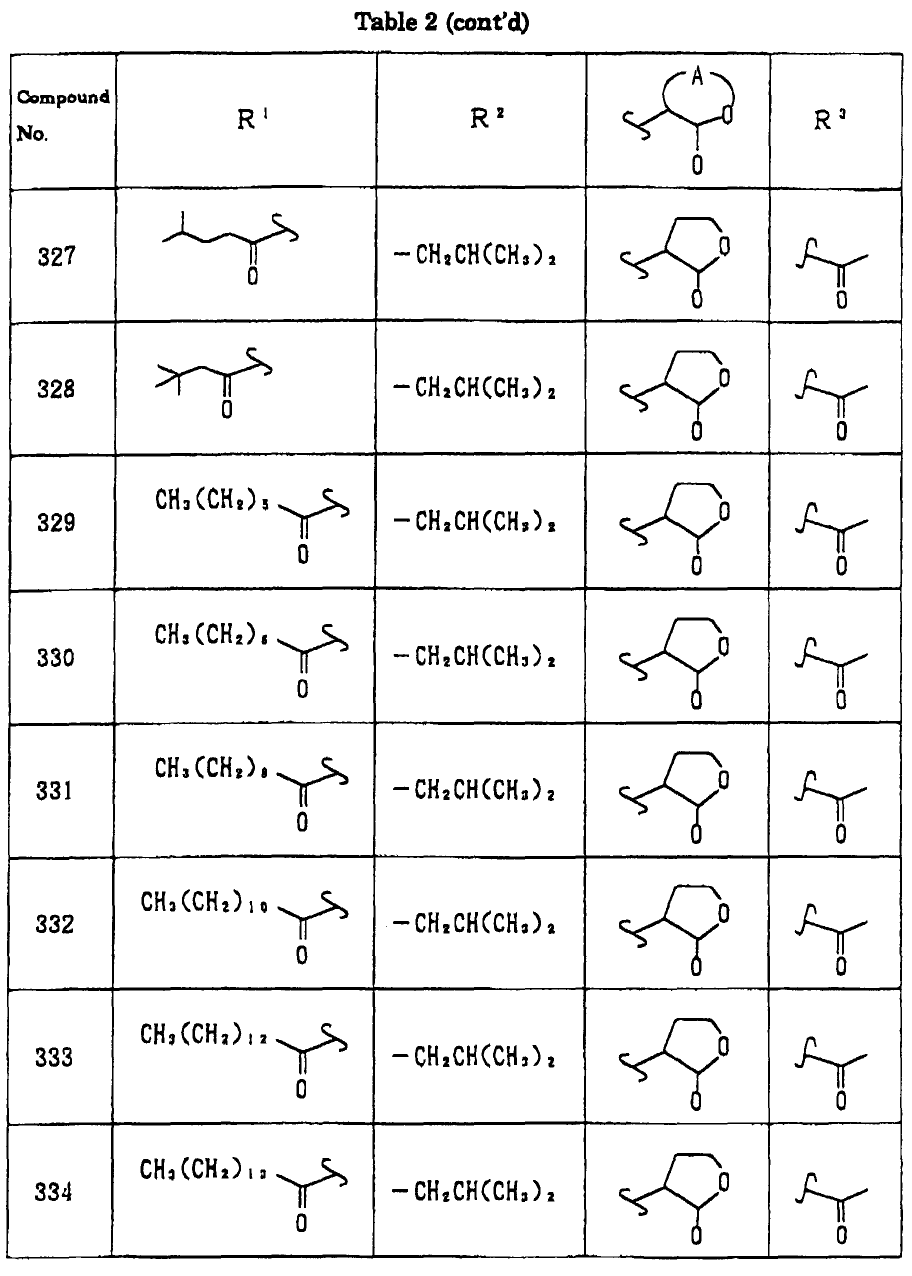

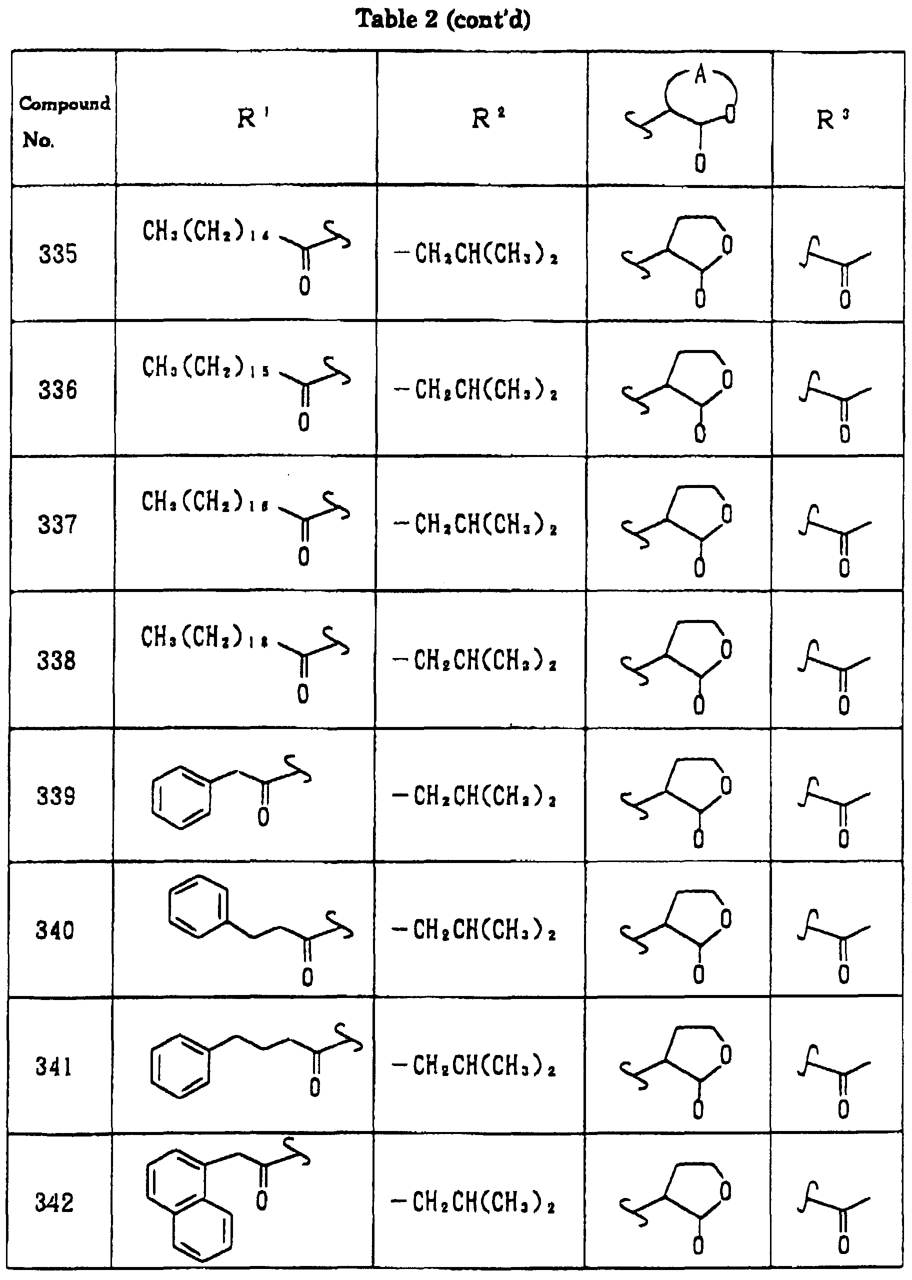

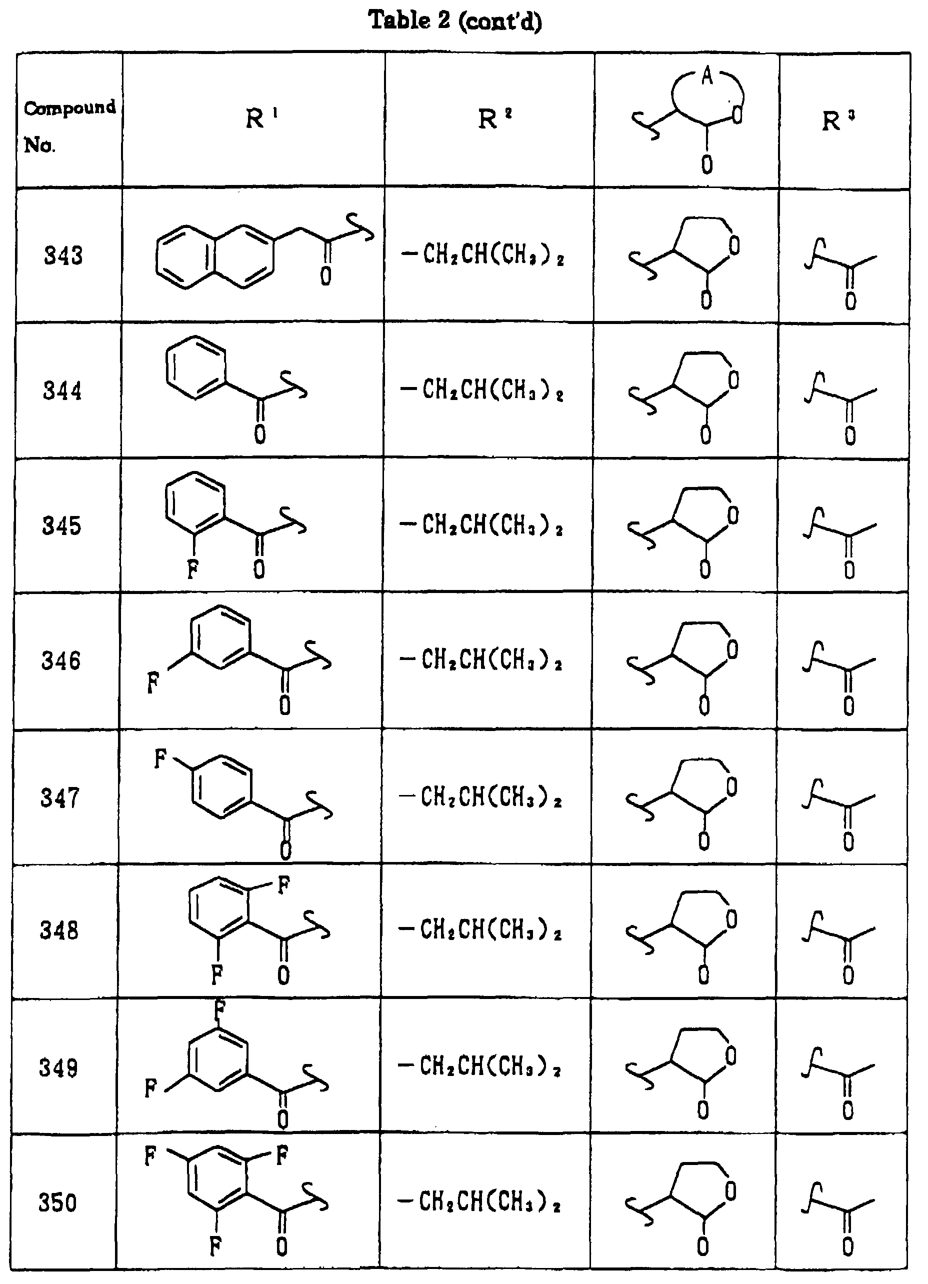

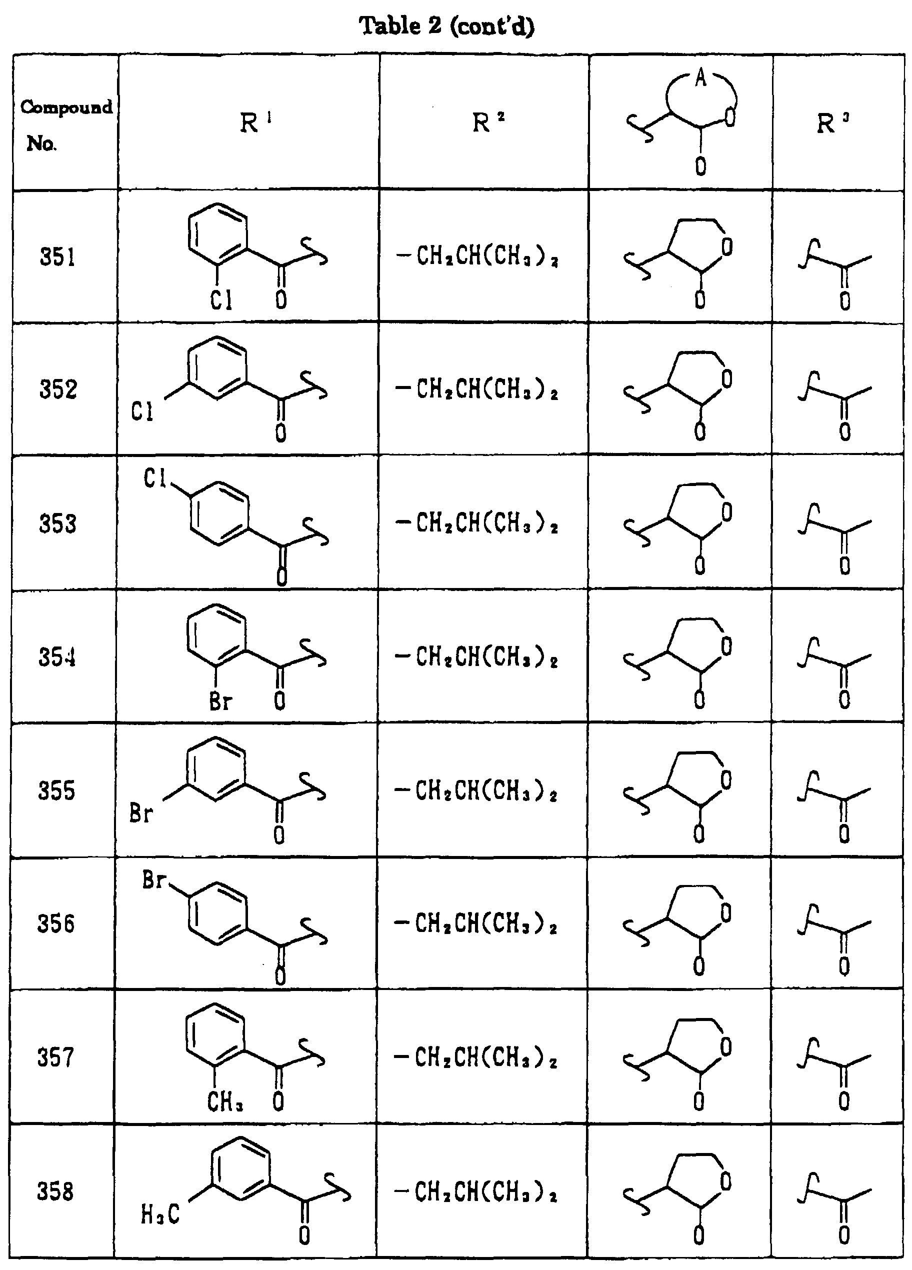

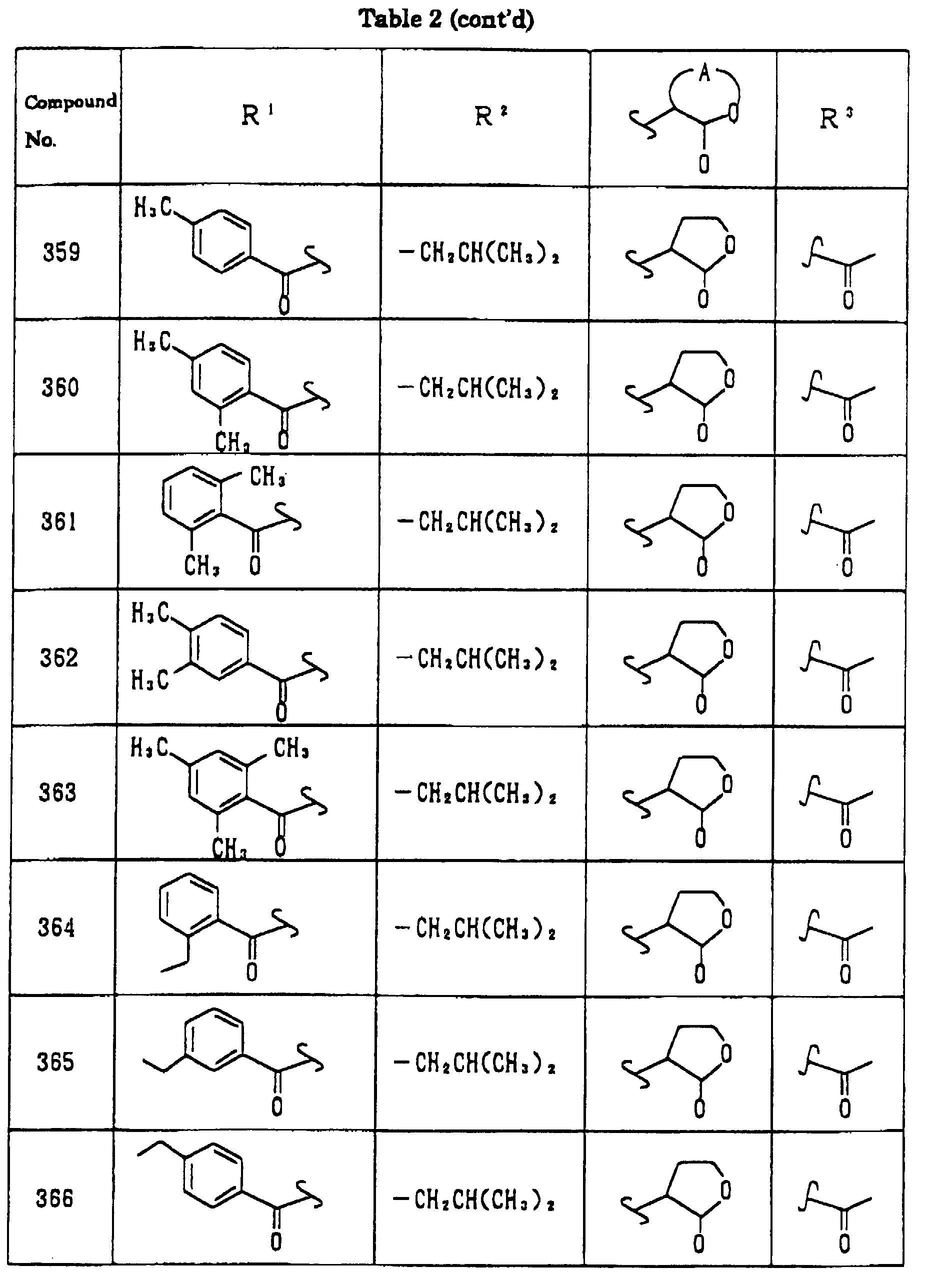

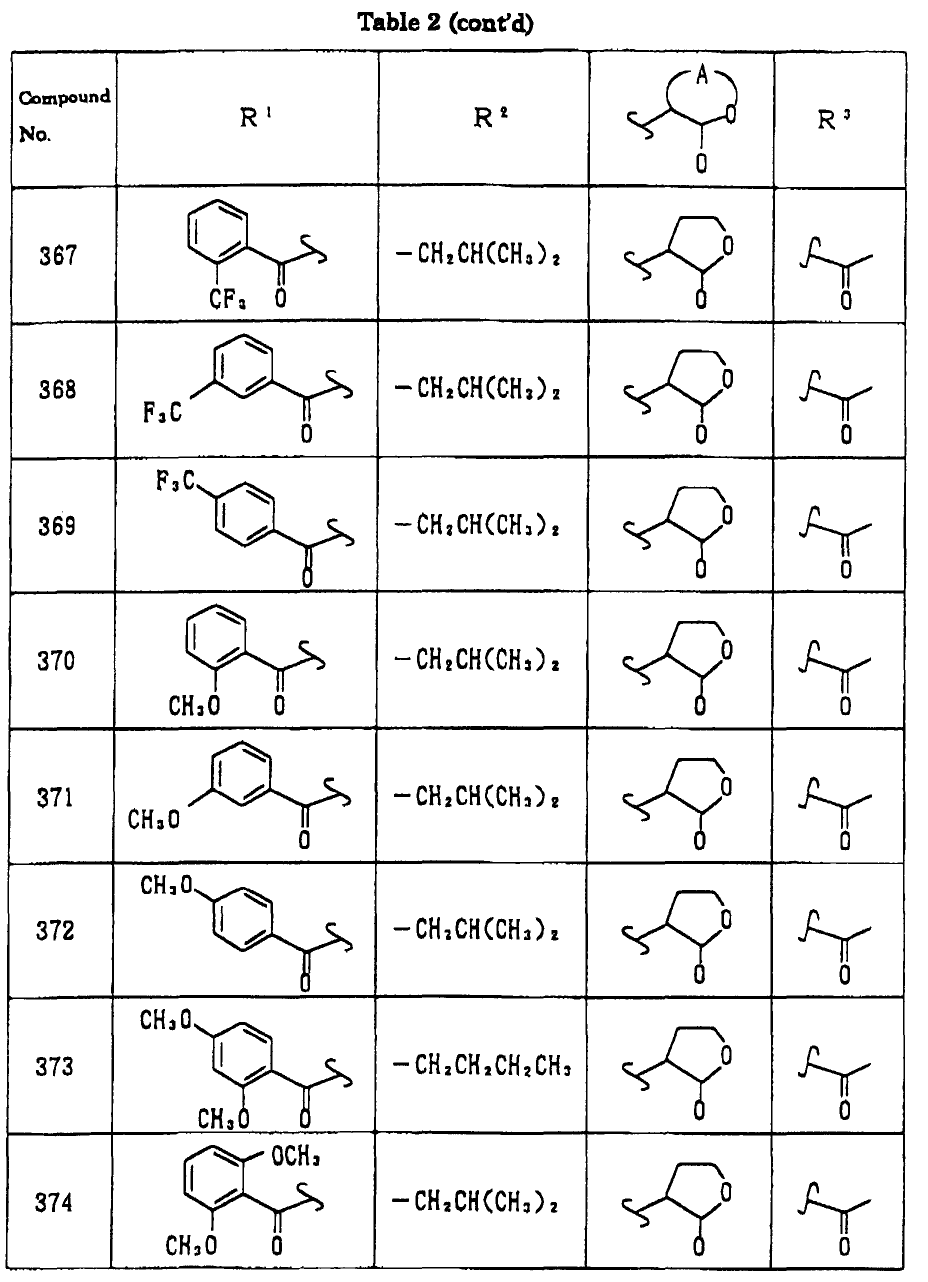

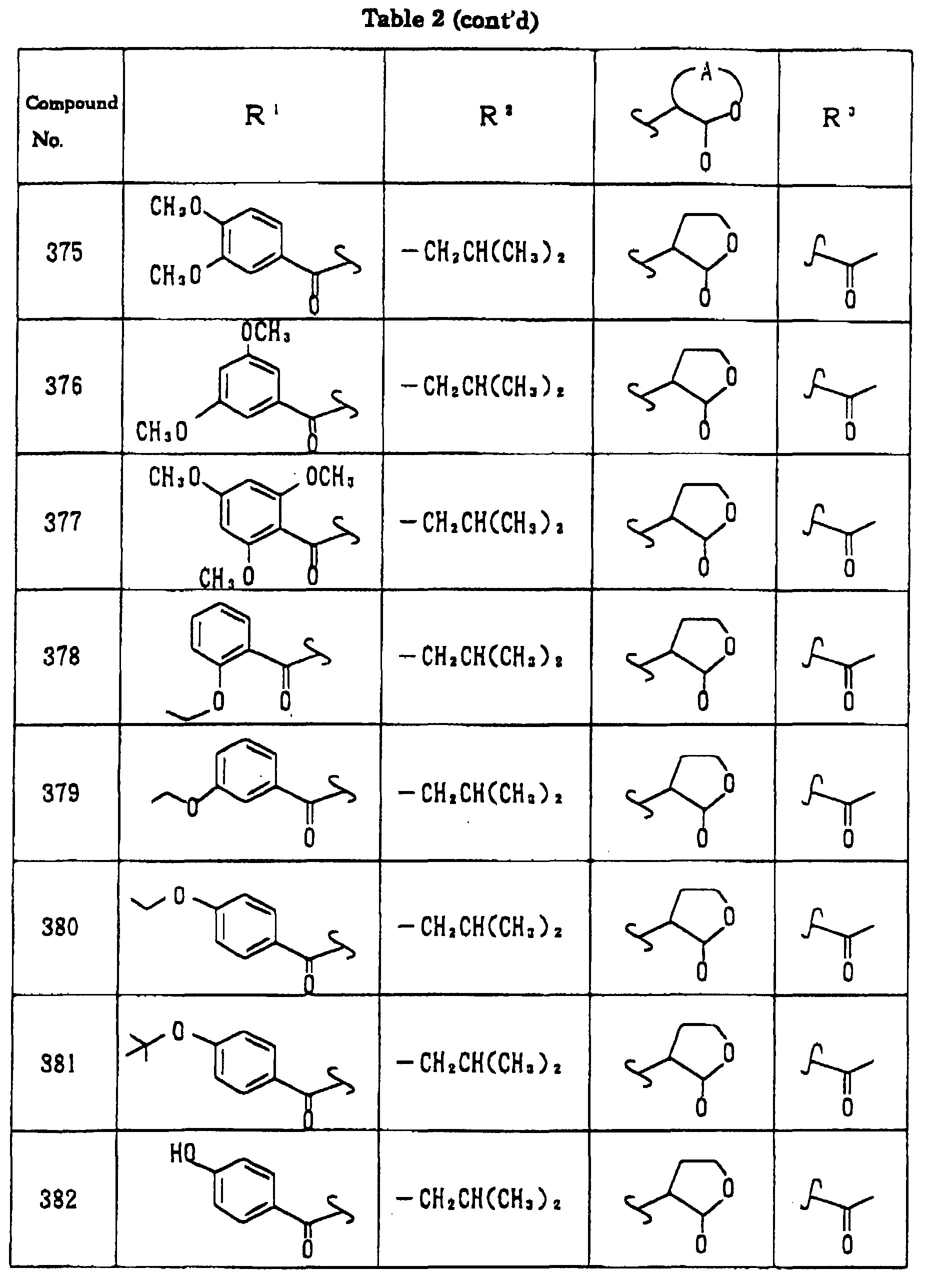

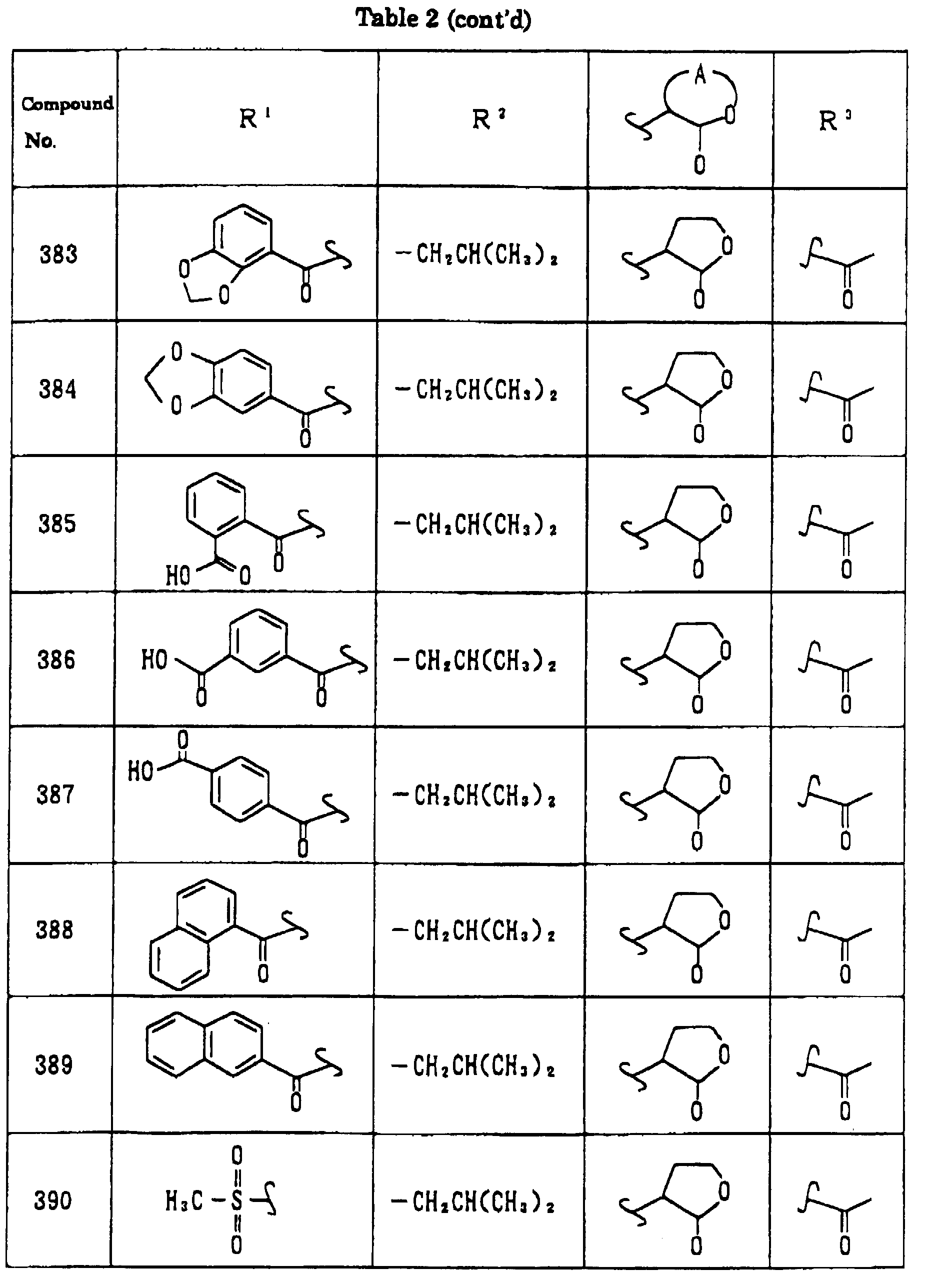

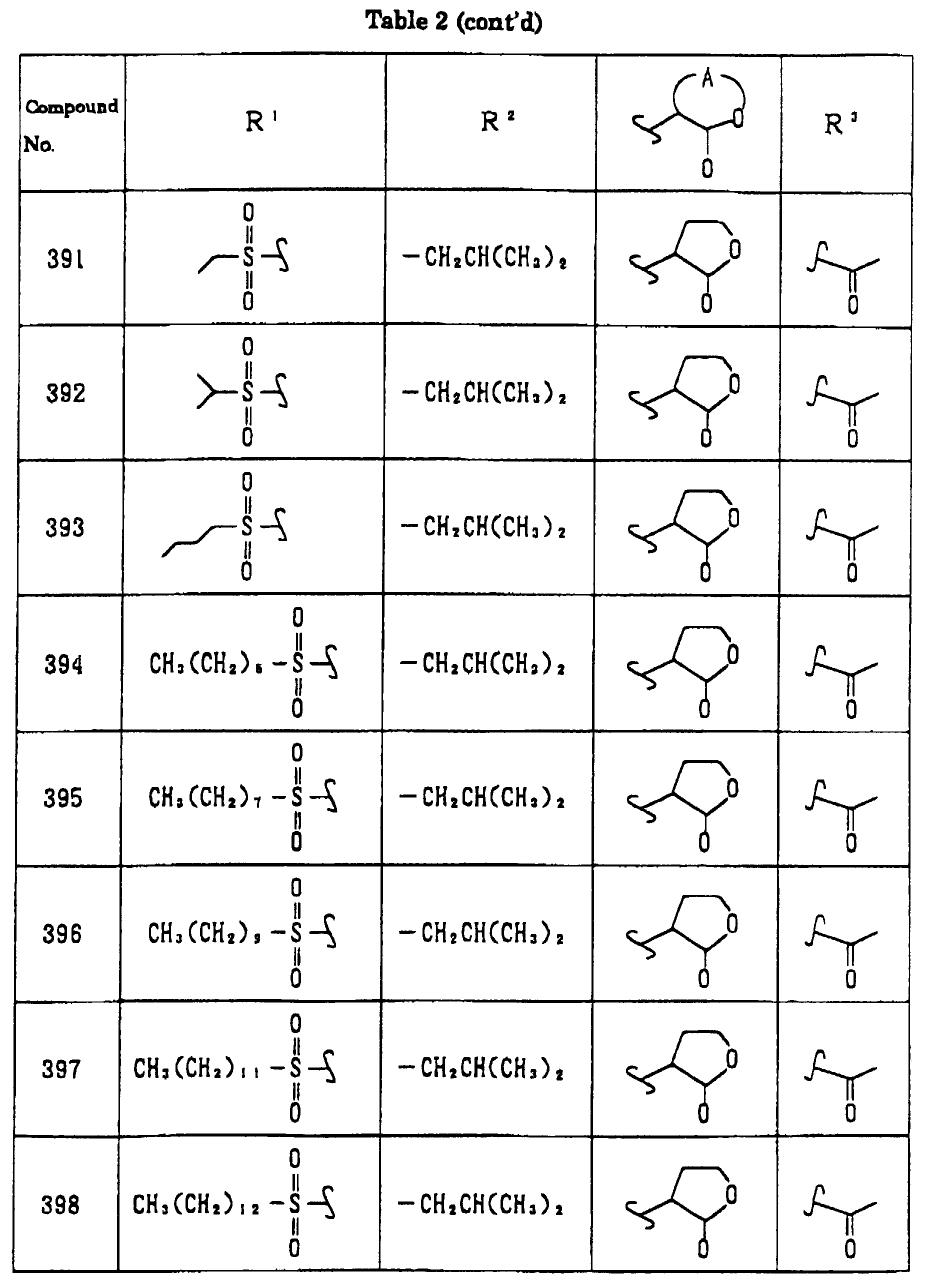

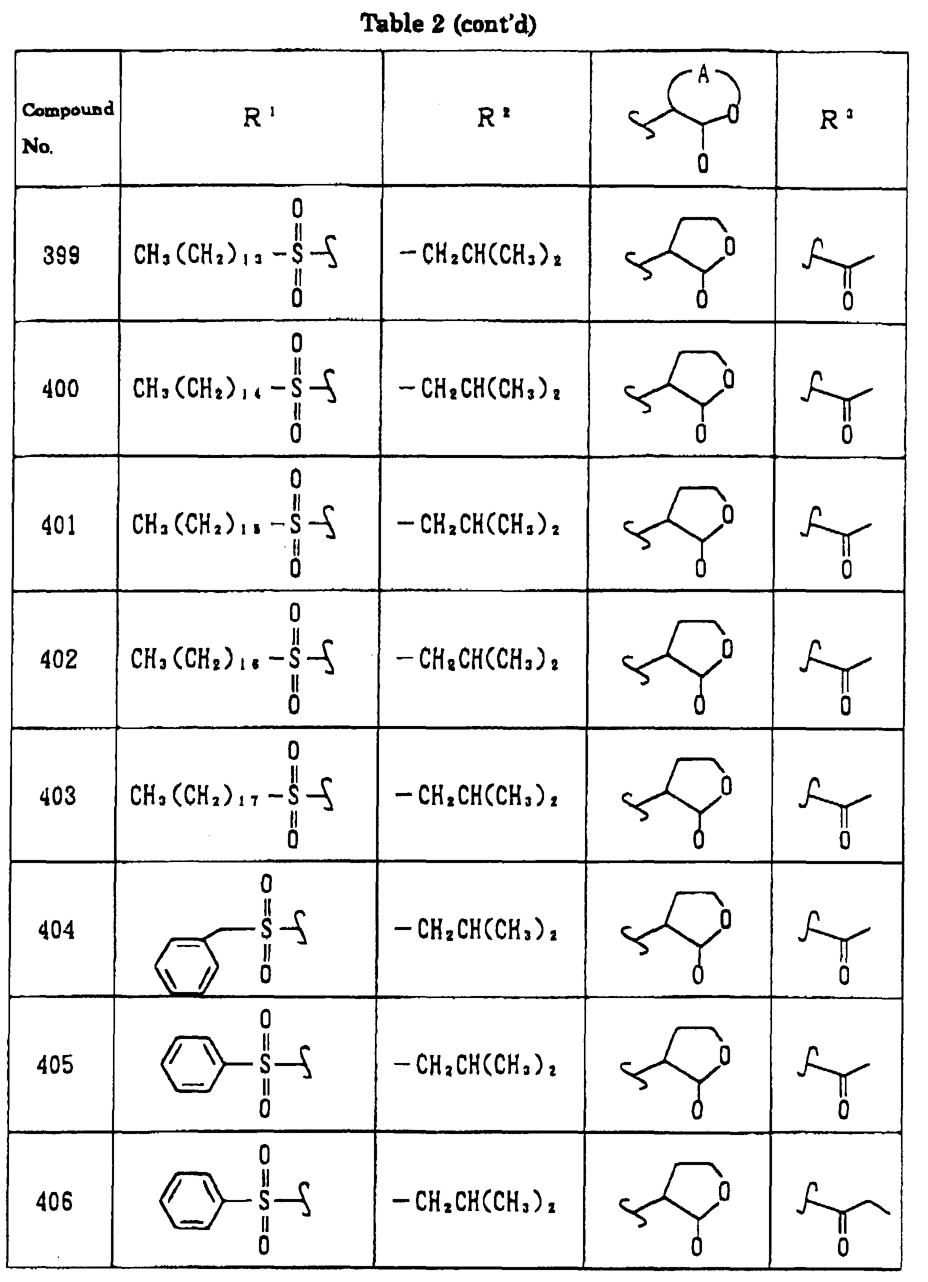

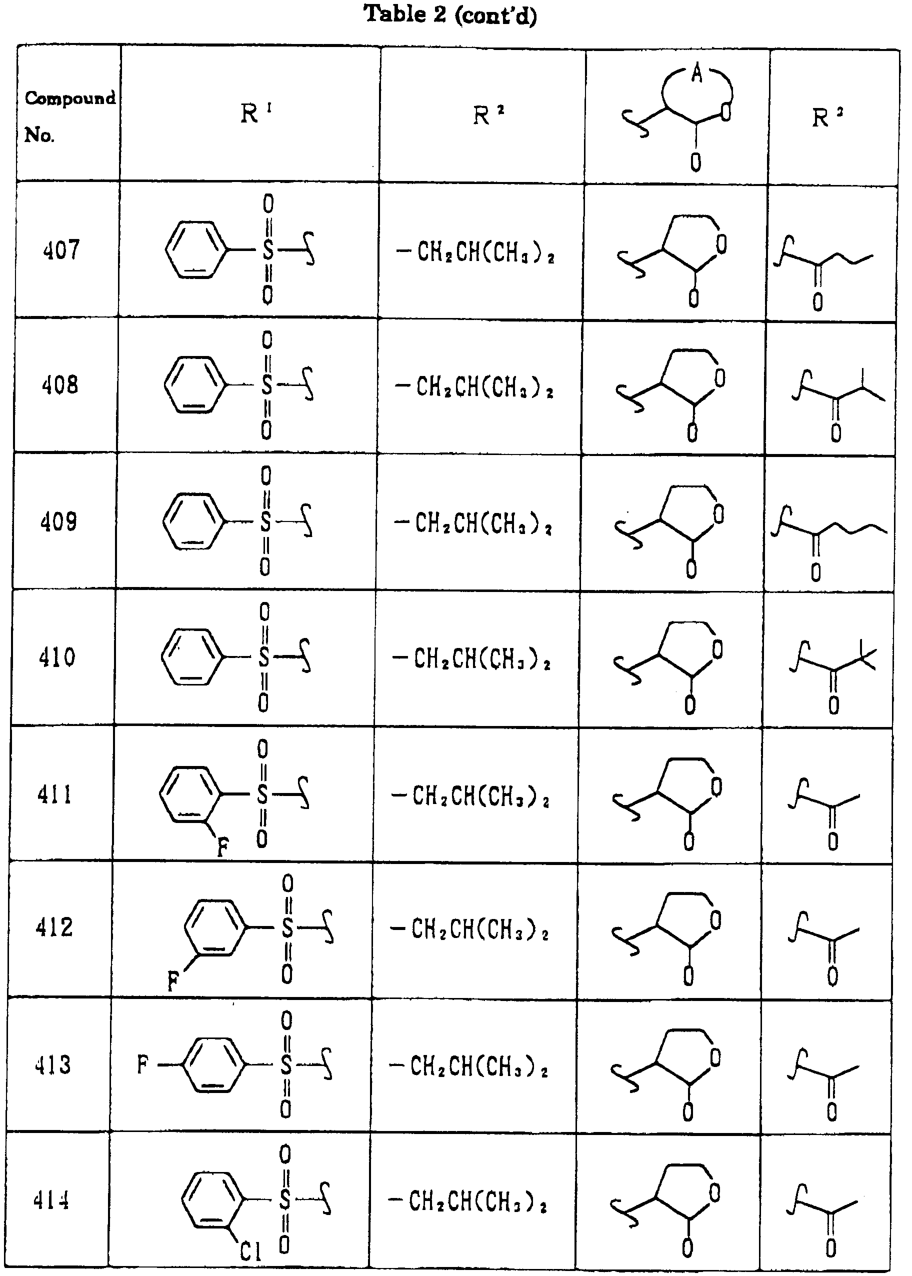

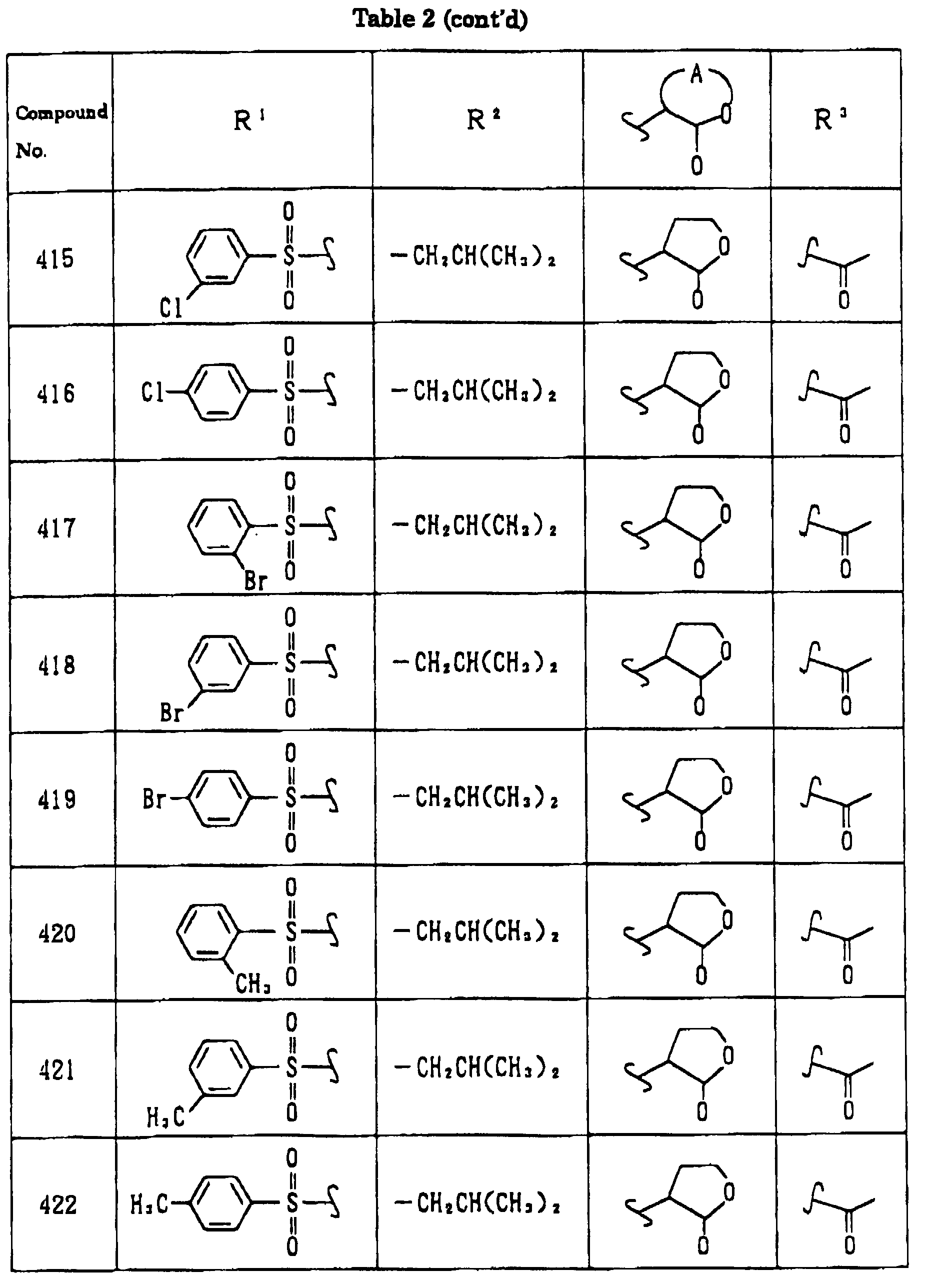

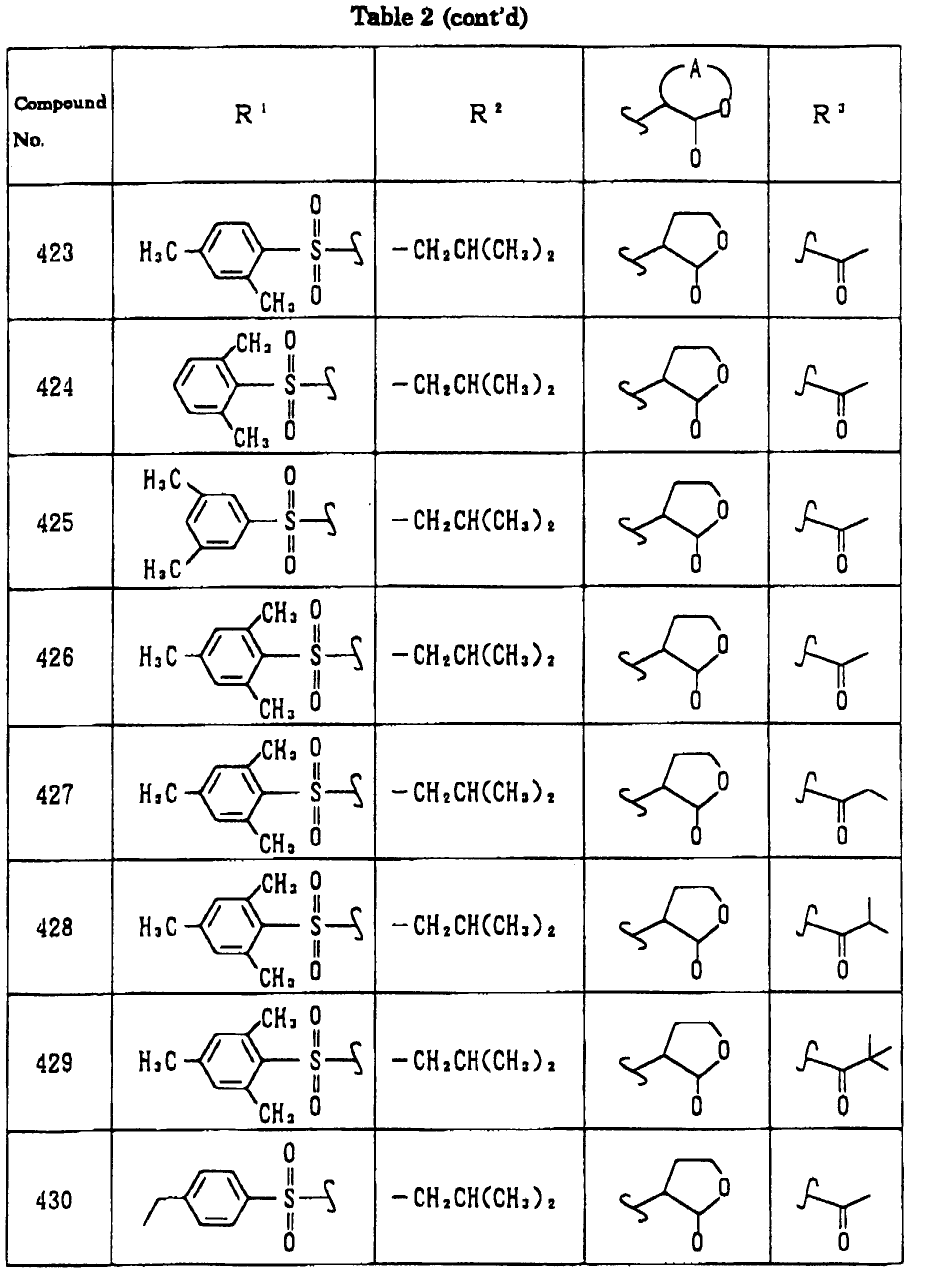

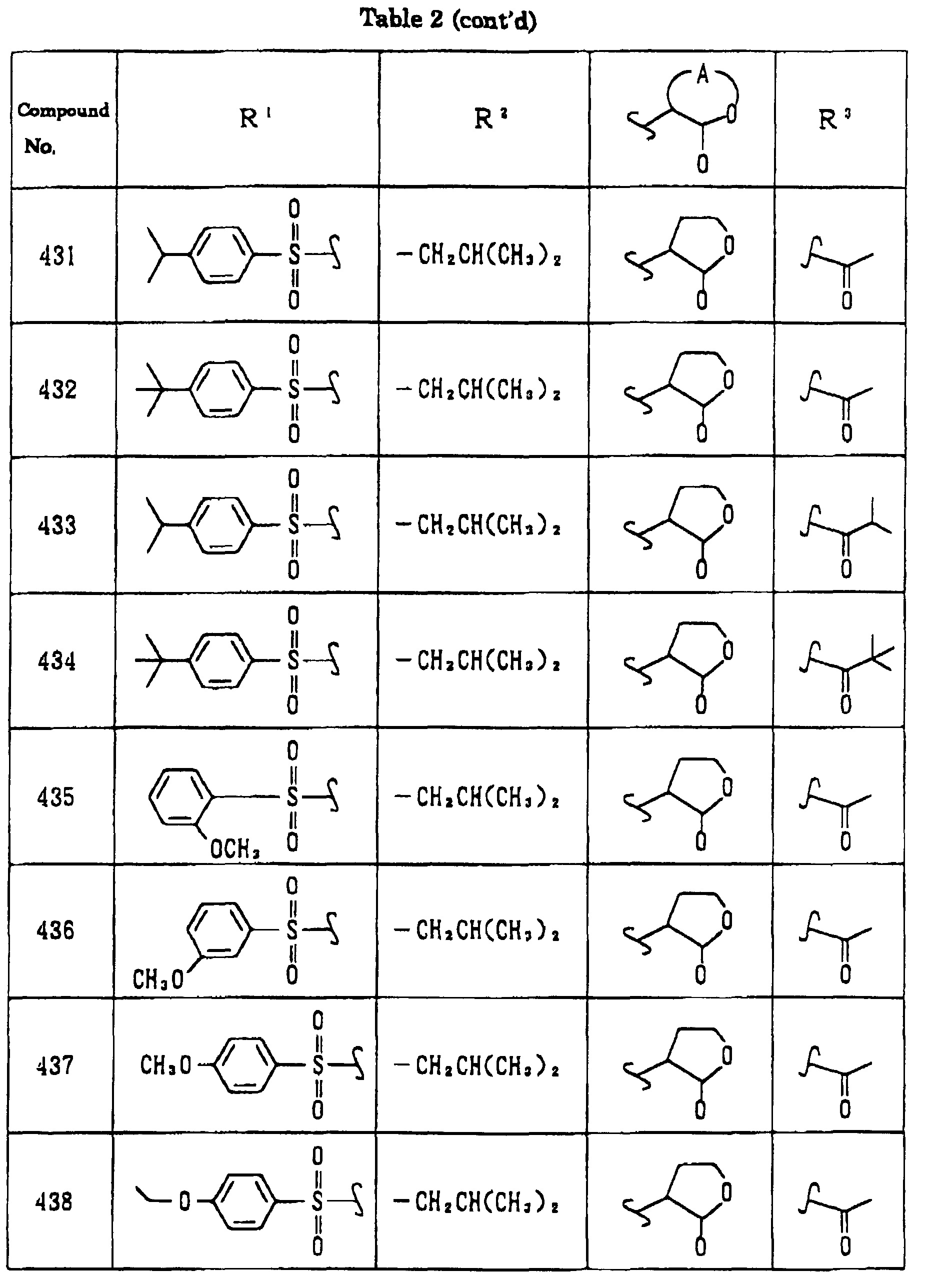

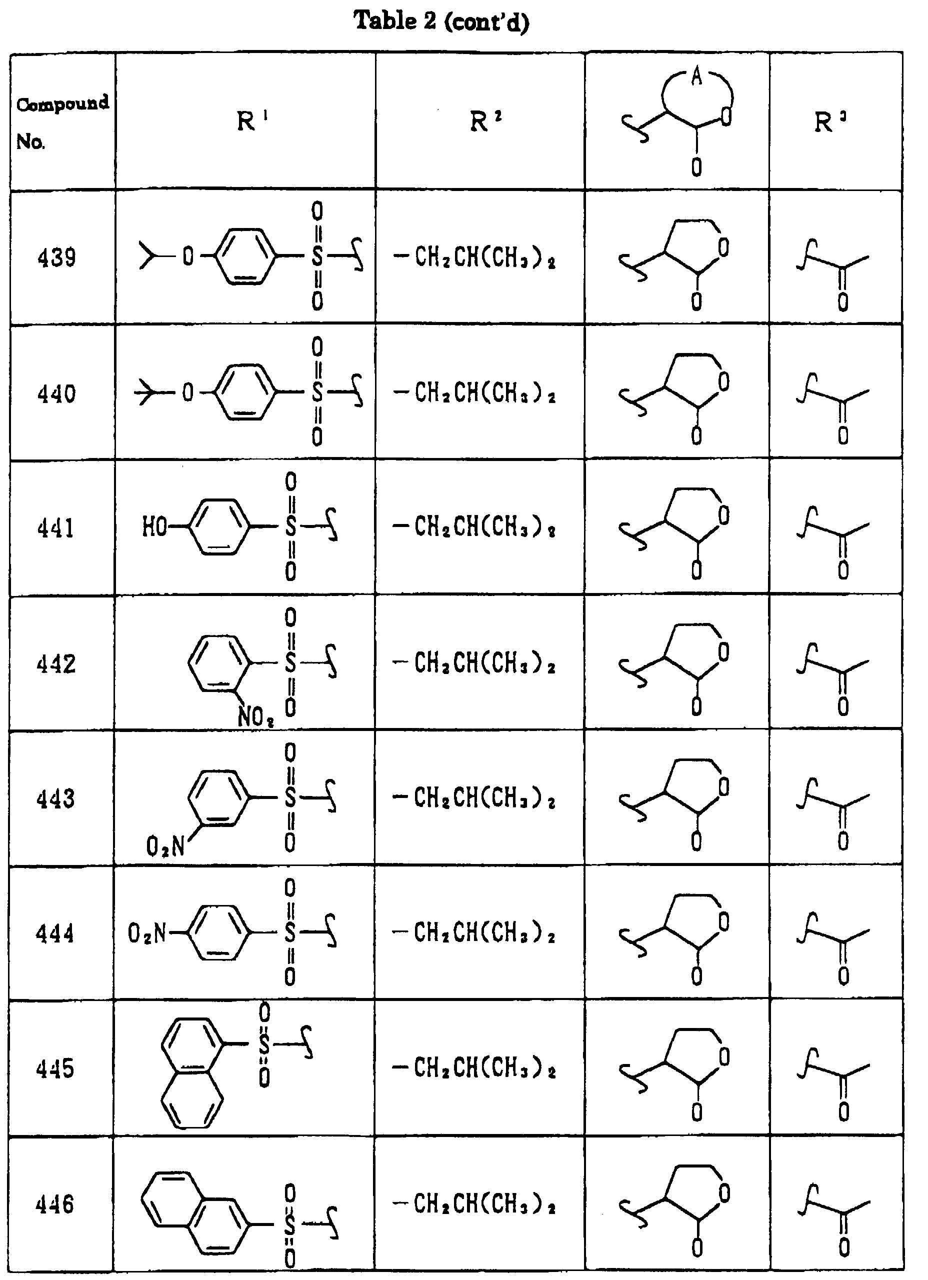

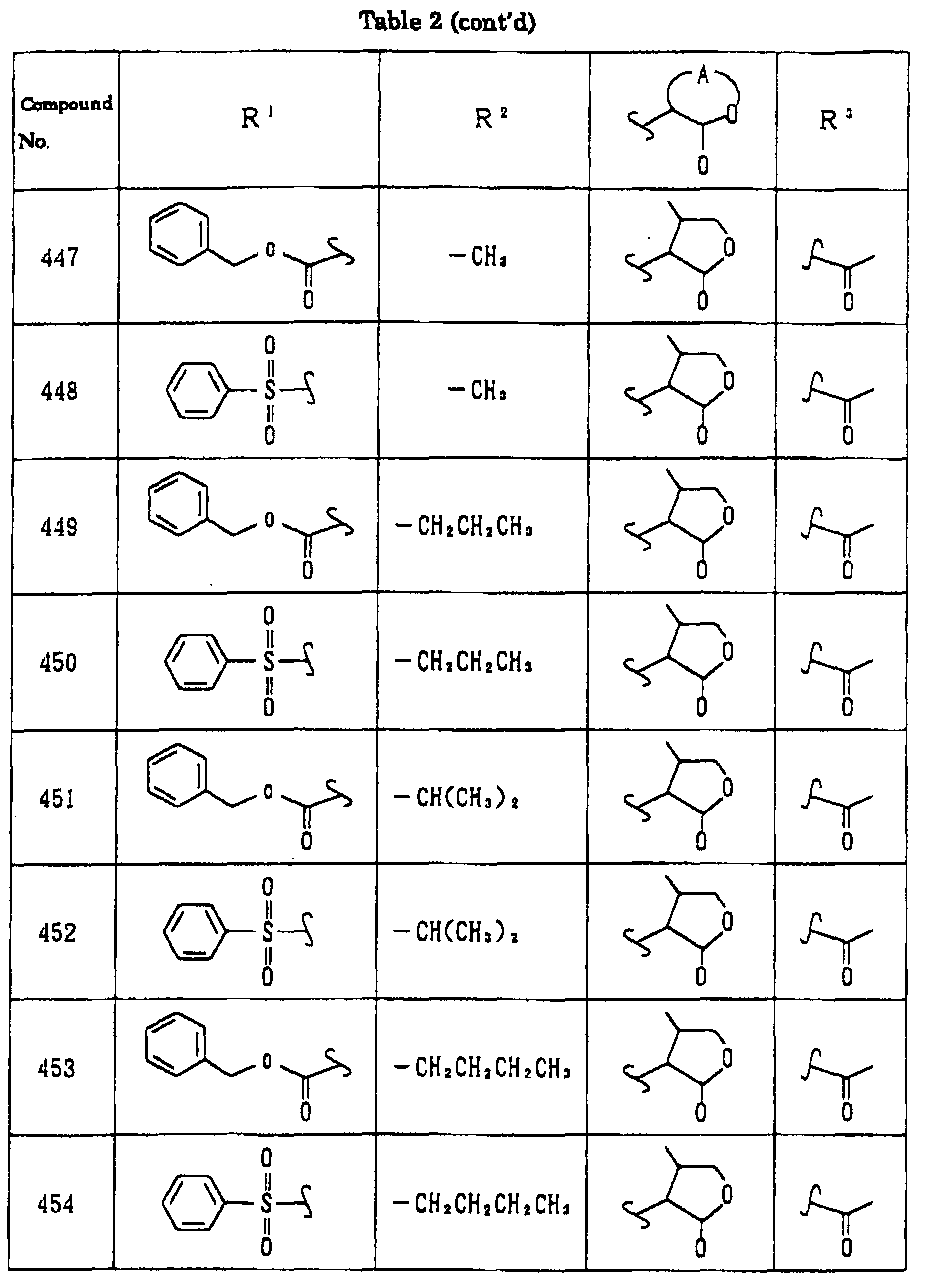

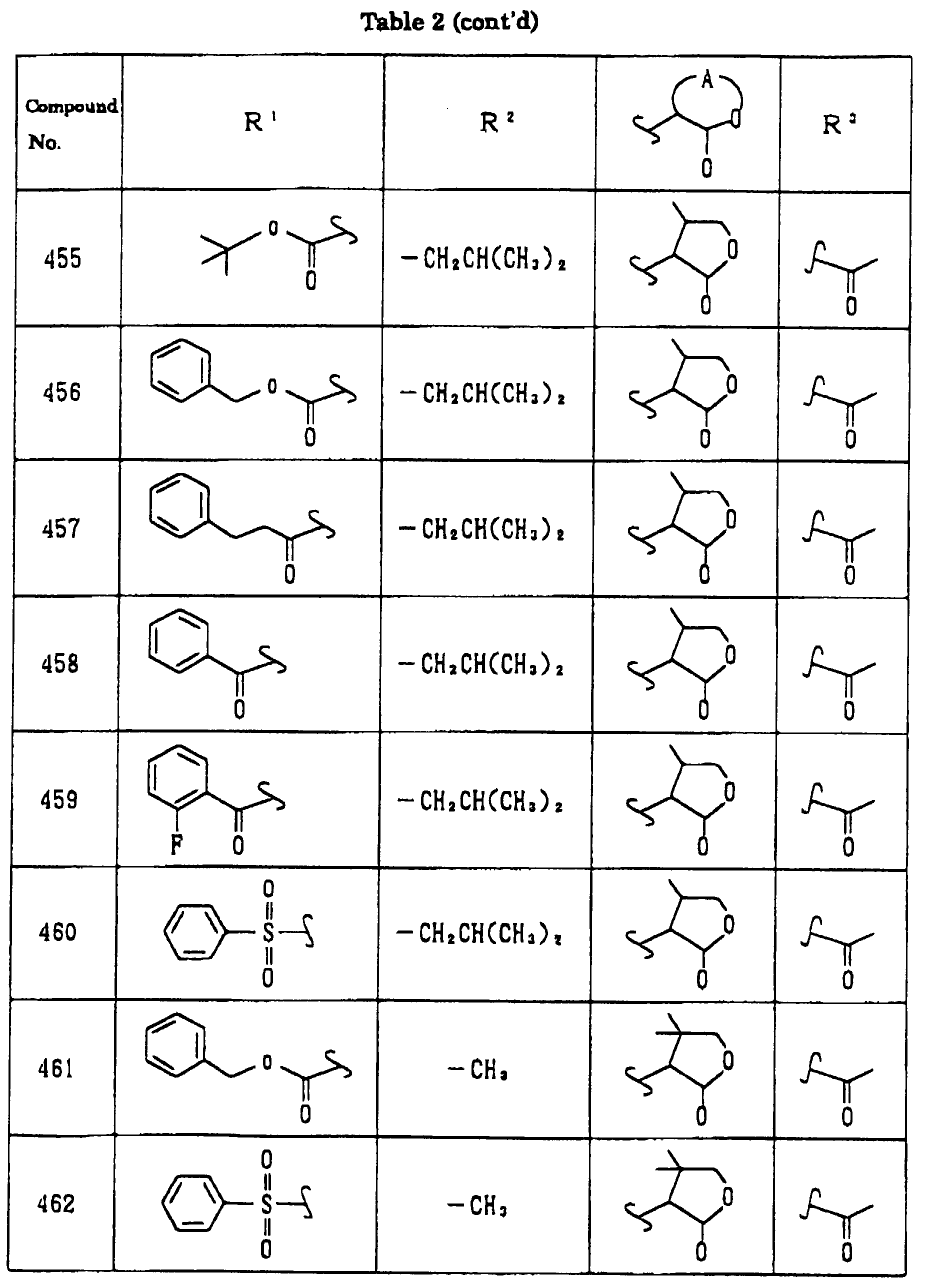

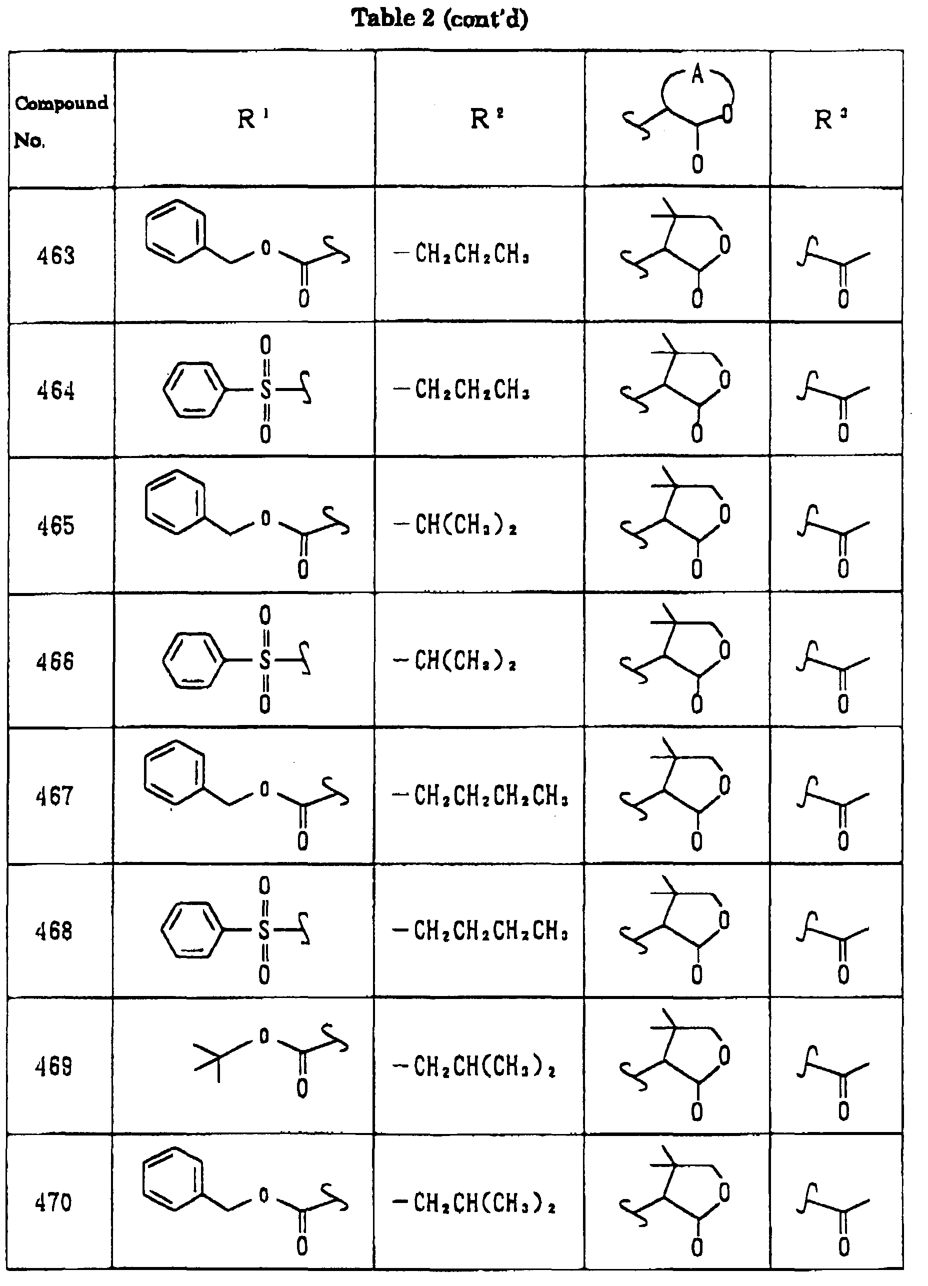

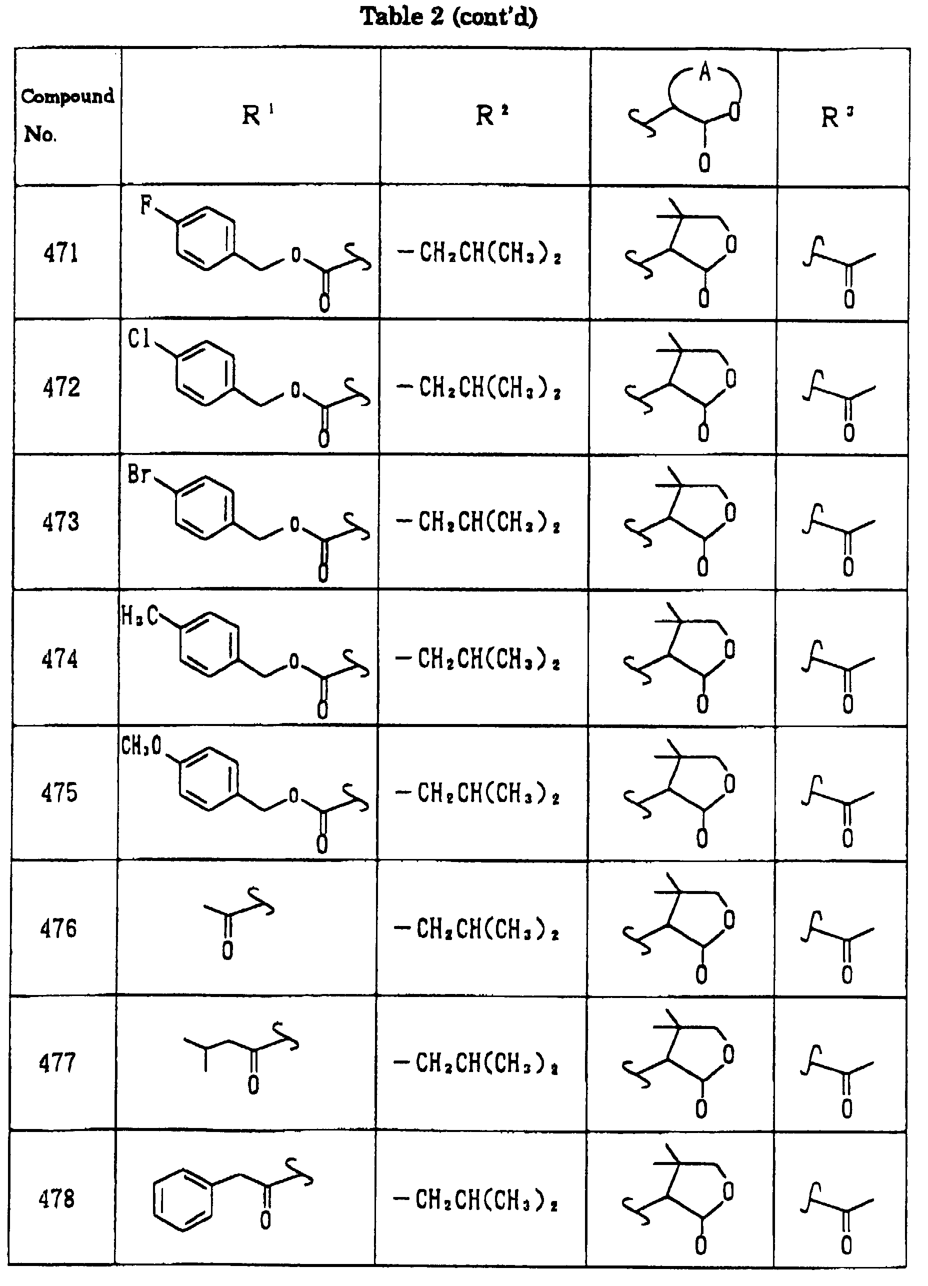

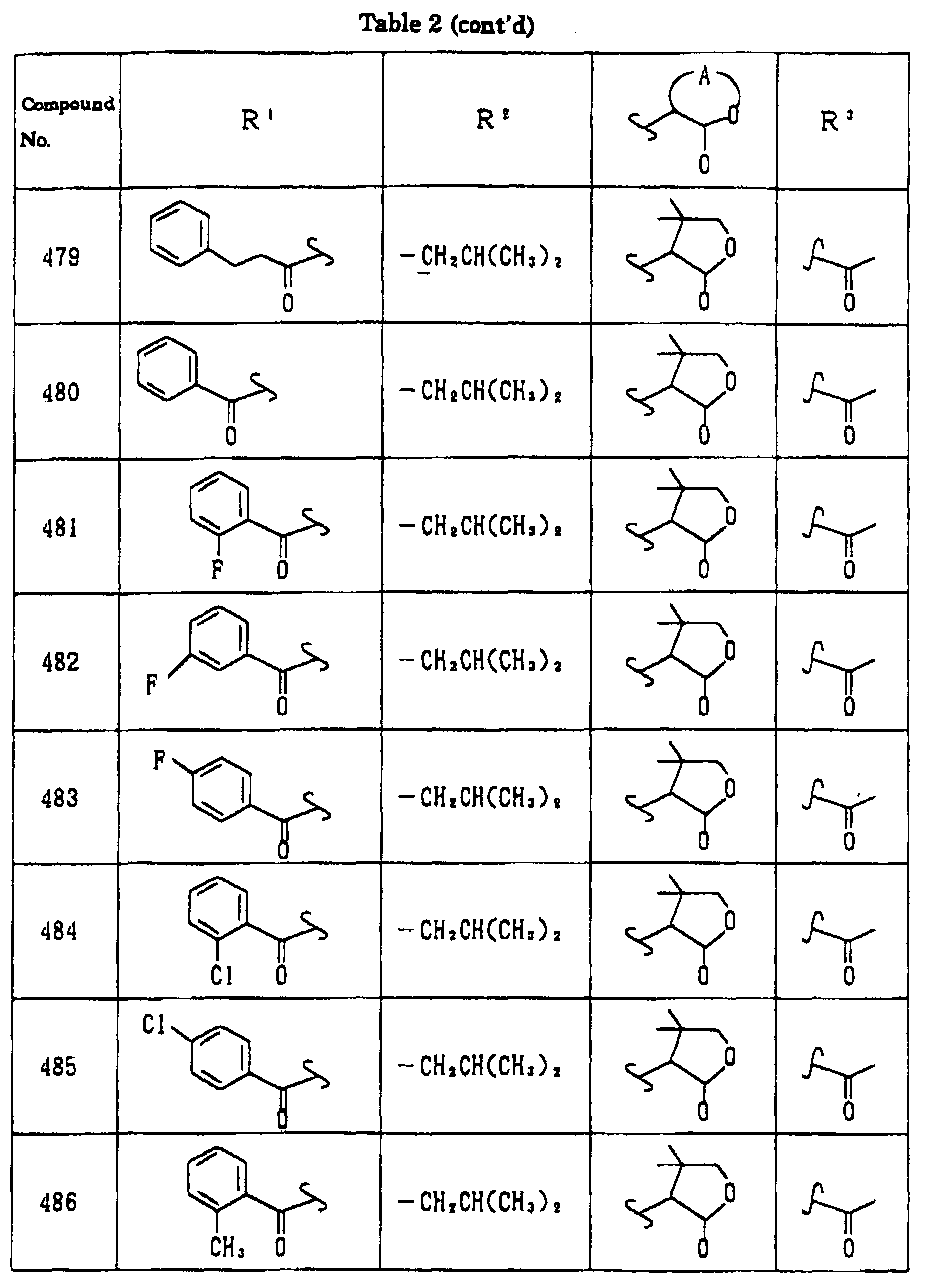

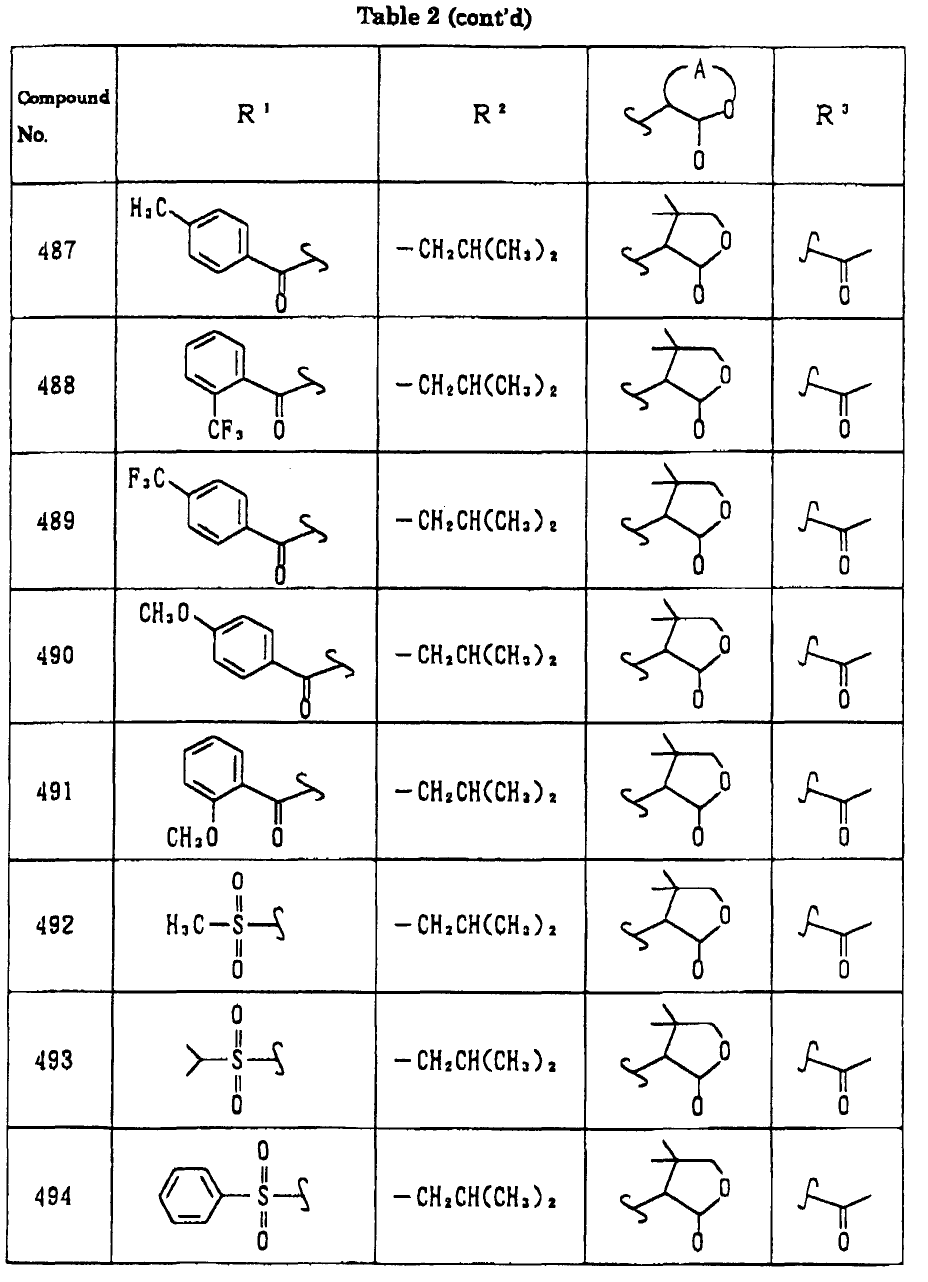

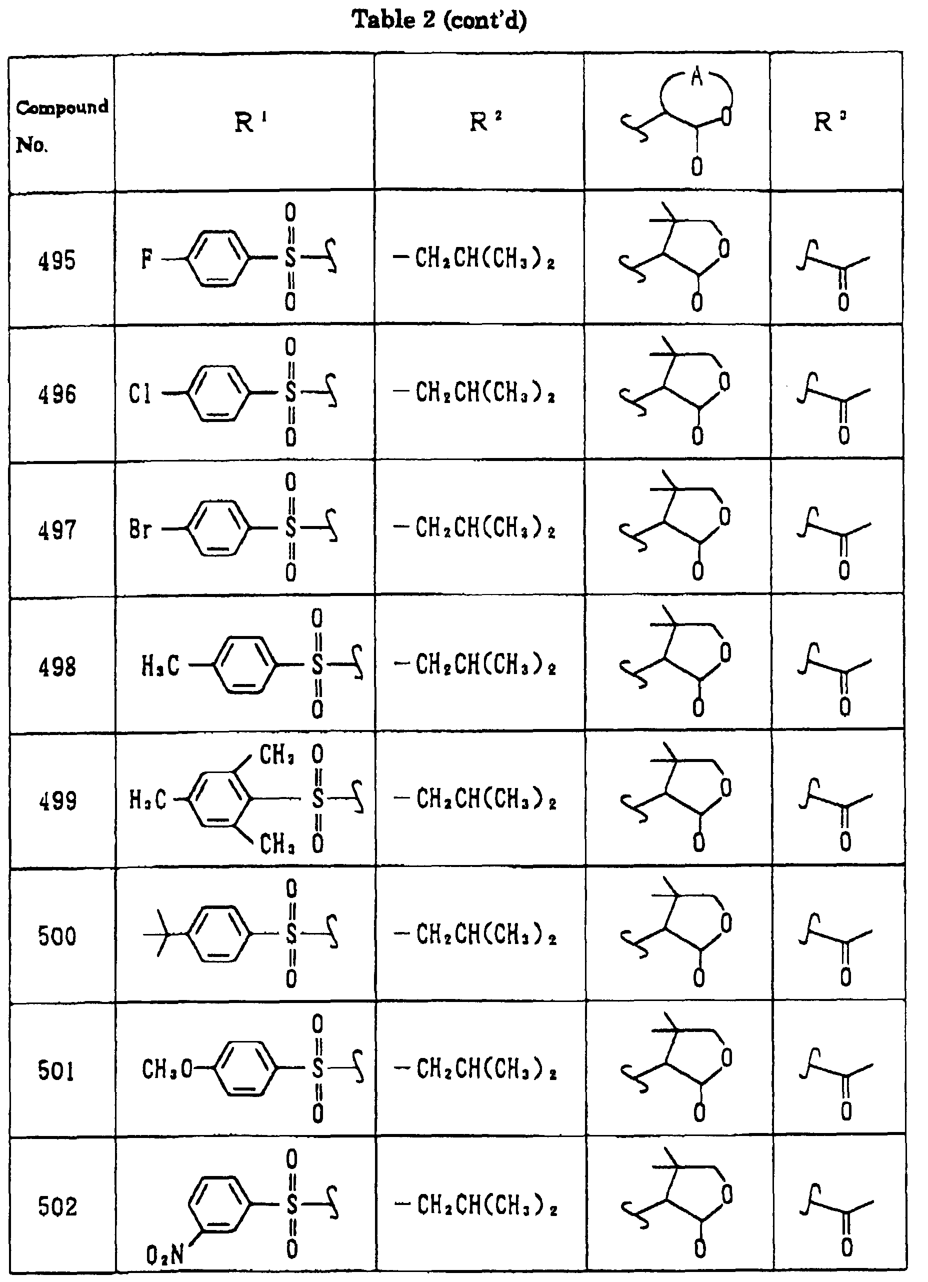

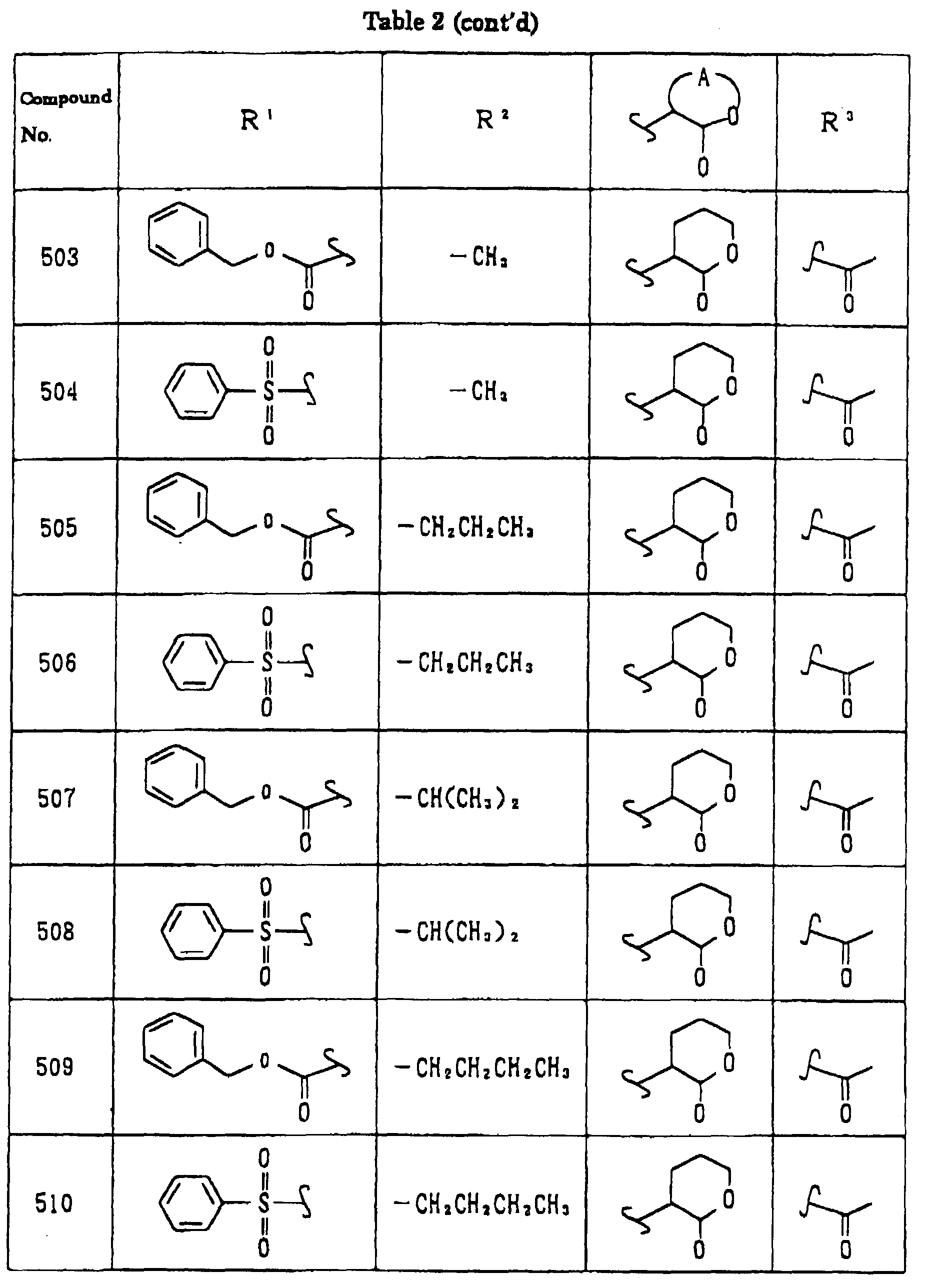

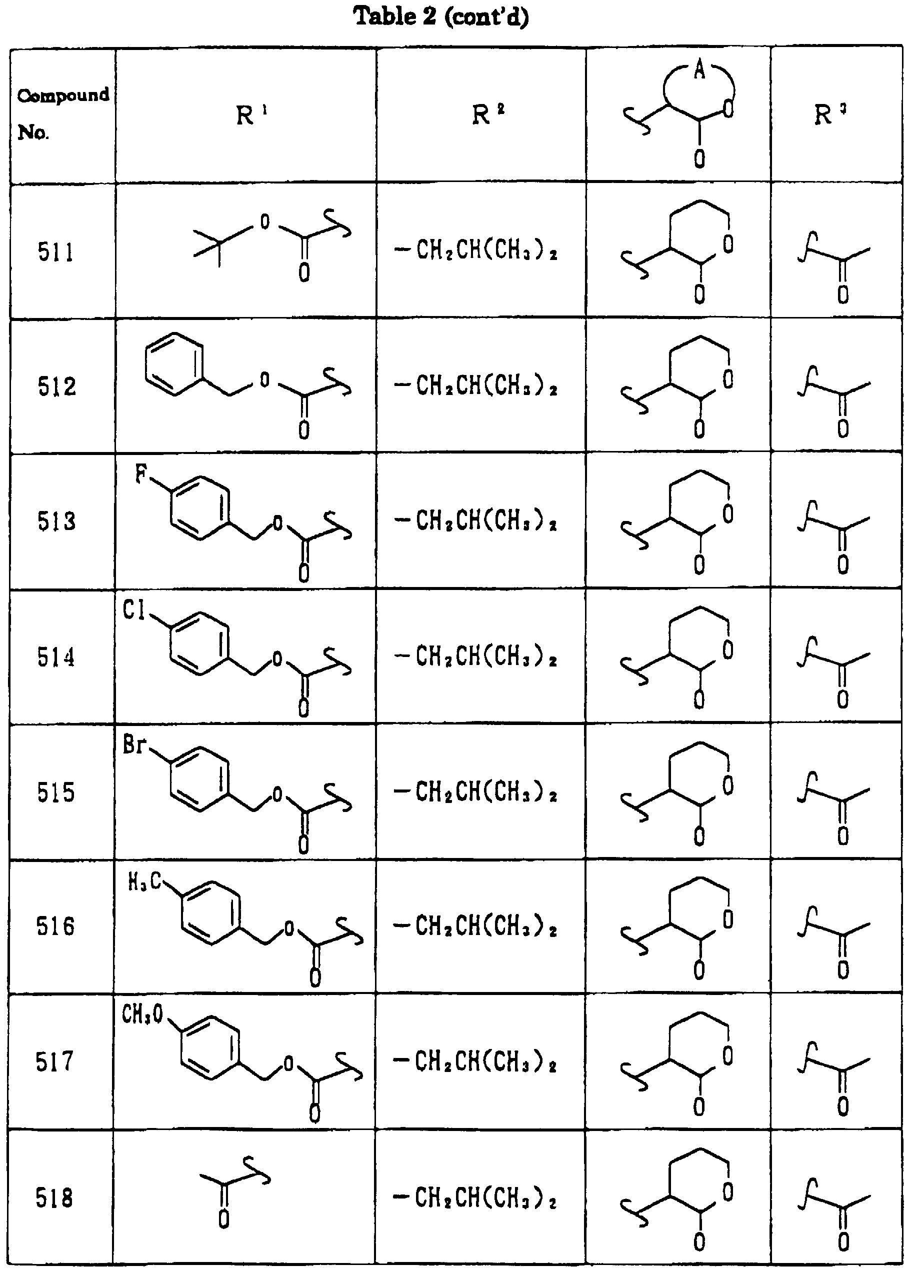

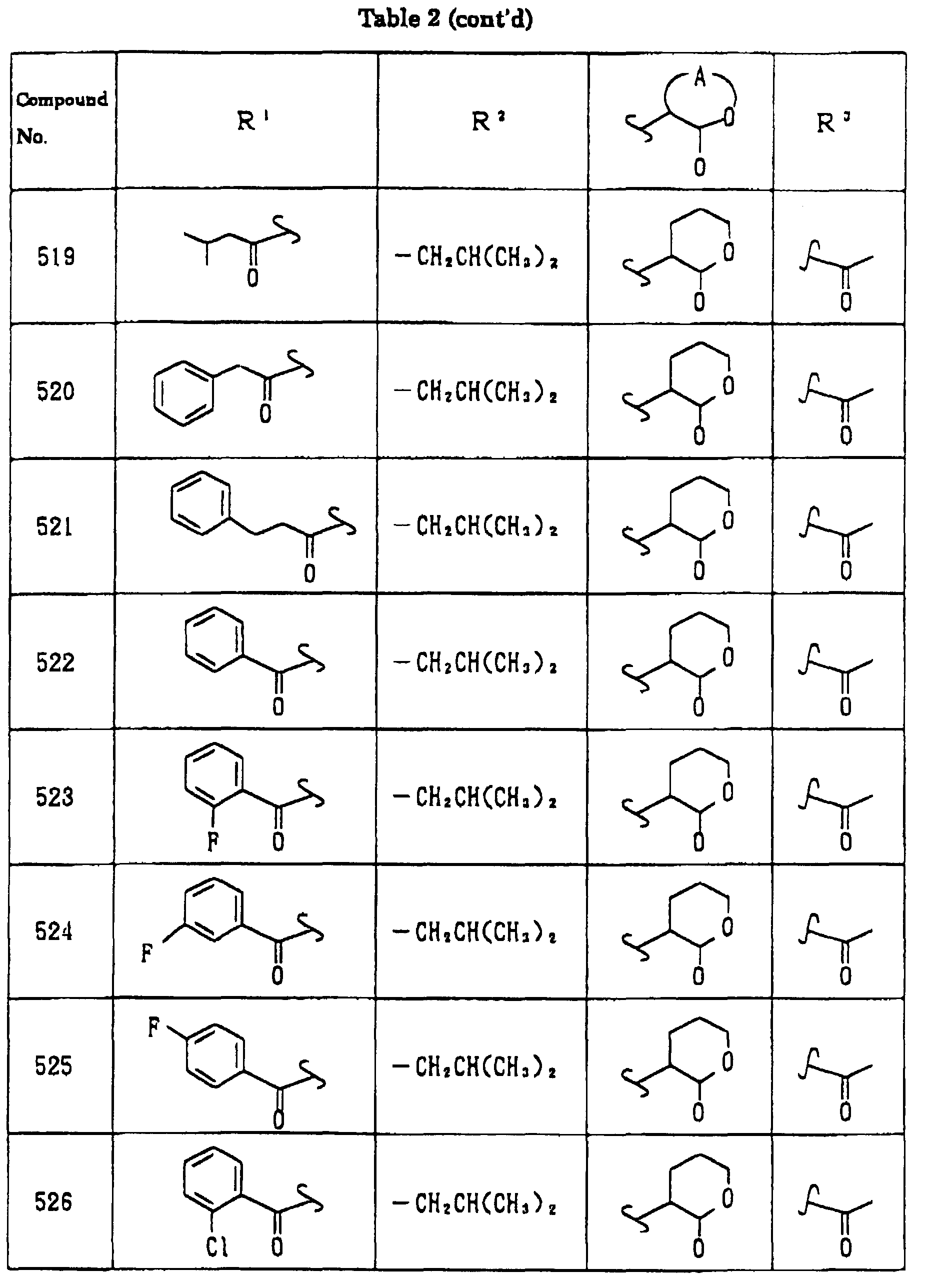

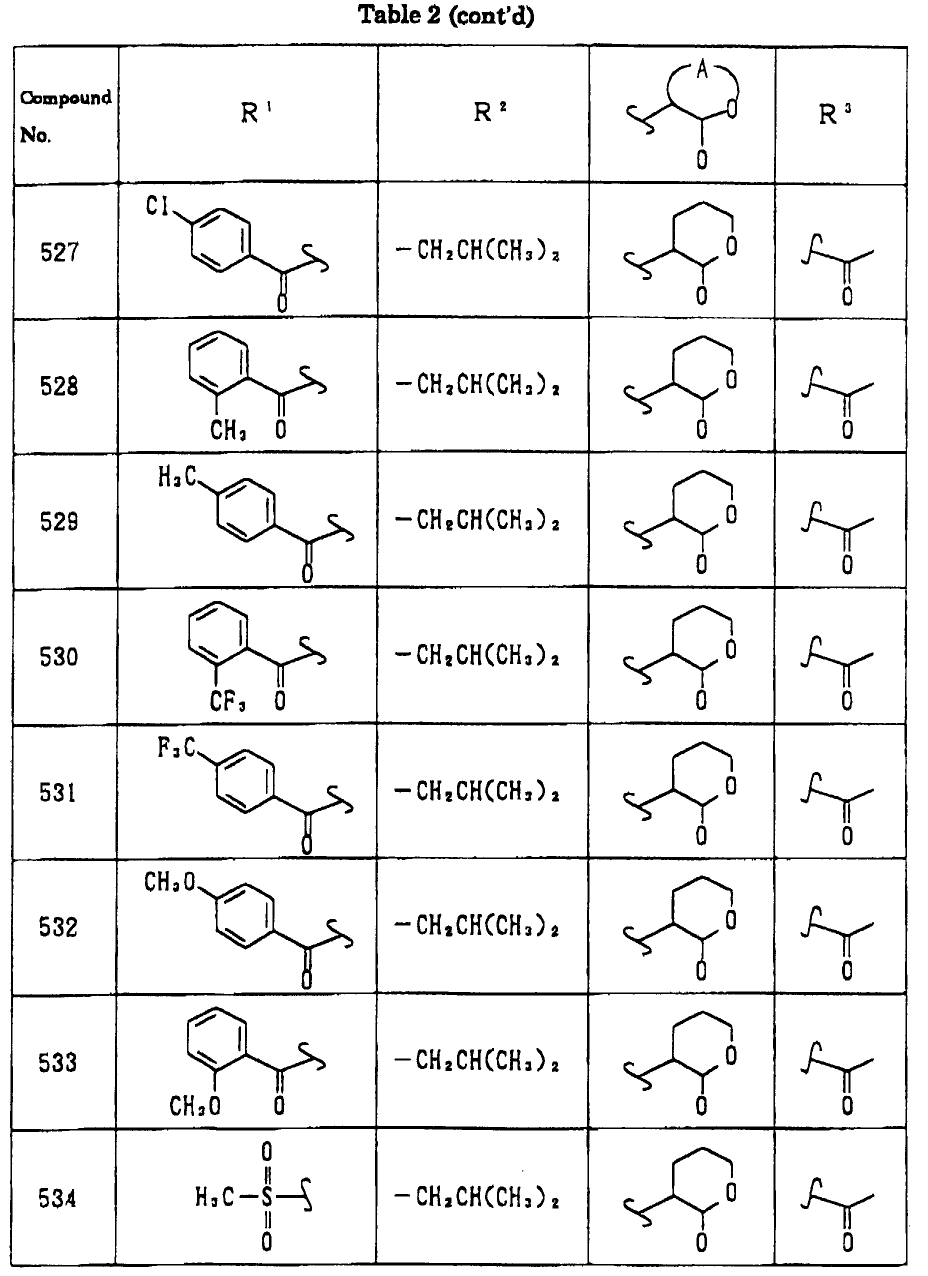

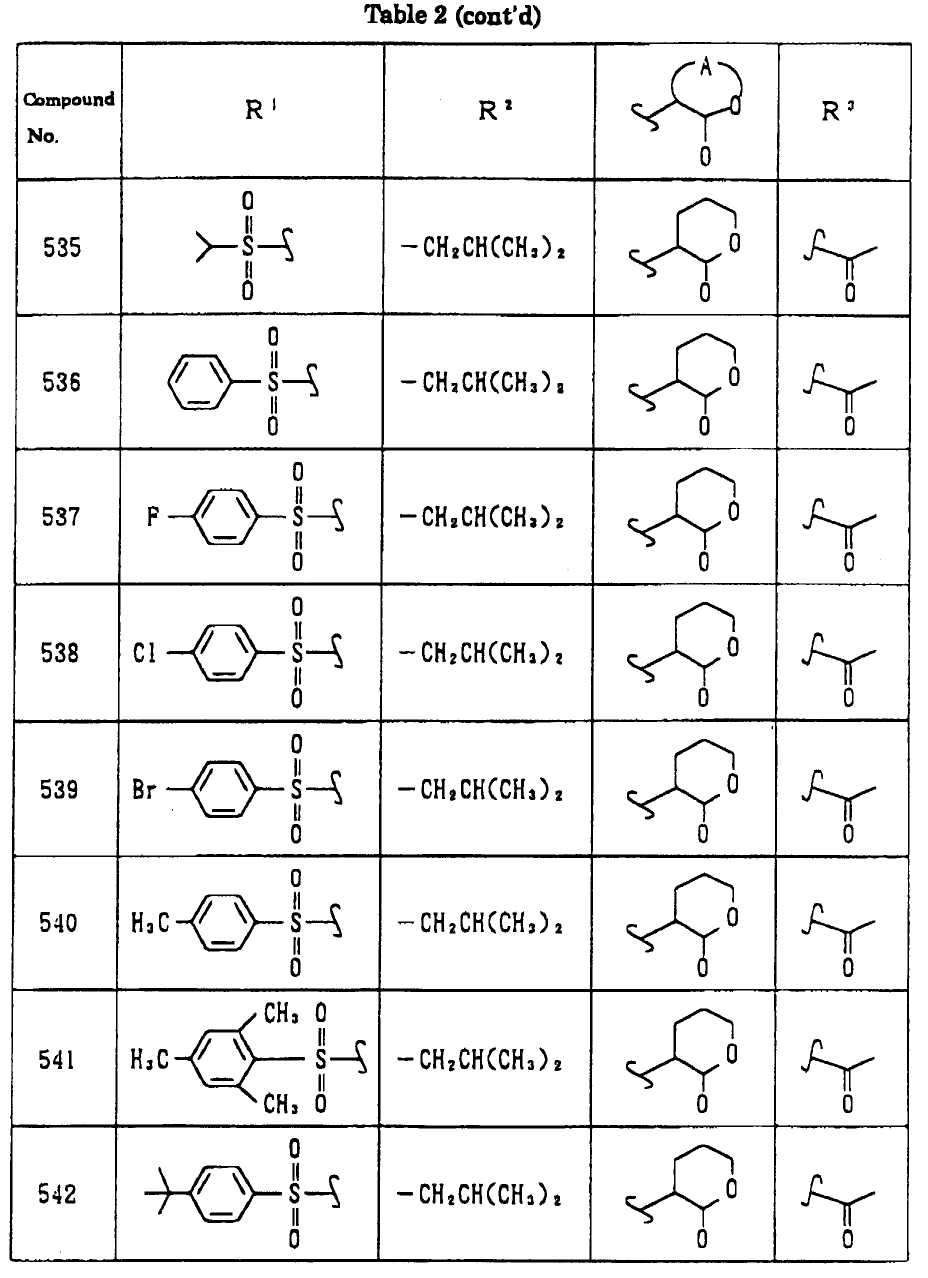

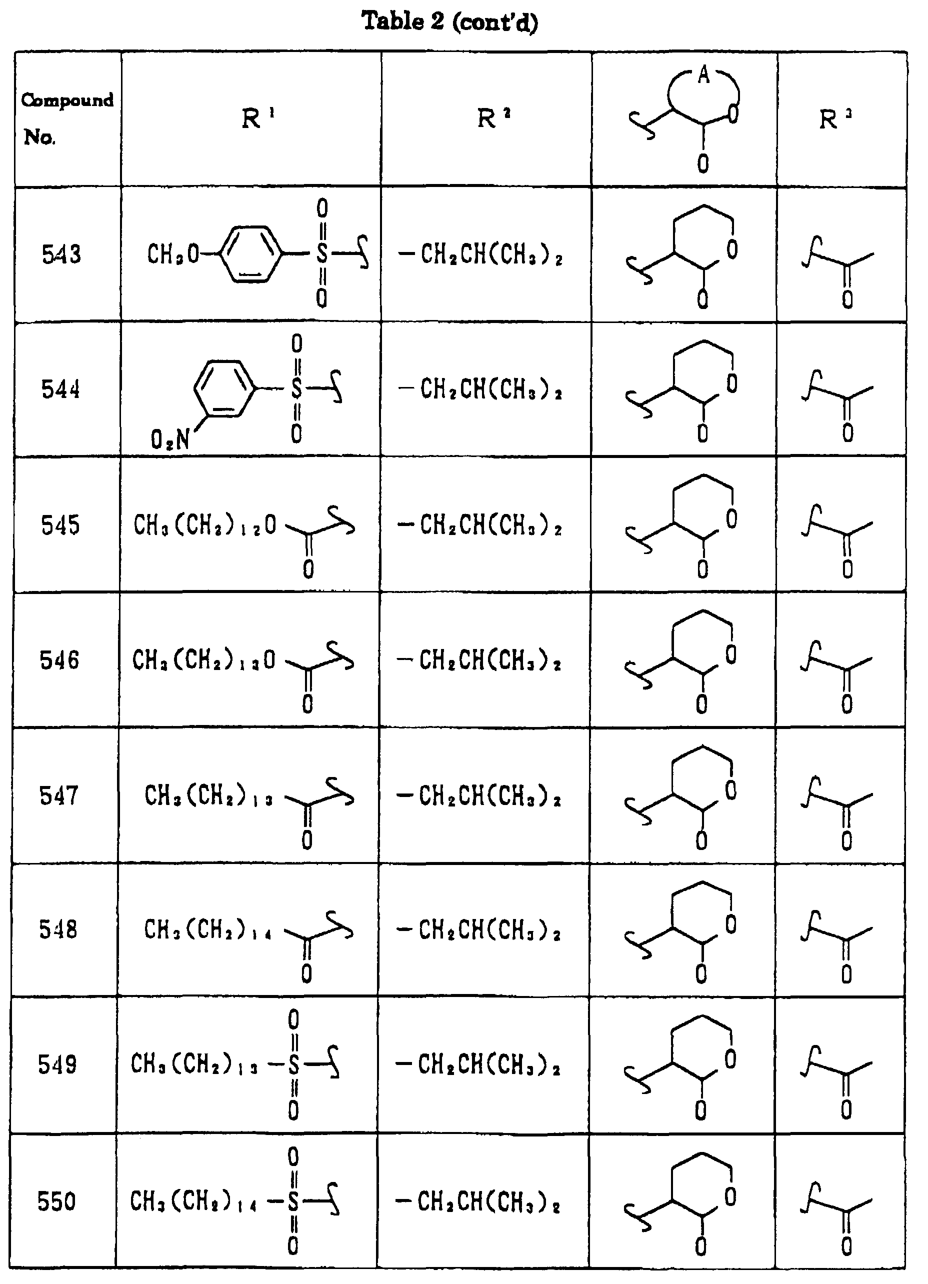

The active ingredient of the ischemic disease treating drug represented by the aforementioned general formula (I) can be produced for example by the following methods.

### Production Method 1

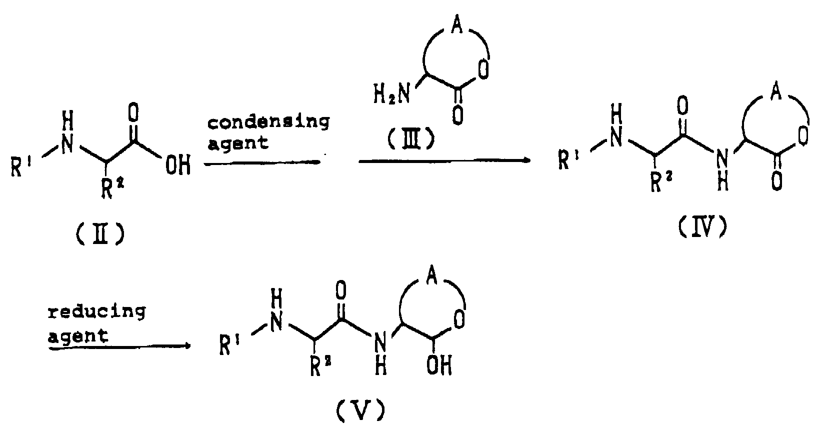

(In the above general formulae, R1, R2 and A are as defined in the foregoing.)

A compound represented by the aforementioned general formula (IV) can be obtained by allowing an amino acid derivative represented by the aforementioned general formula (II) to react with a condensing agent such as dicyclohexylcarbodiimide, diphenylphosphoryl azide, carbonyldiimidazole, oxalyl chloride, isobutyl chloroformate and thionyl chloride, if necessary in the presence of triethylamine, pyridine or the like base, thereby activating the carboxylic acid, and then allowing the thus activated compound to react with a lactone derivative represented by the aforementioned general formula (III). The solvent to be used in the condensation reaction can be optionally selected depending on each condensing agent, and the reaction can also be carried out under suitable conditions for each condensing agent. Next, a compound represented by the aforementioned general formula (V) can be obtained by treating the thus obtained compound (IV) with diisobutylaluminumhydride, sodiumborohydride/cerium chloride or the like reducing agent.

### Production Method 2

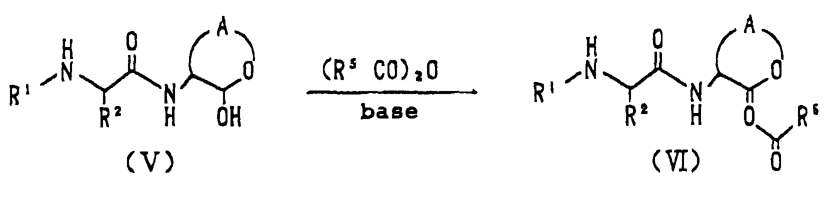

(In the above general formulae, R¹, R², R⁵ and A are as defined in the foregoing.)

A compound represented by the aforementioned general formula (VI) can be obtained by dissolving the compound (V) produced by the production method 1 in an organic solvent such as methylene chloride, 1,2-dichloroethane, dimethylformamide, N-methylpyrrolidone, tetrahydrofuran, ethyl acetate, acetonitrile and toluene, and allowing the thus dissolved compound to react with an acid anhydride represented by the aforementioned general formula (R⁵CO)₂O in the presence of pyridine, triethylamine, 4-dimethylaminopyridine or the like base. This reaction can be carried out in the absence of solvent.

In the series of reactions described above, protection and deprotection of functional groups may be required, and, in that case, the protecting group can be used by selecting a group suitable for each functional group, and the reactions can also be carried out using a known method.

Among compounds represented by the aforementioned general formula (I), the compound (V) in which R³ is hydrogen atom shows strong inhibitory activity upon a calpain, which is a cysteine protease. Also, the compound (VI) in which R³ is 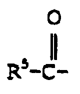 (R⁵ represents a C₁-C₁₀ alkyl group) can be used as a prodrug of the lactol derivative (V) which shows strong inhibitory activity against calpain. That is, when the compound (VI) is orally administered, it is absorbed from intestinal tracts and the like organs and then quickly converted into the active lactol derivative (V) by the action of enzymes and the like in the living body.

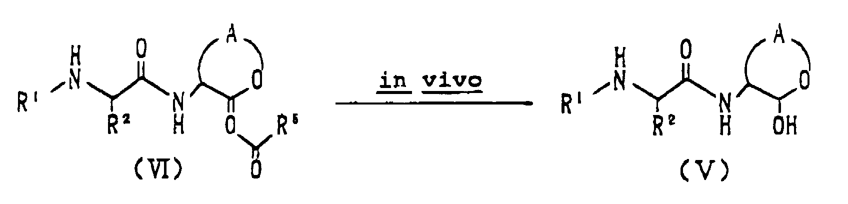

When the inside of the brain becomes an ischemic state such as the case of cerebral thrombosis or cerebral embolism, glutamic acid and the like excitatory amino acids are released in a large quantity from the presynaptic neuron and cause the activation of excitatory amino acid receptors of the postsynaptic neuron and the subsequent influx of Na⁺ and Ca²⁺ through the ion channel. When Na⁺ flows in the cells, depolarization progresses to open the Ca²⁺ channel of neuron, thus causing additional influx of Ca²⁺. By these reactions, Ca²⁺ concentration in the cells becomes extremely high to cause activation of calpain. Microtubule-associated proteins, fodrin and the like cytoskeletal proteins are known as the substrate of calpain, and hydrolysis of these proteins by the activated calpain causes destruction of cell membranes and subsequent cell death (*Dementia*, vol. 7, p. 161, 1993). Since it is considered that such a decomposition of cell membranes can probably be prevented when a calpain inhibitor is present in neuron at the time of cerebral ischemia, it is expected that a calpain inhibitor will be used as a medicament for cerebral ischemic diseases.

In the case of myocardial infarction which is an ischemic disease of the heart, the same phenomena as the case of nerve cells at the time of cerebral ischemia also occur in the heart muscle cells to cause activation of calpain, and it is considered that its inhibition is apt to occur at the time of reperfusion particularly in the heart muscle. Myofibril proteins such as myosin heavy chain, troponin T, troponin I, tropomyosin and α-actinin are known as the substrate of calpain (*Muscular Dystrophy*, p. 265, Tokyo University Press, 1980), and hydrolysis of these proteins by the activated calpain causes the death of heart muscle cells. In consequence, it is considered that a calpain inhibitor will be used as a medicament for myocardial infarction.

In applying the medicament of the present invention to the clinical medicine, ratio of the therapeutically effective component to the carrier component is generally from 1% by weight to 90% by weight. The medicament of the present invention may be used by oral administration through granules, fine subtilaes, powders, hard capsules, soft capsules, syrups, emulsions, suspensions, solutions and the like dosage forms, or by intravenous injection, intramuscular injections or subcutaneous injection in the form of injections. It can be used also as suppositories. Alternatively, it may be made into powder for injection use which is reconstructed prior to use. An organic or inorganic, solid or liquid carrier or diluent for pharmaceutical use which is suited for oral, rectal or parenteral administration can be used for the preparation of the medicament of the present invention. Examples of the filler to be used in the production of solid preparations include lactose, sucrose, starch, talc, cellulose, dextrin, kaolin, calcium carbonate and the like. Emulsions, syrups, suspensions, solutions and the like liquid preparations for use in oral administration contain water, plant oil or the like generally used inert diluent. In addition to the inert diluent, the preparation may further contain moistening agents, suspension assisting agents, sweeteners, aromatics, coloring agents, preservatives and the like auxiliary agents. It may be made into a liquid preparation and included in capsules made of gelatin or the like absorbable material. Examples of the solvent or suspending agent to be used in the production of parenteral administration preparations such as injections and suppositories include water, propylene glycol, polyethylene glycol, benzyl alcohol, ethyl oleate, lecithin and the like. Examples of the base to be used in suppositories include cacao butter, lauric type butter, Witepsol and the like. These pharmaceutical preparations can be prepared in the ordinary method.

When used by oral administration, the clinical dose is generally from 0.01 to 1,000 mg per day per adult as the compound of active ingredient, but it is desirable to increase or decrease the dose optionally depending on the age, morbid state and symptoms of each patient. The just described daily dose of the medicament of the present invention may be administered once a day, divided into 2 or 3 doses per day and administered at appropriate intervals or used by intermittent administration.

When used as injections, it is desirable to use the medicament by continuous or intermittent administration in a daily dose of from 0.001 to 100 mg per adult as the compound of the present invention.

### EXAMPLES:

The following Reference Examples, Inventive Examples and Test Examples are provided to further illustrate the invention, but these examples are not to be construed to limit the scope of the invention.

### Reference Example 1 Production of (S)-3-((S)-4-methyl-2-phenylsulfonylaminovalerylamino)-2-tetrahydrofuranone

A 6 ml portion of thionyl chloride was cooled to -5°C and mixed with 998 mg of N-phenylsulfonyl-L-leucine, the mixture was stirred at -5°C for 10 minutes and then warmed up to room temperature, and the stirring was further continued for 3 hours. Next, the reaction solution was concentrated under a reduced pressure, and the thus obtained residue was mixed with 10 ml of toluene and concentrated, thereby obtaining crude N-phenylsulfonyl-L-leucyl=chloride as the residue. The thus obtained crude N-phenylsulfonyl-L-leucyl=chloride was dissolved in 20 ml of methylene chloride, and the solution was cooled in an ice bath and mixed with 443 mg of L-homoserinelactone hydrochloride and 0.946 ml of triethylamine. The reaction solution was stirred for 15 minutes while cooling in an ice bath and then stirred for 1.5 hours at room temperature. After completion of the reaction, the reaction solution was mixed with dilute hydrochloric acid and extracted with methylene chloride. The extract was washed with water, saturated sodium hydrogencarbonate aqueous solution and saturated brine in that order, and then dried over magnesium sulfate which was subsequently removed by filtration. The resulting filtrate was concentrated, the thus obtained residue was mixed with 10 ml of ethyl acetate and 20 ml of hexane and stirred, and then the thus formed crystals were collected by filtration to obtain 861 mg of the compound of interest.
Yield: 76%
Melting point: 183 - 184°C
IR (KBr, cm⁻¹): 3331, 3256, 1772, 1649.
NMR (CDCl₃, δ): 0.67 (d, J = 6.0 Hz, 3 H), 0.84 (d, J = 6.3 Hz, 3 H), 1.45 - 1.56 (m, 3 H), 2.01 (m, 1 H), 2.63 (m, 1 H), 4.26 (m, 1 H), 4.36 (m, 1 H), 4.45 (ddd, J = 9.3 Hz, 9.3 Hz, 1.8 Hz, 1 H), 5.28 (d, J = 8.1 Hz, 1 H), 6.67 (d, J = 6.0 Hz, 1 H), 7.52 (m, 2 H), 7.60 (m, 1 H), 7.88 (dd, J = 7.2 Hz, 1.5 Hz, 2 H).

### Inventive Example 1 Production of (3S)-3-((S)-4-methyl-2-phenylsulfonylaminovalerylamino)-2-tetrahydrofuranol (Compound No.. 135 in Table 1)

A 413 mg portion of (S)-3-((S)-2-phenylsulfonylamino-4-methylvalerylamino)-2-tetrahydrofuranone obtained in Reference Example 1 was dissolved in 60 ml of methylene chloride and cooled to -78°C. Next, to this solution was added 3.81 ml of toluene solution containing 1.01 mol/l of diisobutylaluminum hydride. Three hours thereafter, the reaction solution was mixed with saturated ammonium chloride aqueous solution and ethyl acetate, returned to room temperature and then filtered through celite which was subsequently washed thoroughly with ethyl acetate. The resulting filtrate was washed with saturated brine and then dried over magnesia sulfate which was subsequently removed by filtration. The thus obtained filtrate was concentrated, and the resulting residue was purified by a silica gel column chromatography (developing solvent, ethyl acetate containing 30% hexane) to obtain 192 mg of the compound of interest.
Yield: 46%
Melting point: 162°C
IR (KBr, cm⁻¹): 3337, 3260, 1649.
NMR (CDCl₃ + DMSO-d₆, δ): 0.72 (d, J = 6.6 Hz, 2.7 H), 0.78 (d, J = 6.3 Hz, 0.3 H), 0.84 (d, J = 6.6 Hz, 2.7 H), 0.86 (d, J = 6.3 Hz, 0.3 H), 1.46 (t, J = 7.2 Hz, 2 H), 1.63 (m, 2 H), 2.09 (m, 0.9 H), 2.25 (m, 0.1 H), 3.68 - 3.81 (m, 2 H), 3.99 - 4.12 (m, 26), 4.95 (s, 0.1 H), 5.03 (d, J = 3.6 Hz, 0.1 H), 5.15 (dd, J = 3.9 Hz, 3.9 Hz, 0.9 H), 5.63 (d, J = 3.9 Hz, 0.9 H), 6.68 (d, J = 9.3 Hz, 0.1 H), 6.81 (d, J = 8.1 Hz, 0.9 H), 6.89 (d, J = 7.8 Hz, 0.9 H), 7.14 (d, J = 7.2 Hz, 0.1 H), 7.46 - 7.58 (m, 3 H), 7.85 (m, 2 H).

### Inventive Example 2 Production of (2S,3S)-2-acetoxy-3-((S)-4-methyl-2-phenylsulfonylaminovalerylamino)tetrahydrofuran (Compound No.. 405 in Table 2)

A 244 mg portion of (S)-3-((S)-4-methyl-2-phenylsulfonylaminovalerylamino)-2-tetrahydrofuranone obtained in Reference Example 1 was dissolved in 35 ml of methylene chloride, and the solution was cooled to -78°C and mixed with 1.91 ml of toluene solution containing 1.01 mol/l of diisobutylaluminum hydride. After 3 hours of stirring at -78°C, the reaction solution was mixed with saturated ammonium chloride aqueous solution and ethyl acetate, returned to room temperature and then filtered through celite which was subsequently washed thoroughly with ethyl acetate. The resulting filtrate was washed with saturated brine and then dried over magnesium sulfate which was subsequently removed by filtration. The resulting filtrate was concentrated to obtain crude (3S)-3-((S)-4-methyl-2-phenylsulfonylaminovalerylamino)-2-tetrahydrofuranol (compound of Inventive Example 1). This was dissolved in 1 ml of pyridine, the solution was cooled in an ice bath and mixed with 1.5 ml of acetic anhydride and then the mixture was stirred at the same temperature for 9 hours, mixed with 1.5 ml of methanol and concentrated. The thus obtained residue was dissolved in ethyl acetate, washed with dilute hydrochloric acid, water, saturated sodium hydrogencarbonate aqueous solution and saturated brine in that order and then dried over magnesium sulfate which was subsequently removed by filtration. The filtrate was concentrated, the thus obtained residue was mixed with 2.5 ml of ethyl acetate and 2.5 ml of hexane and stirred, and then the thus formed crystals were collected by filtration to obtain 120 mg of the compound of interest.
Yield: 44%
Melting point: 177 - 178°C
IR (KBr, cm⁻¹): 3409, 3100, 1753, 1659.
NMR (CDCl₃, δ): 0.54 (d, J = 6.3 Hz, 3 H), 0.80 (d, J = 6.3 Hz, 3 H), 1.37 (m, 2 H), 1.59 (m, 1 H), 1.81 (m, 1 H), 2.16 (s, 3 H), 2.24 (m, 1 H), 3.61 (m, 1 H), 3.95 (ddd, J = 9.3 Hz, 9.0 Hz, 7.5 Hz, 1 H), 4.14 (ddd, J = 9.3 Hz, 9.0 Hz, 3.0 Hz, 1 H), 4.53 (m, 1 H), 4.87 (d, J = 6.6 Hz, 1 H), 6.16 (d, J = 4.5 Hz, 1 H), 6.56 (d, J = 8.7 Hz, 1 H), 7.54 (m, 2 H), 7.62 (m, 1 H), 7.86 (dd, J = 7.2 Hz, 1.5 Hz, 2 H).

Compounds of Inventive Examples 3 to 42 were produced by similar methods of Reference Example 1 and Inventive Examples 1 and 2. Their physical property values are shown in the following.

### Inventive Example 3 Production of (3S)-3-((S)-4-methyl-2-tetradecyloxycarbonylaminovalerylamino)-2-tetrahydrofuranol (Compound No.. 26 in Table 1)

IR (KBr, cm⁻¹): 3410, 3301, 1696, 1649, 1543.
NMR (CDCl₃, δ): 0.83 - 0.98 (m, 9 H), 1.25 (m, 22 H), 1.43 - 1.70 (m, 5 H), 1.70 - 1.95 (m, 1 H), 2.23 - 2.52 (m, 1 H), 3.89 (m, 0.7 H), 3.95 - 4.24 (m, 5 H), 4.34 (m, 1 H), 4.50 (s, 0.3 H), 5.22 - 5.44 (m, 2 H), 6.72 (d, J = 7.7 Hz, 1 H).
Inventive Example 4 Production of (3S)-3-((S)-2-benzyloxycarbonylamino-4-methylvalerylamino)-2-tetrahydrofuranol (Compound No.. 30 in Table 1)
Melting point: 40 - 43°C
IR (KBr, cm⁻¹): 3306, 1705, 1657.
NMR (CDCl₃, δ): 0.92 (d, J = 6.1 Hz, 3 H), 0.94 (d, J = 5.9 Hz, 3 H), 1.52 (m, 1 H), 1.64 (m, 2 H), 1.78 (m, 1 H), 2.29 (m, 0.5 H), 2.41 (m, 0.5 H), 3.51 (s, 0.5 H), 3.74 (s, 0.5 H), 3.85 (ddd, J = 8.0 Hz, 8.0 Hz, 8.0 Hz, 0.5 H), 3.97 (m, 0.5 H), 4.10 (m, 2 H), 4.32 (m, 1 H), 5.09 (s, 1 H), 5.10 (s, 1 H), 5.24 (s, 0.5 H), 5.29 (s, 0.5 H), 5.35 (d, J = 6.5 Hz, 0.5 H), 5.38 (d, J = 8.2 Hz, 0.5 H), 6.45 (d, J = 6.0 Hz, 0.5 H), 6.57 (d, J = 6.0 Hz, 0.5 H), 7.33 (m, 5 H).

### Inventive Example 5 Production of (3S)-3-{(S)-2-(2-chlorobenzyloxycarbonylamino)-4-methylvalerylamino}-2-tetrahydrofuranol (Compound No.. 34 in Table 1)

Melting point: 46 - 49°C
IR (KBr, cm⁻¹): 3308, 1707, 1657, 1541.
NMR (CDCl₃, δ): 0.94 (m, 6 H), 1.48 - 1.86 (m, 4 H), 2.32 (m, 0.6 H), 2.48 (m, 0.4 H), 2.90 (s, 0.4 H), 3.09 (s, 0.6 H), 3.88 (ddd, J = 7.8 Hz, 7.8 Hz, 7.8 Hz, 0.6 H), 4.01 (m, 0.4 H), 4.11 (m, 2 H), 4.34 (m, 1 H), 5.23 (s, 2 H), 5.25 (m, 2 H), 6.18 (s, 0.4 H), 6.45 (d, J = 7.6 Hz, 0.6 H), 7.26 (m, 2 H), 7.40 (m, 2 H).

### Inventive Example 6 Production of (3S)-3-{(S)-4-methyl-2-(4-methylbenzyloxycarbonylamino)valerylamino}-2-tetrahydrofuranol (Compound No.. 42 in Table 1)

IR (KBr, cm⁻¹): 3310, 1703, 1657, 1539.
NMR (CDCl₃, δ): 0.93 (m, 6 H), 1.52 (m, 1 H), 1.58 - 1.86 (m, 3 H), 2.29 (m, 0.5 H), 2.35 (s, 3 H), 2.43 (m, 0.5 H), 2.91 (s, 0.5 H), 3.03 (s, 0.5 H), 3.87 (ddd, J = 7.8 Hz, 7.8 Hz, 7.8 Hz, 0.5 H), 3.97 (m, 0.5 H), 4.10 (m, 2 H), 4.33 (m, 1 H), 5.06 (s, 2 H), 5.14 (m, 1 H), 5.26 (m, 1 H), 6.20 (s, 0.5 H), 6.44 (s, 0.5 H), 7.16 (m, 2 H), 7.23 (m, 2 H).

### Inventive Example 7 Production of (3S)-3-((S)-2-cyclohexyloxycarbonylamino-4-methylvalerylamino)-2-tetrahydrofuranol (Compound No.. 47 in Table 1)

Melting point: 28 - 30°C
IR (KBr, cm⁻¹): 3310, 1696, 1657, 1539.
NMR (CDCl₃, δ): 0.95 (m, 6 H), 1.29 (m, 2 H), 1.36 (m, 4 H), 1.53 (m, 2 H), 1.69 (m, 4 H), 1.85 (m, 2 H), 2.36 (m, 0.5 H), 2.48 (m, 0.5 H), 2.85 (s, 0.5 H), 3.06 (s, 0.5 H), 3.89 (ddd, J = 7.8 Hz, 7.8 Hz, 7.8 Hz, 0.5 H), 4.02 (m, 0.5 H), 4.14 (m, 2 H), 4.36 (m, 1 H), 4.63 (m, 1 H), 4.99 (m, 1 H), 5.26 (m, 0.5 H), 5.32 (m, 0.5 H), 6.22 (s, 0.5 H), 6.47 (s, 0.5 H).

### Inventive Example 8 Production of (3S)-3-((S)-4-methyl-2-pentadecanoylaminovalerylamino)-2-tetrahydrofuranol (Compound No.. 64 in Table 1)

Melting point: 99 - 101°C
IR (KBr, cm⁻¹): 3298, 1636, 1543.
NMR (CDCl₃, δ): 0.81 - 1.05 (m, 9 H), 1.25 (m, 22 H), 1.42 - 1.73 (m, 5 H), 1.83 (m, 1 H), 2.12 - 2.23 (m, 2 H), 2.30 (m, 1 H), 3.04 (m, 0.3 H), 3.57 (m, 0.7 H), 3.89 (m, 0.7 H), 4.02 (m, 0.3 H), 4.12 (m, 1 H), 4.23 - 4.53 (m, 2 H), 5.27 (s, 0.3 H), 5.33 (d, J = 4.3 Hz, 0.7 H), 5.97 (d, J = 8.0 Hz, 1 H), 6.53 (d, J = 8.4 Hz, 0.3 H), 6.61 (d, J = 8.3 Hz, 0.7 H).

### Inventive Example 9 Production of (3S)-3-((S)-2-hexadecanoylamino-4-methylvalerylamino)-2-tetrahydrofuranol (Compound No.. 65 in Table 1)

Melting point 94 - 96°C
IR (KBr, cm⁻¹): 3414, 3297, 1715, 1543.
NMR (CDCl₃, δ): 0.81 - 1.01 (m, 9 H), 1.25 (m, 24 H), 1.40 - 1.80 (m, 5 H), 1.85 (m, 1 H), 2.10 - 2.23 (m, 2 H), 2.32 (m, 0.6 H), 2.41 (m, 0.4 H), 3.83 (m, 0.6 H), 4.02 (m, 0.4 H), 4.10 (m, 1 H), 4.31 (m, 1 H), 4.43 (m, 1 H), 5.27 (s, 0.4 H), 5.32 (d, J = 4.6 Hz, 0.6 H), 6.01 (d, J = 8.2 Hz, 1 H), 6.57 (d, J = 7.5 Hz, 0.4 H), 6.64 (d, J = 8.3 Hz, 0.6 H).

### Inventive Example 10 Production of (3S)-3-{(S)-2-(2-fluorobenzoylamino)-4-methylvalerylamino}-2-tetrahydrofuranol (Compound No.. 75 in Table 1)

Melting point: 62 - 64°C
IR (KBr, cm⁻¹): 3306, 1644, 1534.
NMR (CDCl₃, δ): 0.80 - 1.07 (m, 6 H), 1.59 - 2.01 (m, 4H), 2.40 (m, 1 H), 3.82 (m, 1 H), 3.97 - 4.20 (m, 1.6 H), 4.24 - 4.45 (m, 1.4 H), 4.68 (m, 1 H), 5.31 (d, J = 2.5 Hz, 0.6 H), 5.35 (dd, J = 4.2 Hz, 4.1 Hz, 0.4 H), 6.87 (m, 1 H), 7.02 - 7.20 (m, 2 H), 7.25 (m, 1 H), 7.50 (m, 1 H), 8.00 (m, 1 H).

### Inventive Example 11 Production of (3S)-3-{(S)-2-(4-chlorobenzoylamino)-4-methylvalerylamino}-2-tetrahydrofuranol (Compound No.. 83 in Table 1)

Melting point: 93 - 96°C
IR (KBr, cm⁻¹): 3295, 1636.
NMR (CDCl₃, δ): 0.95 (d, J = 6.0 Hz, 3 H), 0.97 (d, J = 5.7 Hz, 3 H), 1.73 (m, 3 H), 1.86 (m, 1 H), 2.31 (m, 0.7 H), 2.42 (m, 0.3 H), 3.41 (s, 0.3 H), 3.86 (s, 0.7 H), 3.87 (ddd, J = 8.4 Hz, 8.4 Hz, 8.4 Hz, 0.7 H), 4.02 (ddd, J = 7.8 Hz, 7.8 Hz, 7.8 Hz, 0.3 H), 4.12 (m, 1 H), 4.35 (m, 1 H), 4.68 (m, 1 H), 5.30 (s, 0.3 H), 5.35 (d, J = 4.5 Hz, 0.7 H), 6.71 (d, J = 8.1 Hz, 0.7 H), 6.76 (d, J = 6.9 Hz, 0.3 H), 6.87 (d, J = 6.4 Hz, 0.7 H), 6.95 (d, J = 8.1 Hz, 0.3 H), 7.39 (dd, J = 8.4 Hz, 1.8 Hz, 2H), 7.73 (dd, J = 8.4 Hz, 2.1 Hz, 2 H).

### Inventive Example 12 Production of (3S)-3-{(S)-4-methyl-2-(4-methylbenzoylamino)valerylamino}-2-tetrahydrofuranol (Compound No.. 89 in Table 1)

Melting point: 101 - 102°C
IR (KBr, cm⁻¹): 3304, 1634, 1545, 1504.
NMR (CDCl₃, δ): 0.80 - 1.07 (m, 6 H), 1.60 - 1.95 (m, 4 H), 2.37 (m, 1 H), 2.39 (s, 3 H), 3.45 (d, J = 2.9 Hz, 0.6 H), 3.90 (m, 0.4 H), 3.97 - 4.19 (m, 2 H), 4.38 (m, 1 H), 4.71 (m, 1 H), 5.30 (d, J = 2.9 Hz, 0.6 H), 5.35 (dd, J = 6.7 Hz, 6.7 Hz, 0.6 H), 6.74 (d, J = 8.5 Hz, 0.4 H), 6.80 (d, J = 8.5 Hz, 1 H), 6.89 (d, J = 6.7 Hz, 0.6 H), 7.22 (d, J = 8.2 Hz, 2 H), 7.68 (d, J = 8.2 Hz, 2 H).

### Inventive Example 13 Production of (3S)-3-((S)-2-hexadecylsufonylamino-4-methylvalerylamino)-2-tetrahydrofuranol (Compound No.. 131 in Table 1)

IR (KBr, cm⁻¹): 3337, 1647, 1543.
NMR (CDCl₃, δ): 0.85 - 0.98 (m, 9 H), 1.23 - 1.47 (m, 29 H), 1.56 - 1.61 (m, 2 H), 1.78 - 1.88 (m, 2 H), 2.94 - 3.03 (m, 2 H), 3.88 - 4.21 (m, 4 H), 4.25 - 4.53 (m, 1 H), 5.24 - 5.34 (m, 1 H), 6.44 (m, 0.6 H), 6.75 (m, 0.4 H).

### Inventive Example 14 Production of (3S)-3-{(S)-2-(4-chlorophenylsulfonylamino)-4-methylvalerylamino}-2-tetrahydrofuranol (Compound No.. 141 in Table 1)

Melting point: 112 - 115°C
IR (KBr, cm⁻¹): 3335, 3264, 1649.
NMR (CDCl₃, δ): 0.76 (d, J = 6.3 Hz, 0.6 H), 0.80 (d, J = 6.3 Hz, 2.4 H), 0.87 (d, J = 6.9 Hz, 0.6 H), 0.89 (d, J = 6.6 Hz, 2.4 H), 1.48 (m, 3 H), 1.68 (m, 1 H), 2.11 (m, 0.8 H), 2.40 (m, 0.2 H), 3.67 (ddd, J = 6.9 Hz, 6.9 Hz, 6.9 Hz, 1 H), 3.85 (m, 0.8 H), 3.94 (m, 0.2 H), 4.05 - 4.21 (m, 2 H), 5.18 (s, 0.2 H), 5.25 (d, J = 4.5 Hz, 0.8 H), 5.31 (d, J = 9.9 Hz, 0.2 H), 5.35 (d, J = 8.4 Hz, 0.8 H), 5.94 (d, J = 7.8 Hz, 0.2 H), 6.23 (d, J = 7.8 Hz, 0.8 H), 7.48 (d, J = 8.4 Hz, 2 H), 7.80 (d, J = 8.4 Hz, 2 H).

### Inventive Example 15 Production of (3S)-3-{(S)-4-methyl-2-(2,4,6-trimethylphenylsulfonylamino)valerylamino}-2-tetrahydrofuranol (Compound No.. 151 in Table 1)

Melting point: 75 - 77°C
IR (KBr, cm⁻¹): 3328, 1657, 1605, 1541.
NMR (CDCl₃, δ): 0.67 (d, J = 6.3 Hz, 1.35 H), 0.68 (d, J = 6.3 Hz, 1.65 H), 0.83 (d, J = 6.4 Hz, 1.35 H), 0.84 (d, J = 6.4 Hz, 1.65 H), 1.38 - 1.72 (m, 4 H), 2.11 (m, 0.65 H), 2.29 (s, 3 H), 2.31 (m, 0.45 H), 2.63 (s, 6 H), 3.61 (m, 1 H), 3.72 (d, J = 3.0 Hz, 0.45 H), 3.74 - 3.98 (m, 1 H), 3.98 - 4.24 (m, 2.55 H), 5.23 (d, J = 3.0 Hz, 0.45 H), 5.28 (dd, J = 3.9 Hz, 3.9 Hz, 0.55 H), 5.46 (d, J = 8.6 Hz, 0.45 H), 5.60 (d, J = 8.0 Hz, 0.55 H), 6.38 (d, J = 7.4 Hz, 0.45 H), 6.54 (d, J = 8.0 Hz, 0.55 H), 6.95 (s, 2 H).

### Inventive Example 16 Production of (3S)-2-{(S)-2-(4-tert-butylphenylsulfonylamino)-4-methylvalerylamino}-2-tetrahydrofuranol (Compound No.. 154 in Table 1)

Melting point: 140 - 141°C
IR (KBr, cm⁻¹): 3362, 3161, 1647, 1535.
NMR (DMSO-d₆, δ): 0.74 (d, J = 6.5 Hz, 3 H), 0.81 (d, J = 6.6 Hz, 3 H), 1.15 - 1.41 (m, 3 H), 1.29 (s, 9 H), 1.54 (m, 1 H), 1.95 (m, 1 H), 3.46 (m, 1 H), 3.62 - 3.82 (m, 3 H), 4.80 (d, J = 4.3 Hz, 1 H), 6.06 (d, J = 4.3 Hz, 1 H), 7.53 (d, J = 8.5 Hz, 2 H), 7.66 (d, J = 8.5 Hz, 2 H), 7.82 (d, J = 9.4 Hz, 1 H), 7.95 (d, J = 6.7 Hz, 1 H).

### Inventive Example 17 Production of (3S)-3-{(S)-2-(4-methoxyphenylsulfonylamino)-4-methylvalerylamino}-2-tetrahydrofuranol (Compound No.. 157 in Table 1)

Melting point: 153 - 155°C
IR (KBr, cm⁻¹): 3362, 3150, 1647, 1597, 1535, 1501.
NMR (DMSO-d₆, δ): 0.69 (m, 0.6 H), 0.76 (d, J = 6.5 Hz, 2.7 H), 0.82 (d, J = 6.7 Hz, 2.7 H), 1.15 - 1.45 (m, 3 H), 1.56 (m, 1 H), 2.01 (m, 1 H), 3.54 (m, 1 H), 3.60 - 3.90 (m, 3 H), 3.81 (s, 3 H), 4.89 (d, J = 4.6 Hz, 0.9 H), 4.99 (m, 0.1 H), 6.09 (d, J = 4.6 Hz, 0.9 H), 6.41 (d, J = 2.7 Hz, 0.1 H), 7.04 (d, J = 8.8 Hz, 2 H), 7.60 - 7.80 (m, 3.1 H), 7.94 (d, J = 6.9 Hz, 0.9 H).

### Inventive Example 18 Production of (3S)-3-{(S)-4-methyl-2-(2-naphthylsulfonylamino)valerylamino}-2-tetrahydrofuranol (Compound No.. 166 in Table 1)

Melting point: 102 - 104°C
IR (KBr, cm⁻¹): 3351, 1655, 1541.
NMR (DMSO-d₆, δ): 0.65 (d, J = 6.4 Hz, 1.5 H), 0.65 - 0.90 (m, 4.5 H), 1.14 - 1.68 (m, 5 H), 3.25 (m, 0.5 H), 3.57 - 3.91 (m, 3.5 H), 4.73 (s, 0.5 H), 4.89 (dd, J = 4.3 Hz, 4.3 Hz, 0.5 H), 5.98 (s, 0.5 H), 6.32 (d, J = 4.3 Hz, 0.5 H), 7.60 - 7.80 (m, 3.5 H), 7.93 (d, J = 6.7 Hz, 0.5 H), 7.98 - 8.17 (m, 4 H), 8.38 (d, J = 8.3 Hz, 1 H).

### Inventive Example 19 Production of (3S)-4-methyl-3-((S)-4-methyl-2-phenylsulfonylaminovalerylamino)-2-tetrahydrofuranol (Compound No.. 180 in Table 1)

Melting point: 116 - 121°C
IR (KBr, cm⁻¹): 3356, 3272, 1655.
NMR (CDCl₃, δ): 0.70 (d, J = 6.0 Hz, 2.1 H), 0.71 (d, J = 6.0 Hz, 0.9 H), 0.86 (d, J = 6.6 Hz, 3 H), 0.95 (d, J = 6.6 Hz, 2.1 H), 1.04 (d, J = 6.6 Hz, 0.9 H), 1.50 (m, 2 H), 1.62 (m, 1 H), 2.12 (m, 1 H), 3.43 (m, 1 H), 3.70 (m, 1 H), 3.91 (m, 1 H), 4.17 (dd, J = 8.4 Hz, 8.4 Hz, 1 H), 5.12 (d, J = 4.5 Hz, 0.3 H), 5.25 (d, J = 4.5 Hz, 0.7 H), 5.35 (d, J = 7.2 Hz, 0.7 H), 5.40 (d, J = 7.2 Hz, 0.3 H), 6.34 (d, J = 8.7 Hz, 0.3 H), 6.37 (d, J = 8.7 Hz, 0.7 H), 7.49 - 7.62 (m, 3 H), 7.88 (m, 2 H).

### Inventive Example 20 Production of (2S,3S)-2-acetoxy-3-((S)-4-methyl-2-tetradecyloxycarbonylaminovalerylamino)tetrahydrofuran (Compound No.. 296 in Table 2)

Melting point: 114 - 115°C
IR (KBr, cm⁻¹): 3304, 1748, 1696, 1653, 1541.
NMR (CDCl₃, δ): 0.82 - 1.01 (m, 9 H), 1.26 (m, 22 H), 1.42 - 1.75 (m, 5 H), 1.86 (m, 1 H), 2.11 (s, 3 H), 2.37 (m, 1 H), 3.88 - 4.20 (m, 5 H), 4.59 (m, 1H), 4.98 (m, 1 H), 6.16 (d, J = 4.6 Hz, 1 H), 6.31 (d, J = 9.7 Hz, 1 H).

### Inventive Example 21 Production of (2S,3S)-2-acetoxy-3-((S)-2-benzyloxycarbonylamino-4-methylvalerylamino)tetrahydrofuran (Compound No.. 300 in Table 2)

Melting point: 162 - 164°C
IR (KBr, cm⁻¹): 3310, 1637, 1655, 1535.
NMR (CDCl₃, δ): 0.94 (d, J = 6.2 Hz, 3 H), 0.95 (d, J = 6.2 Hz, 3 H), 1.83 (m, 1 H), 2.07 (s, 3 H), 2.11 (m, 3 H), 2.36 (m, 1 H), 3.93 (ddd, J = 7.8 Hz, 7.8 Hz, 7.8 Hz, 1 H), 4.09 (m, 2 H), 4.79 (m, 1 H), 5.12 (s, 2 H), 5.32 (s, 1 H), 6.04 (d, J = 4.2 Hz, 1 H, 6.17 (d, J = 4.4 Hz, 1 H), 7.25 (m, 5 H).

### Inventive Example 22 Production of (2S,3S)-2-acetoxy-3-((S)-4-methyl-2-pentadecanoylaminovalerylamino)tetrahydrofuran (Compound No.. 334 in Table 2)

Melting point: 129 - 130°C
IR (KBr, cm⁻¹): 3297, 1748, 1642, 1545.
NMR (CDCl₃, δ): 0.78 - 1.05 (m, 9 H), 1.25 (m, 22 H), 1.42 - 1.70 (m, 3 H), 1.70 - 2.00 (m, 2 H), 2.05 (m, 1 H), 2.07 (s, 3 H), 2.18 (t, J = 6.9 Hz, 2 H), 2.33 (m, 1 H), 3.95 (m, 1 H), 4.11 (m, 1 H), 4.45 (m, 1 H), 4.55 (m, 1 H), 6.04 (d, J = 8.0 Hz, 1 H), 6.18 (d, J = 4.5 Hz, 1 H), 6.69 (d, J = 8.4 Hz, 1 H).

### Inventive Example 23 Production of (2S,3S)-2-acetoxy-3-((S)-2-hexadecanoylamino-4-methylvalerylamino)tetrahydrofuran (Compound No.. 335 in Table 2)

Melting point: 137°C
IR (KBr, cm⁻¹): 3297, 1748, 1642, 1543.
NMR (CDCl₃, δ): 0.78 - 1.01 (m, 9 H), 1.25 (m, 24 H), 1.42 - 1.70 (m, 5 H), 1.83 (m, 1 H), 2.09 (s, 3 H), 2.20 (t, J = 7.0 Hz, 2 H), 2.35 (m, 1 H), 3.95 (m, 1 H), 4.13 (m, 1 H), 4.40 (m, 1 H), 4.55 (m, 1 H), 5.91 (d, J = 8.3 Hz, 1 H), 6.17 (d, J = 4.6 Hz, 1 H, 6.57 (d, J = 10.0 Hz, 1 H).

### Inventive Example 24 Production of (2S,3S)-2-acetoxy-3-{(S)-2-(2-fluorobenzoylamino)-4-methylvalerylamino}tetrahydrofuran (Compound No.. 345 in Table 2)

Melting point: 165 - 166°C
IR (KBr, cm⁻¹): 3343, 3304, 1748, 1694, 1640, 1551.
NMR (CDCl₃, δ): 0.96 (d, J = 5.7 Hz, 3 H), 0.98 (d, J = 5.7 Hz, 3 H), 1.58 - 1.95 (m, 4 H), 2.10 (s, 3 H), 2.39 (m, 1 H), 3.95 (ddd, J = 9.0 Hz, 9.0 Hz, 7.4 Hz, 1 H), 4.14 (ddd, J = 9.0 Hz, 9.0 Hz, 2.9 Hz, 1 H), 4.55 - 4.75 (m, 2 H), 6.19 (d, J = 4.8 Hz, 1 H), 6.61 (d, J = 8.2 Hz, 1 H), 7.00 (d, J = 7.4 Hz, 0.5 H), 7.02 (d, J = 7.4 Hz, 0.5 H), 7.15 (dd, J = 8.2 Hz, 7.5 Hz, 1 H), 7.29 (ddd, J = 6.8 Hz, 6.8 Hz, 0.9 Hz, 1 H), 7.55 (m, 1 H), 8.06 (ddd, J = 6.8 Hz, 6.8 Hz, 1.9 Hz, 1 H).

### Inventive Example 25 Production of (2S,3S)-2-acetoxy-3-{(S)-2-(4-chlorobenzoylamino)-4-methylvalerylamino}tetrahydrofuran (Compound No.. 353 in Table 2)

Melting point: 204 - 205°C
IR (KBr, cm⁻¹): 3304, 1746, 1674, 1635.
NMR (CDCl₃, δ): 0.96 (d, J = 6.1 Hz, 3 H), 0.97 (d, J = 6.0 Hz, 3 H), 1.58 - 1.78 (m, 3 H), 1.85 (m, 1 H), 2.13 (s, 3 H), 2.31 (m, 1 H), 3.95 (m, 1 H), 4.14 (ddd, J = 8.6 Hz, 8.6 Hz, 2.9 Hz, 1 H), 4.55 - 4.70 (m, 2 H), 6.20 (d, J = 4.6 Hz, 1 H), 6.62 (d, J = 8.3 Hz, 1 H), 6.81 (d, J = 7.8 Hz, 1 H), 7.41 (d, J = 6.7 Hz, 2 H), 7.73 (d, J = 6.7 Hz, 2 H).

### Inventive Example 26 Production of (2S,3S)-2-acetoxy-3-((S)-2-hexadecylsulfonylamino-4-methylvalerylamino)tetrahydrofuran (Compound No.. 401 in Table 2)

NMR (CDCl₃, δ): 0.86 - 0.97 (m, 9 H), 1.21 - 1.41 (m, 30 H), 1.45 - 1.89 (m, 3 H), 2.12 (s, 3 H), 2.98 (m, 2 H), 3.85 (m, 1 H), 3.98 (m, 1 H), 4.15 (m, 1 H), 4.60 (m, 1 H), 4.76 (d, J = 8.6 Hz, 1 H), 6.18 (d, J = 4.6 Hz, 1 H), 6.32 (d, J = 8.6 Hz, 1 H).

### Inventive Example 27 Production of (2S,3S)-3-((S)-4-methyl-2-phenylsulfonylaminovalerylamino)-2-pivaloyloxytetrahydrofuran (Compound No.. 410 in Table 2)

Melting point: 165 - 166°C
IR (KBr, cm⁻¹): 3293, 1640.
NMR (CDCl₃, δ): 0.64 (d, J = 5.9 Hz, 3 H), 0.81 (d, J = 6.1 Hz, 3 H), 1.26 (s, 9 H), 1.48 (m, 3 H), 1.72 (m, 1 H), 2.23 (m, 1 H), 3.64 (m, 1 H), 3.94 (ddd, J = 9.1 Hz, 9.1 Hz, 9.1 Hz, 1 H), 4.10 (ddd, J = 9.1 Hz, 9.1 Hz, 3.1 Hz, 1 H), 4.45 (m, 1 H), 5.07 (d, J = 7.7 Hz, 1 H), 6.10 (d, J = 4.5 Hz, 1 H), 6.21 (d, J = 8.4 Hz, 1 H), 7.51 (m, 2 H), 7.61 (m, 1 H), 7.86 (d, J = 7.1 Hz, 2 H).

### Inventive Example 28 Production of (2S,3S)-2-acetoxy-3-{(S)-2-(4-chlorophenylsulfonylamino)-4-methylvalerylamino}tetrahydrofuran (Compound No.. 416 in Table 2)

Melting point: 136 - 137°C
IR (KBr, cm⁻¹): 3335, 3258, 1744, 1651, 1535.
NMR (CDCl₃, δ): 0.61 (d, J = 6.2 Hz, 3 H), 0.83 (d, J = 6.3 Hz, 3 H), 1.35 - 1.62 (m, 3 H), 1.80 (m, 1 H), 2.15 (s, 3 H), 2.21 (m, 1 H), 3.61 (m, 1 H), 3.97 (ddd, J = 9.1 Hz, 9.1 Hz, 9.1 Hz, 1 H), 4.14 (ddd, J = 9.1 Hz, 9.1 Hz, 2.9 Hz, 1 H), 4.50 (m, 1 H), 5.09 (d, J = 7.3 Hz, 1 H), 6.15 (d, J = 4.6 Hz, 1 H), 6.42 (d, J = 8.8 Hz, 1 H), 7.51 (d, J = 8.6 Hz, 2 H), 7.80 (d, J = 8.6 Hz, 2 H).

### Inventive Example 29 Production of (2S,3S)-2-acetoxy-3-{(S)-4-methyl-2-(2,4,6-trimethylphenylsulfonylamino)valerylamino}tetrahydrofuran (Compound No.. 426 in Table 2)

Melting point: 158 - 159°C
IR (KBr, cm⁻¹): 3416, 3191, 1755, 1661, 1605, 1535.
NMR (CDCl₃, δ): 0.56 (d, J = 6.3 Hz, 3 H), 0.79 (d, J = 6.3 Hz, 3 H), 1.39 (m, 2 H), 1.58 (m, 1 H), 1.81 (m, 1 H), 2.16 (s, 3 H), 2.24 (m, 1 H), 2.31 (s, 3 H), 2.62 (s, 6 H), 3.56 (m, 1 H), 3.94 (m, 1 H), 4.14 (m, 1 H), 4.52 (m, 1 H), 5.00 (d, J = 7.1 Hz, 1 H), 6.15 (d, J = 4.6 Hz, 1 H), 6.61 (d, J = 8.7 Hz, 1 H), 6.97 (s, 2 H).

### Inventive Example 30 Production of (2S,3S)-3-{(S)-2-(4-tert-butylphenylsulfonylamino)-4-methylvalerylamino}-2-pivaloyloxytetrahydrofuran (Compound No.. 434 in Table 2)

Melting point: 166 - 167°C
IR (KBr, cm⁻¹): 3360, 1728, 1682, 1665, 1547.
NMR (CDCl₃, δ): 0.55 (d, J = 6.0 Hz, 3 H), 0.78 (d, J = 6.0 Hz, 3 H), 1.25 (s, 9 H), 1.32 (s, 9 H), 1.38 - 1.82 (m, 4 H), 2.25 (m, 1 H), 3.60 (m, 1 H), 3.95 (m, 1 H), 4.12 (ddd, J = 9.0 Hz, 9.0 Hz, 3.0 Hz, 1 H), 4.46 (m, 1 H), 5.05 (d, J = 7.1 Hz, 1 H), 6.10 (d, J = 4.5 Hz, 1 H), 6.41 (d, J = 8.3 Hz, 1 H), 7.52 (d, J = 8.6 Hz, 2 H), 7.78 (d, J = 8.6 Hz, 2 H).

### Inventive Example 31 Production of (2S,3S)-2-acetoxy-3-{(S)-2-(4-methoxyphenylsulfonylamino)-4-methylvalerylamino}tetrahydrofuran (Compound No.. 437 in Table 2)

Melting point: 157 - 158°C
IR (KBr, cm⁻¹): 3329, 3273, 1746, 1659, 1597, 1544, 1501.
NMR (CDCl₃, δ): 0.54 (d, J = 6.1 Hz, 3 H), 0.81 (d, J = 6.3 Hz, 3 H), 1.38 (m, 2 H), 1.56 (m, 1 H), 1.84 (m, 1 H), 2.16 (s, 3 H), 2.22 (m, 1 H), 3.56 (m, 1 H), 3.88 (s, 3 H), 3.95 (m, 1 H), 4.15 (m, 1 H), 4.56 (m, 1 H), 4.81 (d, J = 6.4 Hz, 1 H), 6.16 (d, J = 4.5 Hz, 1 H), 6.68 (d, J = 9.2 Hz, 1 H), 699 (d, J = 8.6 Hz, 2 H), 7.79 (d, J = 8.6 Hz, 2 H).

### Inventive Example 32 Production of (2S,3S)-2-acetoxy-3-{(S)-4-methyl-2-(2-naphthylsulfonylamino)valerylamino}tetrahydrofuran (Compound No.. 446 in Table 2)

Melting point: 158 - 159°C
IR (KBr, cm⁻¹): 3337, 3275, 1723, 1676, 1543.
NMR (CDCl₃, δ): 0.48 (d, J = 6.1 Hz, 3 H), 0.76 (d, J = 6.2 Hz, 3 H), 1.33 - 1.75 (m, 4 H), 1.97 (m, 1 H), 2.15 (s, 3 H), 3.68 (m, 1 H), 3.87 (m, 1 H), 4.05 (m, 1 H), 4.42 (m, 1 H), 5.19 (d, J = 7.1 Hz, 1 H), 6.12 (d, J = 4.6 Hz, 1 H), 6.53 (d, J = 8.7 Hz, 1 H), 6.53 (d, J = 8.7 Hz, 2 H), 7.58 - 7.71 (m, 2 H), 7.82 (dd, J = 8.7 Hz, 1.9 Hz, 1 H), 7.87 - 8.03 (m, 3 H), 8.44 (s, 1 H).

### Inventive Example 33 Production of (3S)-3-((S)-2-heptadecanoylamino-4-methylvalerylamino)-2-tetrahydrofuranol (Compound No.. 66 in Table 1)

Melting point: 79 - 82°C
IR (KBr, cm⁻¹): 3300, 1637, 1543.
NMR (CDCl₃, δ): 0.90 - 1.03 (m, 9 H), 1.25 (m, 26 H), 1.45 - 1.70 (m, 5 H), 1.70 - 1.95 (m, 2 H), 2.10 - 2.22 (m, 2 H), 2.20 - 2.50 (m, 1 H), 3.78 - 4.18 (m, 2 H), 4.20 - 4.60 (m, 2 H), 5.12 - 5.18 (m, 1 H), 6.13 (d, J = 8.4 Hz, 0.6 H), 6.22 (d, J = 8.3 Hz, 0.4 H), 6.77 (d, J = 8.4 Hz, 0.6 H), 6.82 (d, J = 7.0 Hz, 0.4 H).

### Inventive Example 34 Production of (2S,3S)-2-acetoxy-3-((S)-2-heptadecanoylamino-4-methylvalerylamino)tetrahydrofuran (Compound No.. 336 in Table 2)

Melting point: 127 - 128°C
IR (KBr, cm⁻¹): 3297, 1750, 1642, 1545.
NMR (CDCl₃, δ): 0.82 - 0.98 (m, 9 H), 1.25 (m, 26 H), 1.43 - 1.70 (m, 5 H), 1.83 (m, 1 H), 2.13 (s, 3 H), 2.20 (t, J = 7.2 Hz, 2 H), 2.32 (m, 1 H), 3.97 (m, 1 H), 4.14 (m, 1 H), 4.40 (m, 1 H), 4.55 (m, 1 H), 6.01 (d, J = 8.1 Hz, 1 H), 6.17 (d, J = 4.6 Hz, 1 H), 6.66 (d, J = 8.6 Hz, 1 H).

### Inventive Example 35 Production of (3S)-3-((S)-4-methyl-2-tridecyloxycarbonylaminovalerylamino)-2-tetrahydrofuranol (Compound No.. 25 in Table 1)

Melting point: 75 - 76°C
IR (KBr, cm⁻¹): 3302, 1696, 1649, 1545.
NMR (CDCl₃, δ): 0.89 - 1.02 (m, 9 H), 1.15 - 1.40 (m, 19 H), 1.45 - 1.75 (m, 5 H), 1.80 (m, 1 H), 2.05 (m, 1 H), 2.38 (m, 1 H), 3.86 (m, 1 H), 3.96 - 4.25 (m, 4.6 H), 4.35 (m, 1 H), 4.48 (s, 0.4 H), 5.27 (d, J = 2.4 Hz, 0.6 H), 5.33 (dd, J = 3.8 Hz, 3.8 Hz, 0.4 H), 5.40 (d, J = 7.8 Hz, 1 H), 6.72 (d, J = 7.8 Hz, 1 H).

### Inventive Example 36 Production of (2S,3S)-2-acetoxy-3-((S)-4-methyl-2-tridecyloxycarbonylaminovalerylamino)tetrahydrofuran (Compound No.. 295 in Table 2)

Melting point: 112°C
IR (KBr, cm⁻¹): 3304, 1748, 1696, 1655, 1541.
NMR (CDCl₃, δ): 0.88 (t, J = 6.1 Hz, 3 H), 0.91 - 0.99 (m, 6 H), 1.15 - 1.40 (m, 21 H), 1.43 - 1.77 (m, 4 H), 1.87 (m, 1 H), 2.11 (s, 3 H), 2.36 (m, 1 H), 3.95 (m, 1 H), 4.01 - 4.20 (m, 4 H), 4.58 (m, 1 H), 5.05 (d, J = 8.3 Hz, 1 H), 6.17 (d, J = 4.6 Hz, 1 H), 6.36 (d, J = 7.9 Hz, 1 H).

### Inventive Example 37 Production of (3S)-3-((S)-4-methyl-2-pentadecyloxycarbonylaminovalerylamino)-2-tetrahydrofuranol (Compound No.. 27 in Table 1)

IR (KBr, cm⁻¹): 3302, 1695, 1649, 1543.
NMR (CDCl₃, δ): 0.85 - 0.96 (m, 9 H), 1.25 - 1.29 (m, 24 H), 1.50 - 1.89 (m, 6 H), 2.29 (m, 0.7 H), 2.42 (m, 0.3 H), 3.81 - 4.25 (m, 5 H), 4.28 - 4.41 (m, 1 H), 5.24 (s, 1 H), 5.26 (s, 0.3 H), 5.32 (d, J = 4.6 Hz, 0.7 H), 6.50 (s, 0.3 H), 6.60 (d, J = 7.8 Hz, 0.7 H).

### Inventive Example 38 Production of (2S,3S)-2-acetoxy-4-((S)-4-methyl-2-pentadecyloxycarbonylaminovalerylamino)tetrahydrofuran (Compound No.. 297 in Table 2)

Melting point: 114 - 115°C
IR (KBr, cm⁻¹): 3304, 1693, 1655, 1545.
NMR (CDCl₃, δ): 0.88 (m, 3 H), 0.91 - 0.99 (m, 6 H), 1.23 - 1.39 (m, 24 H), 1.51 - 1.69 (m, 5 H), 1.84 (m, 1 H), 2.10 (s, 3 H), 2.36 (m, 1 H), 3.92 - 4.17 (m, 5 H), 4.58 (m, 1 H), 4.98 (m, 1 H), 6.16 (d, J = 4.7 Hz, 1 H), 6.31 (d, J = 8.3 Hz, 1 H).

### Inventive Example 39 Production of (3S)-3-((S)-4-methyl-2-tetradecylsulfonylaminovalerylamino)-2-tetrahydrofuranol (Compound No.. 129 in Table 1)

IR (KBr, cm⁻¹): 3346, 1643, 1533.
NMR (CDCl₃, δ): 0.85 - 0.90 (m, 3 H), 0.94 - 0.98 (m, 6 H), 1.25 - 1.39 (m, 22 H), 1.56 - 1.83 (m, 5 H), 2.20 - 2.38 (m, 1 H), 2.69 - 2.81 (m, 1 H), 2.98 - 3.04 (m, 2 H), 3.92 - 3.98 (m, 1 H), 4.46 - 4.58 (m, 2 H), 5.04 - 5.07 (m, 1 H), 6.85 - 6.87 (m, 1 H).

### Inventive Example 40 Production of (2S,3S)-2-acetoxy-3-((S)-4-methyl-2-tetradecylsulfonylaminovalerylamino)tetrahydrofuran (Compound No.. 399 in Table 2)

Melting point: 84 - 85°C
IR (KBr, cm⁻¹): 3315, 1651, 1545.
NMR (CDCl₃, δ): 0.88 (m, 3 H), 0.94 - 0.98 (m, 6 H), 1.23 - 1.94 (m, 28 H), 2.12 (s, 3 H), 2.36 (m, 1 H), 2.98 (m, 2 H), 3.85 (m, 1 H), 3.97 (m, 1 H), 4.15 (m, 1 H), 4.59 (m, 1 H), 4.74 (m, 1 H), 6.17 (d, J = 4.5 Hz, 1 H), 6.32 (d, J = 8.5 Hz, 1 H).

### Inventive Example 41 Production of (3S)-3-((S)-4-methyl-2-pentadecylsulfonylaminovalerylamino)-2-tetrahydrofuranol (Compound No.. 130 in Table 1)

IR (KBr, cm⁻¹): 3348, 1643, 1541.
NMR (CDCl₃, δ): 0.85 - 0.90 (m, 3 H), 0.94 - 0.98 (m, 6 H), 1.25 - 1.42 (m, 24 H), 1.58 - 1.63 (m, 2 H), 1.72 - 1.86 (m, 3 H), 2.30 - 2.39 (m, 0.8 H), 2.40 - 2.58 (m, 0.2 H), 2.94 - 3.02 (m, 2 H), 3.80 - 3.94 (m, 2 H), 4.04 (m, 0.2 H), 4.10 - 4.17 (m, 0.8 Hz), 4.32 - 4.41 (m, 1 H), 5.00 - 5.05 (m, 1 H), 5.27 (m, 0.2 H), 5.34 (m, 0.8 H), 6.22 (d, J = 7.2 Hz, 0.2 H), 6.49 (d, J = 8.1 Hz, 0.8 H).

### Inventive Example 42 Production of (2S,3S)-2-acetoxy-3-((S)-4-methyl-2-pentadecylsulfonylaminovalerylamino)tetrahydrofuranol (Compound No.. 400 in Table 2)

Melting point: 80 - 81°C
IR (KBr, cm⁻¹): 3315, 1651, 1548.
NMR (CDCl₃, δ): 0.88 (m, 3 H), 0.92 - 0.98 (m, 6 H), 1.23 - 1.48 (m, 26 H), 1.50 - 1.98 (m, 5 H), 2.12 (s, 3 H), 2.97 (dd, J = 6.9 Hz, 3.0 Hz, 2 H), 3.84 (m, 1 H), 3.96 (m, 1 H), 4.15 (m, 1 H), 4.61 (m, 1 H), 4.69 (m, 1 H), 6.18 (d, J = 4.5 Hz, 1 H), 6.30 (d, J = 9.0 Hz, 1 H).

### Test Example 1 Measurement of inhibitory activity against calpain

m-Calpain was purified from rat brain in accordance with the method described in *Journal of Biological Chemistry*, vol. 259, p. 3210, 1984, and its inhibitory activity was measured in accordance with the method described in *Journal of Biological Chemistry*, vol. 259, p. 12489, 1984. The results are shown in Table 3.

It can be seen from Table 3 that the compounds of the present invention show strong inhibitory activity against calpain.

**Table 3**

| (Calpain ihhibitory activity) | |
|---|---|
| Test No. | IC₅₀ (µM) |
| 1 (No. 135) | 0.62 |
| 3 (No. 26) | 0.11 |
| 4 (No. 30) | 0.66 |
| 5 (No. 34) | 0.34 |
| 6 (No. 42) | 0.48 |
| 7 (No. 47) | 0.65 |
| 8 (No. 64) | 0.11 |
| 9 (No. 65) | 0.16 |
| 10 (No. 75) | 0.17 |
| 11 (No. 83) | 0.36 |
| 12 (No. 89) | 0.33 |
| 13 (No. 131) | 0.42 |
| 14 (No. 141) | 0.56 |
| 15 (No. 151) | 0.45 |
| 16 (No. 154) | 0.39 |
| 17 (No. 157) | 0.67 |
| 18 (No. 166) | 0.36 |
| 19 (No. 180) | 2.5 |

The parenthesized number indicates Compound No. shown in Table 1

### Test Example 2 Formation of active metabolite in blood

(2S,3S)-2-Acetoxy-3-((S)-4-methyl-2-phenylsulfonylaminovalerylamino)tetrahydrofuran obtained in Inventive Example 2 was dissolved in acetonitrile and added to rat serum to a final concentration of 100 µM. After 5 minutes of incubation at 37°C, this was mixed with acetonitrile and centrifuged and then the thus obtained supernatant fluid was checked by HPLC. It was found as the result that a 97% portion of the thus added (2S,3S)-2-acetoxy-3-((S)-4-methyl-2-phenylsulfonylaminovalerylamino)-tetrahydrofuran was hydrolyzed and converted into its active metabolite (3S)-3-((S)-4-methyl-2-phenylsulfonylaminovalerylamino)-2-tetrahydrofuranol (compound of Inventive Example 1). The same procedure was repeated using human and dog sera to confirm that the same active metabolite is formed thereby.

HPLC was carried out under the following conditions.
Column: Nucleosil 100 ₅C₁₈ 4.6 x 250 mm (manufactured by Nargel)
Column temperature: 50°C
Mobile phase: CH₃CN:H₂O:PIC A Low UV (manufactured by Waters) 22:78:1
Flow rate: 1 ml/min
Detection wave length: UV 222 nm

It is evident from these results that, among compounds of the aforementioned general formula (I), a compound in which R³ is not hydrogen atom is quickly converted in the living body into its lactol derivative as an active metabolite in which R³ is hydrogen atom. Test Example 3 Examination of the action to improve passive avoidance response after a short period of fore-brain ischemia

Each of male Wistar rats put under pentobarbital anesthesia was subjected to cauterization of both vertebral arteries and then to ligation of both common carotid arteries, and animals which did not show hemiplegia and the like neurological deficites were used in the test. Also, animals treated only with cauterization of vertebral arteries were used as a pseudo-operation group. During ischemia and after reperfusion, body temperature of the animals was maintained by putting them on a heat insulating mat until righting reflex was recovered.

An apparatus composed of a light room (50 x 50 x 50 cm) equipped with a 100 W bulb and a dark room (20 x 14 x 20 cm) was used in the passive avoidance response test. Each of the rats which have been subjected to cauterization of both vertebral arteries 48 hours prior to the test was put into the light room and its latency to enter the dark room was measured to be used as the response latency at the time of acquisition trial. In this case, animals which showed the latency of 90 seconds or more were excluded from this test. When each rat entered the dark room, the guillotine door was closed and the rat was immediately exposed to an electric shock (2.9 mA, 5 seconds) from the floor grid, thus completing the acquisition trial. Subsequently, 5 minutes of ischemia loading (occlusion of both common carotid arteries) was carried out under ether anesthesia, and retrieval test was carried out 48 hours after reperfusion. Similar to the case of the acquisition trial, each rat was put into the light room and its latency to enter the dark room (response latency) was observed for a maximum of 300 seconds. When an animal did not enter into the dark room for 300 seconds or more, its response latency was recorded as 300 seconds. Presence or absence of the action to improve passive avoidance response was judged by comparing response latencies of the acquisition trial and retrieval test. Each drug to be tested was orally administered 30 minutes before the ischemia loading. Also, a calcium antagonist flunarizine was used as a control drug.

As the results, flunarizine showed the improving action at a dose of 30 mg/kg, po, while each of the compound of Inventive Example 2 (Compound No.. 405 in Table 2) and the compound of Inventive Example 24 (Compound No.. 345 in Table 2) showed the improving action at a dose of 1 mg/kg, po. In consequence, the compound of the present invention can be made into a drug for use in the treatment of cerebral stroke.

### Test Example 4 The myocardial infarct size-limiting effect in acute myocardial infarction model of rat

An experiment was performed on female SD rats (14 weeks of age). The rats were lightly anesthetized with ether and a left thoracotomy wag performed. A permanent occlusion was placed around the proximal left coronary artery to produce acute myocardial infarction. Infarct size evaluated in each excised heart at 24 hours after the ligation.

The animals were divided into 2 groups at random, and 10 mg/kg of the compound of Inventive Example 29 (Compound No.. 426 in Table 2) suspended in water with Tween 80 was orally administered in a volume of 1 ml/kg to each animal of one of these groups (drug group) 1 hour before the coronary artery ligation, while the same volume of tap water was administered to the other group (control group). A rat with successful thoracotomy and which survived until 24 hours after the coronary ligation was used as the final evaluation subject. This study was continued until the final number of animals in each group reached 8.

Evaluation of the myocardial infarct size was carried out in the following manner. Each of the excised heart was cut into the slices at 2 mm thick from apex to base, and the sections were stained by incubating them in TTC solution (1%) at 37°C for 20 minutes to confirm necrotic region and non-necrotic region. Thereafter, sectional images of the sections were photographed to carry out image analysis on a computer. The infarction size was expressed as a % value obtained by dividing total area of necrotic area of all sliced sections by total left ventricle area (including septum part and excluding left ventricle cavity).

As the results, in the control group, 2 animals died during the thoracotomy and 9 animals died within 24 hours after the operation. In the drug group, on the other hand, 3 animals died during the thoracotomy and 10 animals died within 24 hours after the operation. No difference was obserbed between these two groups in terms of the number of deaths during the period until the final evaluation numbers reached 8 cases. Also, the total left ventricle area of sliced sections was 105.6 ± 6.3 mm² in the control group and 114.6 ± 3.8 mm² in the drug group, thus showing no significant difference between groups. The infarct size, on the other hand, was 37.3 ± 3.5% in the control group but 25.0 ± 1.7% in the drug group, thus showing significant reduction (p<0.05) in the myocardial infarct size effected by the oral administration of the compound of the present invention prior to the coronary artery ligation. The infarct size reducing ratio was 33.0% based on the control group.

These results suggested that the compound of the present invention can inhibit the development of heart muscle necrosis in a rat acute myocardial infarction model.

### Test Example 5 Acute toxicity test

Active ingredient of the medicament of the present invention was suspended in 0.5% CMC-Na aqueous solution and orally administered by force to female and male SD rats, and their symptoms were observed for 7 days.

LD₅₀ values of the compounds of Inventive Examples 2 and 29 were both >2,000 mg/kg.

### Test Example 6 Formulation examples

### (1) Tablets

The following components were mixed in the ordinary method and made into tablets using a conventional tablet making machine.

| (Per tablet) | |
|---|---|
| Compound of Inventive Example 2 | 30 mg |
| Crystalline cellulose | 60 mg |
| Corn starch | 100 mg |
| Lactose | 200 mg |
| Magnesium stearate | 4 mg |

### (2) Soft capsules

The following components were mixed in the ordinary method and packed in soft capsules.

| (Per capsule) | |
|---|---|
| Compound of Inventive Example 2 | 30 mg |
| Olive oil | 300 mg |
| Lecithin | 20 mg |

### (3) Preparations for injection use

The following components were mixed in the ordinary method to prepare 1 ml ampoules.

| (Per ampoule) | |
|---|---|
| Compound of Inventive Example 1 | 3 mg |
| Sodium chloride | 4 mg |
| Distilled water for injection use | 1 ml |

### INDUSTRIAL APPLICABILITY:

Since the ischemic disease-treating drug of the present invention shows excellent improving actions in a rat ischemia model and a rat acute myocardial infarction model, it is useful as a medicament for ischemic diseases such as ischemic brain diseases, cerebral stroke, cerebral thrombosis, cerebral embolism and myocardial infarction.

## Claims

1. A medicament for ischemic diseases, which comprises as its active ingredient an oxygen-containing heterocyclic derivative represented by the following general formula (I): 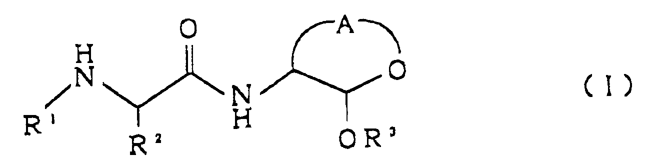 [wherein R¹ represents 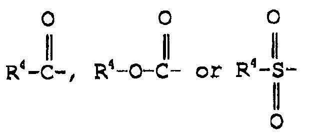 (wherein R⁴ represents a C₁-C₂₀ alkyl group which may be substituted with a C₆-C₁₄ aryl group that may have a substituent group; a C₃-C₈ cycloalkyl group; or a C₆-C₁₄ aryl group which may have a substituent group), R² represents a C₁-C₆ alkyl group, R³ represents hydrogen atom or 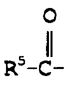 (wherein R⁵ represents a C₁-C₁₀ alkyl group), and A represents a C₁-C₃ alkylene group which may have a C₁-C₃ alkyl group], a pharmaceutically acceptable salt thereof, or a solvate or hydrate thereof.

2. The medicament for ischemic diseases according to claim 1 wherein R¹ represents 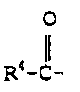 (wherein R⁴ represents a C₁-C₂₀ alkyl group which may be substituted with a C₆-C₁₄ aryl group that may have a substituent group, or a C₆-C₁₄ aryl group which may have a substituent group), R² represents a C₁-C₆ alkyl group, R³ represents hydrogen atom or 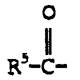 (wherein R⁵ represents a C₁-C₁₀ alkyl group), and A represents a C₁-C₃ alkylene group.

3. The medicament for ischemic diseases according to claim 2 wherein R⁴ is a C₁₄-C₁₆ alkyl group.

4. The medicament for ischemic diseases according to claim 1 wherein R¹ represents 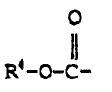 (wherein R⁴ represents a C₁-C₂₀ alkyl group which may be substituted with a C₆-C₁₄ aryl group that may have a substituent group, or a C₃-C₈ cycloalkyl group), R² represents a C₁-C₆ alkyl group, R³ represents hydrogen atom or 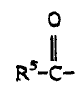 (wherein R⁵ represents a C₁-C₁₀ alkyl group), and A represents a C₁-C₃ alkylene group.

5. The medicament for ischemic diseases according to claim 4 wherein R⁴ is a C₁₃-C₁₅ alkyl group.

6. The medicament for ischemic diseases according to claim 1 wherein R¹ represents 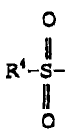 (wherein R⁴ represents a C₁-C₂₀ alkyl group or a C₆-C₁₄ aryl group which may have a substituent group), R² represents a C₁-C₆ alkyl group, R³ represents hydrogen atom or 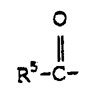 (wherein R⁵ represents a C₁-C₁₀ alkyl group), and A represents a C₁-C₃ alkylene group which may have a C₁-C₃ alkyl group.

7. The medicament for ischemic diseases according to claim 6 wherein R⁴ is a C₁₄-C₁₆ alkyl group.

8. The medicament for ischemic diseases according to claim 6 wherein R⁴ is a C₆-C₁₄ aryl group which may have a substituent group.

9. A medicament for ischemic diseases, which comprises as its active ingredient (3S)-3-((S)-4-methyl-2-phenylsulfonylaminovalerylamino)-2-tetrahydrofuranol, a pharmaceutically acceptable salt thereof, or a solvate or hydrate thereof.

10. A medicament for ischemic diseases, which comprises as its active ingredient (2S,3S)-2-acetoxy-3-((S)-4-methyl-2-phenylsulfonylaminovalerylamino)tetrahydrofuran, a pharmaceutically acceptable salt thereof, or a solvate or hydrate thereof.

11. A medicament for ischemic diseases, which comprises as its active ingredient (3S)-3-{(S)-4-methyl-2-(2,4,6-trimethylphenylsulfonylamino)valerylamino}-2-tetrahydrofuranol, a pharmaceutically acceptable salt thereof, or a solvate or hydrate thereof.

12. A medicament for ischemic diseases, which comprises as its active ingredient (2S,3S)-2-acetoxy-3-{(S)-4-methyl-2-(2,4,6-trimethylphenylsulfonylamino)valerylamino}tetrahydrofuran, a pharmaceutically acceptable salt thereof, or a solvate or hydrate thereof.

13. A medicament for ischemic diseases, which comprises as its active ingredient (3S)-3-((S)-4-methyl-2-pentadecanoylaminovalerylamino)-2-tetrahydrofuranol, a pharmaceutically acceptable salt thereof, or a solvate or hydrate thereof.

14. A medicament for ischemic diseases, which comprises as its active ingredient (2S,3S)-2-acetoxy-3-((S)-4-methyl-2-pentadecanoylaminovalerylamino)tetrahydrofuran, a pharmaceutically acceptable salt thereof, or a solvate or hydrate thereof.

15. A medicament for ischemic diseases, which comprises as its active ingredient (3S)-3-((S)-2-hexadecanoylamino-4-methylvalerylamino)-2-tetrahydrofuranol, a pharmaceutically acceptable salt thereof, or a solvate or hydrate thereof.

16. A medicament for ischemic diseases, which comprises as its active ingredient (2S,3S)-2-acetoxy-3-((S)-2-hexadecanoylamino-4-methylvalerylamino)tetrahydrofuran, a pharmaceutically acceptable salt thereof, or a solvate or hydrate thereof.

17. A medicament for ischemic diseases, which comprises as its active ingredient (3S)-3-((S)-2-heptadecanoylamino-4-methylvalerylamino)-2-tetrahydrofuranol, a pharmaceutically acceptable salt thereof, or a solvate or hydrate thereof.

18. A medicament for ischemic diseases, which comprises as its active ingredient (2S,3S)-2-acetoxy-3-((S)-2-heptadecanoylamino-4-methylvalerylamino)tetrahydrofuran, a pharmaceutically acceptable salt thereof, or a solvate or hydrate thereof.

19. A medicament for ischemic diseases, which comprises as its active ingredient (3S)-3-((S)-4-methyl-2-tridecyloxycarbonylaminovalerylamino)-2-tetrahydrofuranol, a pharmaceutically acceptable salt thereof, or a solvate or hydrate thereof.

20. A medicament for ischemic diseases, which comprises as its active ingredient (2S,3S)-2-acetoxy-3-((S)-4-methyl-2-tridecyloxycarbonylaminovalerylamino)tetrahydrofuran, a pharmaceutically acceptable salt thereof, or a solvate or hydrate thereof.

21. A medicament for ischemic diseases, which comprises as its active ingredient (3S)-3-((S)-4-methyl-2-tetradecyloxycarbonylaminovalerylamino)-2-tetrahydrofuranol, a pharmaceutically acceptable salt thereof, or a solvate or hydrate thereof.

22. A medicament for ischemic diseases, which comprises as its active ingredient (2S,3S)-2-acetoxy-3-((S)-4-methyl-2-tetradecyloxycarbonylaminovalerylamino)tetrahydrofuran, a pharmaceutically acceptable salt thereof, or a solvate or hydrate thereof.

23. A medicament for ischemic diseases, which comprises as its active ingredient (3S)-3-((S)-4-methyl-2-pentadecyloxycarbonylaminovalerylamino)-2-tetrahydrofuranol, a pharmaceutically acceptable salt thereof, or a solvate or hydrate thereof.

24. A medicament for ischemic diseases, which comprises as its active ingredient (2S,3S)-2-acetoxy-3-((S)-4-methyl-2-pentadecyloxycarbonylaminovalerylamino)tetrahydrofuran, a pharmaceutically acceptable salt thereof, or a solvate or hydrate thereof.

25. A medicament for ischemic diseases, which comprises as its active ingredient (3S)-3-((S)-4-methyl-2-tetradecylsulfonylaminovalerylamino)-2-tetrahydrofuranol, a pharmaceutically acceptable salt thereof, or a solvate or hydrate thereof.

26. A medicament for ischemic diseases, which comprises as its active ingredient (2S,3S)-2-acetoxy-3-((S)-4-methyl-2-tetradecylsulfonylaminovalerylamlno)tetrahydrofuran, a pharmaceutically acceptable salt thereof, or a solvate or hydrate thereof.

27. A medicament for ischemic diseases, which comprises as its active ingredient (3S)-3-((S)-4-methyl-2-pentadecylsulfonylaminovalerylamino)-2-tetrahydrofuranol, a pharmaceutically acceptable salt thereof, or a solvate or hydrate thereof.

28. A medicament for ischemic diseases, which comprises as its active ingredient (2S,3S)-2-acetoxy-3-((S)-4-methyl-2-pentadecylsulfonylaminovalerylamino)tetrahydrofuran, a pharmaceutically acceptable salt thereof, or a solvate or hydrate thereof.

29. A medicament for ischemic diseases, which comprises as its active ingredient (3S)-3-((S)-2-hexadecylsulfonylamino-4-methylvalerylamino)-2-tetrahydrofuranol, a pharmaceutically acceptable salt thereof, or a solvate or hydrate thereof.

30. A medicament for ischemic diseases, which comprises as its active ingredient (2S,3S)-2-acetoxy-3-((S)-2-hexadecylsulfonylamino-4-methylvalerylamino)tetrahydrofuran, a pharmaceutically acceptable salt thereof, or a solvate or hydrate thereof.

31. A medicament for ischemic diseases, which comprises as its active ingredient (3S)-4-methyl-3-((S)-1-methyl-2-phenylsulfonylaminovalerylamino)-2-tetrahydrofuranol, a pharmaceutically acceptable salt thereof, or a solvate or hydrate thereof.

32. The medicament for ischemic diseases according to any one of claims 1 to 31 wherein the ischemic disease is an ischemic brain disease.

33. The medicament for ischemic diseases according to any one of claims 1 to 31 wherein the ischemic disease is cerebral stroke.

34. The medicament for ischemic diseases according to any one of claims 1 to 31 wherein the ischemic disease is cerebral thrombosis.

35. The medicament for ischemic diseases according to any one of claims 1 to 31 wherein the ischemic disease is cerebral embolism.

36. The medicament for ischemic diseases according to any one of claims 1 to 31 wherein the ischemic disease is myocardial infarction.
